# EUROPÄISCHE PATENTSCHRIFT

(11) **EP 4 225 733 B1**
(45) Veröffentlichungstag und Bekanntmachung des Hinweises auf die Patenterteilung: **27.11.2024**
(21) Anmeldenummer: 21759101.5
(22) Anmeldetag: 20.08.2021
(51) Int. Cl.: C07D 201/00, C07D 209/86, C07D 251/24, C07D 307/91, C07D 405/12, C09K 11/06, C07C 211/54, C07C 211/58, C07C 211/61, H10K 85/60

(54) **VERBINDUNGEN FÜR ELEKTRONISCHE VORRICHTUNGEN**
COMPOUNDS FOR ELECTRONIC DEVICES
COMPOSÉS POUR DISPOSITIFS ÉLECTRONIQUES

(30) Priorität: 09.10.2020 EP 20201069; 07.05.2021 EP 21172857
(43) Veröffentlichungstag der Anmeldung: 16.08.2023
(73) Patentinhaber: Merck Patent GmbH, 64293 Darmstadt (DE)
(72) Erfinder: MONTENEGRO, Elvira, 64293 DARMSTADT (DE); MUJICA-FERNAUD, Teresa, 64293 DARMSTADT (DE); TUFFIN, Rachel, 64293 DARMSTADT (DE)
(74) Vertreter: Merck Patent Association
(86) Internationale Anmeldenummer: PCT/EP2021/073110
(87) Internationale Veröffentlichungsnummer: WO 2021/214348

(56) Entgegenhaltungen:
- US-A1- 2019 372 025
- XIAO HAIBO ET AL: "tert-Butylated spirobifluorene derivative incorporating triphenylamine groups: A deep-blue emitter with high thermal stability and good hole transport ability for organic light emitting diode applications", DYES AND PIGMENTS, vol. 121, 1 October 2015 (2015-10-01), GB, pages 7 - 12, XP055870013, ISSN: 0143-7208, DOI: 10.1016/j.dyepig.2015.03.027
- BRAVEENTH RAMANASKANDA ET AL: "Spirobifluorene Core-Based Novel Hole Transporting Materials for Red Phosphorescence OLEDs", MOLECULES, vol. 22, no. 3, 1 March 2017 (2017-03-01), DE, pages 464, XP055869985, ISSN: 1433-1373, DOI: 10.3390/molecules22030464
- SHEN J Y ET AL: "High Tg blue emitting materials for electroluminescent devices", JOURNAL OF MATERIALS CHEMISTRY, ROYAL SOCIETY OF CHEMISTRY, GB, vol. 15, no. 25, 12 May 2005 (2005-05-12), pages 2455 - 2463, XP003011710, ISSN: 0959-9428, DOI: 10.1039/B501819F
- SARAGI T P I ET AL: "COMPARISON OF CHARGE-CARRIER TRANSPORT IN THIN FILMS OF SPIRO-LINKED COMPOUNDS AND THEIR CORRESPONDING PARENT COMPOUNDS", ADVANCED FUNCTIONAL MATERIALS, WILEY - V C H VERLAG GMBH & CO. KGAA, DE, vol. 16, no. 7, 2 May 2006 (2006-05-02), pages 966 - 974, XP001241765, ISSN: 1616-301X, DOI: 10.1002/ADFM.200500361
- LI QIANNAN ET AL: "Hole-transporting material based on spirobifluorene unit with perfect amorphous and high stability for efficient OLEDs", JOURNAL OF MATERIALS SCIENCE: MATERIALS IN ELECTRONICS, CHAPMAN AND HALL, LONDON, GB, vol. 30, no. 12, 13 May 2019 (2019-05-13), pages 11440 - 11450, XP036802136, ISSN: 0957-4522, [retrieved on 20190513], DOI: 10.1007/S10854-019-01493-9

## Beschreibung

Die vorliegende Anmeldung betrifft Spirobifluoren-Amine, die bestimmte aromatische oder heteroaromatische Ringsysteme am Amin-Stickstoffatom aufweisen. Die Verbindungen eignen sich zur Verwendung in elektronischen Vorrichtungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs (organische Elektrolumineszenzvorrichtungen) verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an einer Verbesserung der Leistungsdaten. In diesen Punkten konnte noch keine vollständig zufriedenstellende Lösung gefunden werden.

Einen großen Einfluss auf die Leistungsdaten von elektronischen Vorrichtungen haben Emissionsschichten und Schichten mit lochtransportierender Funktion. Zur Verwendung in diesen Schichten werden weiterhin neue Verbindungen gesucht, insbesondere lochtransportierende Verbindungen und Verbindungen, die als lochtransportierendes Matrixmaterial, insbesondere für phosphoreszierende Emitter, in einer emittierenden Schicht dienen können. Hierzu werden insbesondere Verbindungen gesucht, die eine hohe Glasübergangstemperatur, eine hohe Stabilität, und eine hohe Leitfähigkeit für Löcher aufweisen. Eine hohe Stabilität der Verbindung ist eine Voraussetzung, um eine lange Lebensdauer der elektronischen Vorrichtung zu erreichen. Weiterhin werden Verbindungen gesucht, deren Verwendung in elektronischen Vorrichtungen zur Verbesserung der Leistungsdaten der Vorrichtungen führt, insbesondere zu hoher Effizienz, langer Lebensdauer und geringer Betriebsspannung.

Im Stand der Technik sind insbesondere Triarylaminverbindungen wie Spirobifluorenamine und Fluorenamine als Lochtransportmaterialien und lochtransportierende Matrixmaterialien für elektronische Vorrichtungen bekannt. Solche Verbindungen sind beispielsweise in US2019/372025 A1 offenbart. Es besteht jedoch weiterhin Verbesserungsbedarf bezüglich der oben genannten Eigenschaften.

Es wurde nun gefunden, dass sich Spirobifluoren-Amine gemäß untenstehender Formel (I), die dadurch gekennzeichnet sind, dass sie bestimmte aromatische oder heteroaromatische Ringsysteme am Amin-Stickstoffatom aufweisen, hervorragend zur Verwendung in elektronischen Vorrichtungen eignen. Sie eignen sich insbesondere zur Verwendung in OLEDs, nochmals insbesondere darin zur Verwendung als Lochtransportmaterialien und zur Verwendung als lochtransportierende Matrixmaterialien, insbesondere für phosphoreszierende Emitter. Die Verbindungen führen zu hoher Lebensdauer, hoher Effizienz und geringer Betriebsspannung der Vorrichtungen. Weiterhin bevorzugt weisen die gefundenen Verbindungen eine hohe Glasübergangstemperatur, eine hohe Stabilität und eine hohe Leitfähigkeit für Löcher auf.

Gegenstand der vorliegenden Anmeldung sind damit Verbindungen einer Formel (I) 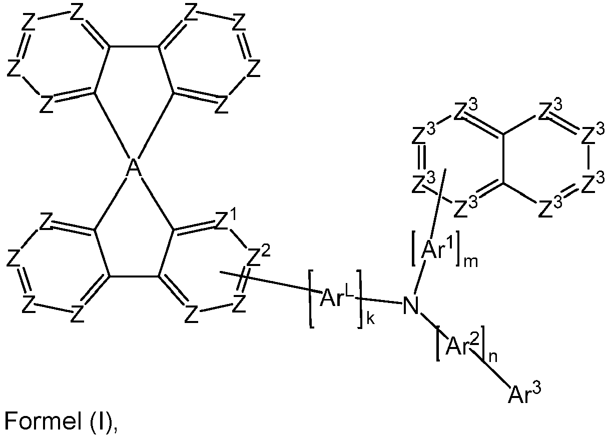
wobei die mit variabler Bindungsposition am Spirobifluoren gezeichnete Aminogruppe in Formel (I) entweder an Z¹ oder an Z² gebunden ist; und
wobei für die auftretenden Gruppen und Indices gilt:
   A ist gleich C oder Si;
   Ar^{L} ist bei jedem Auftreten gleich oder verschieden gewählt aus aromatischen Ringsystemen mit 6 bis 40 aromatischen Ringatomen, die jeweils mit Resten R² substituiert sind, und heteroaromatischen Ringsystemen mit 5 bis 40 aromatischen Ringatomen, die jeweils mit Resten R² substituiert sind;
   Ar¹ ist bei jedem Auftreten gleich oder verschieden gewählt aus aromatischen Ringsystemen mit 6 bis 24 aromatischen Ringatomen, die mit Resten R³ substituiert sind, und heteroaromatischen Ringsystemen mit 5 bis 24 aromatischen Ringatomen, die mit Resten R³ substituiert sind;
   Ar² ist bei jedem Auftreten gleich oder verschieden gewählt aus Phenyl, das mit Resten R⁵ substituiert ist, Naphthyl, das mit Resten R⁵ substituiert ist, und heteroaromatischen Ringsystemen mit 6 bis 10 aromatischen Ringatomen, die jeweils mit Resten R⁵ substituiert sind;
   Ar³ ist gewählt aus Gruppen der Formeln (Ar³-1) bis (Ar³-7), die über die mit * markierte Bindung an den Rest der Formel (I) binden 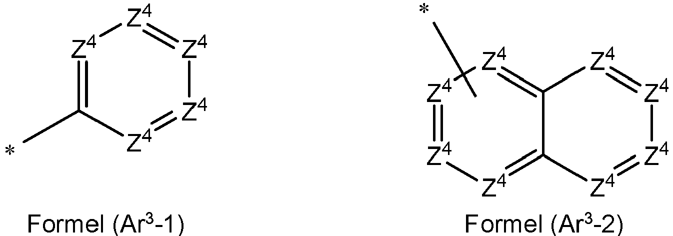 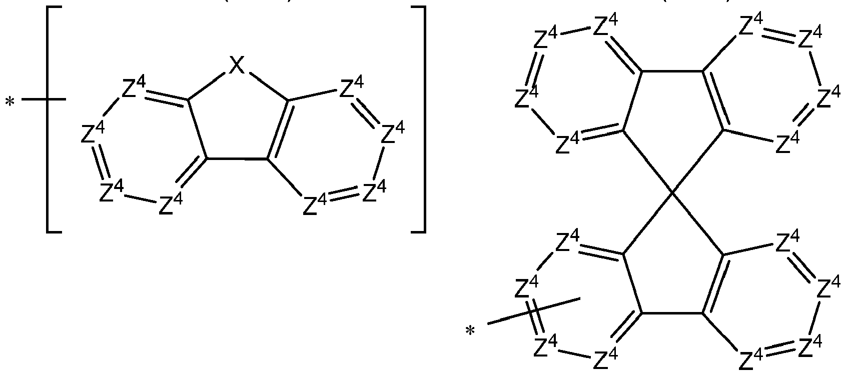 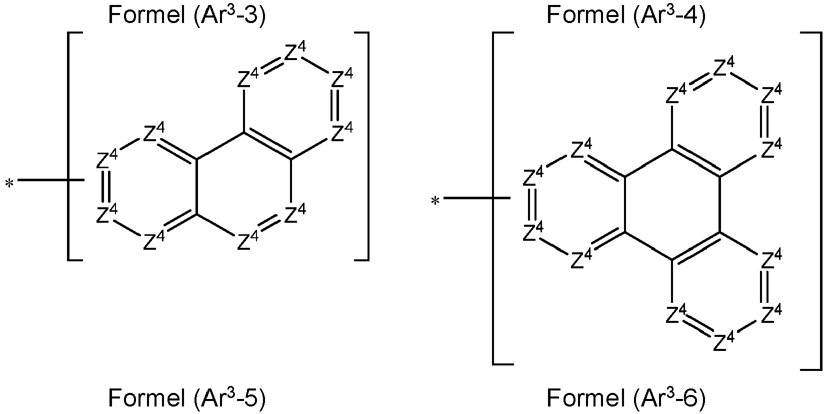 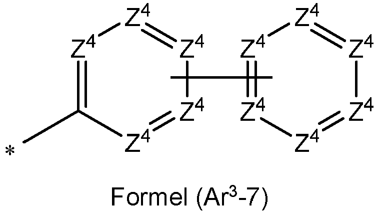
   wobei Z⁴ bei jedem Auftreten gleich oder verschieden gewählt ist aus CR⁶ und N, und wobei in Formeln (Ar³-2) bis (Ar³-7) diejenige Gruppe Z⁴ gleich C ist, die an eine frei gezeichnete Bindung bindet; wobei in Formel (Ar³-3) auch X gleich C(R⁶)₂ an den Rest der Formel (I) binden kann, wobei dann eine Gruppe R⁶ in dieser Gruppe X durch die Bindung an den Rest der Formel (I) ersetzt ist;
   und wobei X gewählt ist aus O, S, NR⁶ und C(R⁶)₂; und wobei in Formel (Ar³-3) eine oder mehrere Einheiten Z⁴=Z⁴ durch eine Einheit gewählt aus den Einheiten 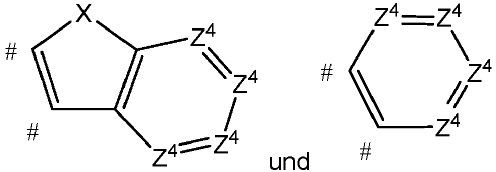
   ersetzt sein können, wobei die mit # markierten Atome jeweils die Atome Z⁴ ersetzen;
   und wobei in Formel (Ar³-3) eine oder mehrere Einheiten Z⁴-Z⁴ durch eine Einheit gewählt aus den Einheiten 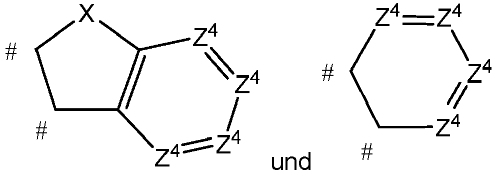
   ersetzt sein können, wobei die mit # markierten Atome jeweils die Atome Z⁴ ersetzen;
   Z ist bei jedem Auftreten gleich oder verschieden gewählt aus CR¹ und N;
   Z¹ und Z² sind jeweils C, wenn die mit variabler Bindungsposition gezeichnete Aminogruppe daran gebunden ist, und ansonsten gewählt aus CR¹ und N;
   Z³ ist bei jedem Auftreten gleich oder verschieden gewählt aus CR⁴ und N;
   R¹ ist bei jedem Auftreten gleich oder verschieden gewählt aus H, D, F, Cl, Br, I, C(=O)R⁷, CN, Si(R⁷)₃, N(R⁷)₂, -(Ar^{L})ₖ-N(Ar⁴)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹, R², R³, R⁴, R⁵ bzw. R⁶ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁷ substituiert sind; und wobei eine oder mehrere CH₂-Gruppen in den genannten Alkyl-, Alkoxy-, Alkenyl- und Alkinylgruppen durch - R⁷C=CR⁷-, -C≡C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
   R², R³, R⁴, R⁵ und R⁶ sind jeweils bei jedem Auftreten gleich oder verschieden gewählt aus H, D, F, Cl, Br, I, C(=O)R⁷, CN, Si(R⁷)₃, N(R⁷)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R², R³, R⁴, R⁵ bzw. R⁶ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁷ substituiert sind; und wobei eine oder mehrere CH₂-Gruppen in den genannten Alkyl-, Alkoxy-, Alkenyl- und Alkinylgruppen durch -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
   Ar⁴ ist bei jedem Auftreten gleich oder verschieden gewählt aus aromatischen Ringsystemen mit 6 bis 40 aromatischen Ringatomen, die mit einem oder mehreren Resten R⁷ substituiert sind, und heteroaromatischen Ringsystemen mit 5 bis 40 aromatischen Ringatomen, die mit einem oder mehreren Resten R⁷ substituiert sind;
   R⁷ ist bei jedem Auftreten gleich oder verschieden gewählt aus H, D, F, Cl, Br, I, C(=O)R⁸, CN, Si(R⁸)₃, N(R⁸)₂, P(=O)(R⁸)₂, OR⁸, S(=O)R⁸, S(=O)₂R⁸, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹, R², R³, R⁴, R⁵ bzw. R⁶ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁸ substituiert sind; und wobei eine oder mehrere CH₂-Gruppen in den genannten Alkyl-, Alkoxy-, Alkenyl- und Alkinylgruppen durch -R⁸C=CR⁸-, -C=C-, Si(R⁸)₂, C=O, C=NR⁸, -C(=O)O-, -C(=O)NR⁸-, NR⁸, P(=O)(R⁸), -O-, -S-, SO oder SO₂ ersetzt sein können
   R⁸ ist bei jedem Auftreten gleich oder verschieden gewählt aus H, D, F, Cl, Br, I, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁸ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit einem oder mehreren Resten gewählt aus F und CN substituiert sein können;
   k ist gleich 0, 1, 2, 3 oder 4;
   m ist gleich 0, 1, 2, 3 oder 4;
   n ist gleich 1, 2, 3 oder 4.

Die in eckigen Klammern stehenden Einheiten in Formeln (Ar³-3) und (Ar³-5) können an einer beliebigen ihrer Positionen an die mit * markierte Bindung gebunden sein.

Die folgenden Definitionen gelten für die chemischen Gruppen, die in der vorliegenden Anmeldung verwendet werden. Sie gelten, soweit keine spezielleren Definitionen angegeben sind.

Unter einer Arylgruppe im Sinne dieser Erfindung wird entweder ein einzelner aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einzelnen aromatischen Cyclen. Unter Kondensation zwischen Cyclen ist dabei zu verstehen, dass die Cyclen mindestens eine Kante miteinander teilen. Eine Arylgruppe im Sinne dieser Erfindung enthält 6 bis 40 aromatische Ringatome. Weiterhin enthält eine Arylgruppe kein Heteroatom als aromatisches Ringatom, sondern nur Kohlenstoffatome.

Unter einer Heteroarylgruppe im Sinne dieser Erfindung wird entweder ein einzelner heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einzelnen aromatischen oder heteroaromatischen Cyclen, wobei wenigstens einer der aromatischen und heteroaromatischen Cyclen ein heteroaromatischer Cyclus ist. Unter Kondensation zwischen Cyclen ist dabei zu verstehen, dass die Cyclen mindestens eine Kante miteinander teilen. Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S.

Unter einer Aryl- oder Heteroarylgruppe, die jeweils mit den oben genannten Resten substituiert sein kann werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Benzimidazolo[1,2-a]benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung ist ein System, welches nicht notwendigerweise nur Arylgruppen enthält, sondern welches zusätzlich einen oder mehrere nicht-aromatische Ringe enthalten kann, die mit wenigstens einer Arylgruppe kondensiert sind. Diese nicht-aromatischen Ringe enthalten ausschließlich Kohlenstoffatome als Ringatome. Beispiele für Gruppen, die von dieser Definition umfasst sind, sind Tetrahydronaphthalin, Fluoren und Spirobifluoren. Weiterhin umfasst der Begriff aromatisches Ringsystem Systeme, die aus zwei oder mehr aromatischen Ringsystemen bestehen, die über Einfachbindungen miteinander verbunden sind, beispielsweise Biphenyl, Terphenyl, 7-Phenyl-2-fluorenyl, Quaterphenyl und 3,5-Diphenyl-1-phenyl. Ein aromatisches Ringsystem im Sinne dieser Erfindung enthält 6 bis 40 C-Atome und keine Heteroatome im Ringsystem. Die Definition von "aromatisches Ringsystem" umfasst nicht Heteroarylgruppen.

Ein heteroaromatisches Ringsystem entspricht der oben genannten Definition eines aromatischen Ringsystems, mit dem Unterschied dass es mindestens ein Heteroatom als Ringatom enthalten muss. Wie es beim aromatischen Ringsystem der Fall ist, muss das heteroaromatische Ringsystem nicht ausschließlich Arylgruppen und Heteroarylgruppen enthalten, sondern es kann zusätzlich einen oder mehrere nicht-aromatische Ringe enthalten, die mit wenigstens einer Aryl- oder Heteroarylgruppe kondensiert sind. Die nicht-aromatischen Ringe können ausschließlich C-Atome als Ringatome enthalten, oder sie können zusätzlich ein oder mehrere Heteroatome enthalten, wobei die Heteroatome bevorzugt gewählt sind aus N, O und S. Ein Beispiel für ein derartiges heteroaromatisches Ringsystem ist Benzopyranyl. Weiterhin werden unter dem Begriff "heteroaromatisches Ringsystem" Systeme verstanden, die aus zwei oder mehr aromatischen oder heteroaromatischen Ringsystemen bestehen, die miteinander über Einfachbindungen verbunden sind, wie beispielsweise 4,6-Diphenyl-2-triazinyl. Ein heteroaromatisches Ringsystem im Sinne dieser Erfindung enthält 5 bis 40 Ringatome, die gewählt sind aus Kohlenstoff und Heteroatomen, wobei mindestens eines der Ringatome ein Heteroatom ist. Die Heteroatome des heteroaromatischen Ringsystems sind bevorzugt ausgewählt aus N, O und S.

Die Begriffe "heteroaromatisches Ringsystem" und "aromatisches Ringsystem" gemäß der Definition der vorliegenden Anmeldung unterscheiden sich damit dadurch voneinander, dass ein aromatisches Ringsystem kein Heteroatom als Ringatom aufweisen kann, während ein heteroaromatisches Ringsystem mindestens ein Heteroatom als Ringatom aufweisen muss. Dieses Heteroatom kann als Ringatom eines nicht-aromatischen heterocyclischen Rings oder als Ringatom eines aromatischen heterocyclischen Rings vorliegen.

Entsprechend der obenstehenden Definitionen ist jede Arylgruppe vom Begriff "aromatisches Ringsystem" umfasst, und jede Heteroarylgruppe ist vom Begriff "heteroaromatisches Ringsystem" umfasst.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Bevorzugt ist die Gruppe A gleich C.

Bevorzugt sind Z¹ und Z² jeweils C, wenn die mit variabler Bindungsposition gezeichnete Aminogruppe daran gebunden ist, und ansonsten gleich CR¹.

Bevorzugt ist Z³ gleich CR⁴.

Bevorzugt ist Z gleich CR¹.

Bevorzugt ist Z⁴ gleich CR⁶.

Besonders bevorzugt sind Z¹ und Z² jeweils C, wenn die mit variabler Bindungsposition gezeichnete Aminogruppe daran gebunden ist, und ansonsten gleich CR¹, Z ist gleich CR¹, Z³ ist gleich CR⁴, und Z⁴ ist gleich CR⁶.

Bevorzugt ist R¹ bei jedem Auftreten gleich oder verschieden gewählt aus H, D, F, CN, Si(R⁷)₃, N(R⁷)₂, -[Ar^{L}]ₖ-N(Ar⁴)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit Resten R⁷ substituiert sind; und wobei in den genannten Alkyl- oder Alkoxygruppen eine oder mehrere CH₂-Gruppen durch -C=C-, -R⁷C=CR⁷-, Si(R⁷)₂, C=O, C=NR⁷, -NR⁷-, -O-, -S-, -C(=O)O- oder -C(=O)NR⁷- ersetzt sein können. Besonders bevorzugt ist R¹ bei jedem Auftreten gleich oder verschieden gewählt aus H, -[Ar^{L}]ₖ-N(Ar⁴)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit Resten R⁷ substituiert sind. Ganz besonders bevorzugt ist R¹ gleich H.

Bevorzugte Gruppen R¹ in der Verbindung der Formel (I) sind gewählt aus den folgenden Gruppen:

| | | |
|---|---|---|
| 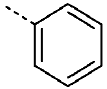 | 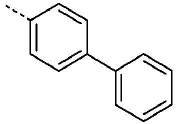 | 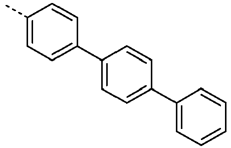 |
| R-1 | R-2 | R-3 |
| 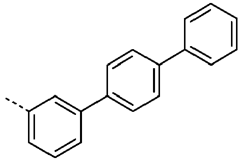 | 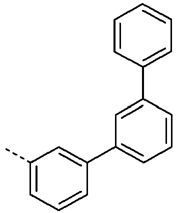 | 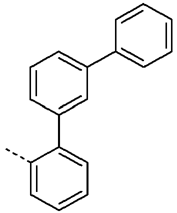 |
| R-4 | R-5 | R-6 |
| 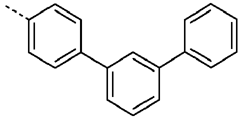 | 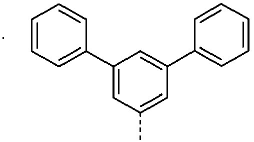 | 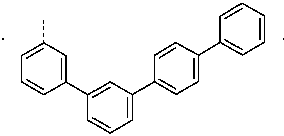 |
| R-7 | R-8 | R-9 |
| 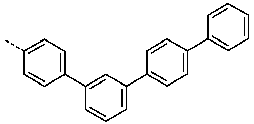 | 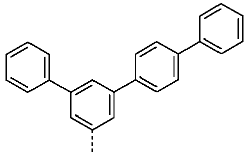 | 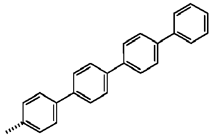 |
| R-10 | R-11 | R-12 |
| 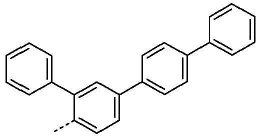 | 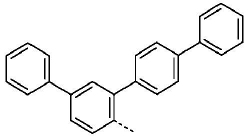 | 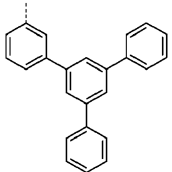 |
| R-13 | R-14 | R-15 |
| 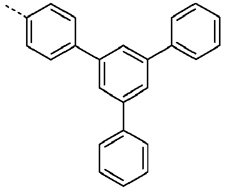 | 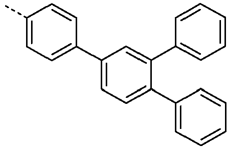 | 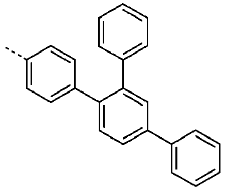 |
| R-16 | R-17 | R-18 |
| 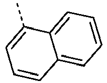 | 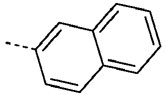 | 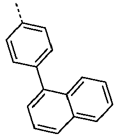 |
| R-19 | R-20 | R-21 |
| 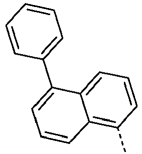 | 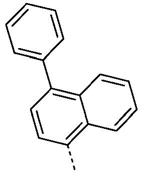 | 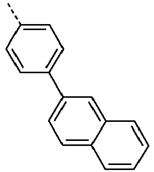 |
| R-22 | R-23 | R-24 |
| 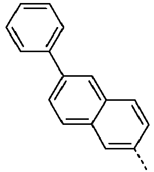 | 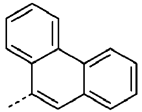 | 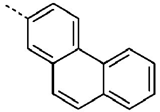 |
| R-25 | R-26 | R-27 |
| 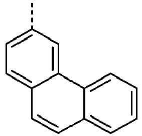 | 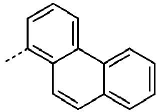 | 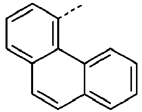 |
| R-28 | R-29 | R-30 |
| 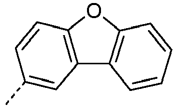 | 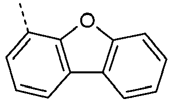 | 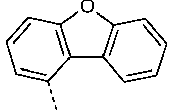 |
| R-31 | R-32 | R-33 |
| 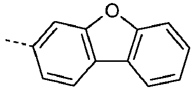 | 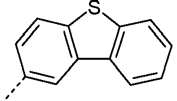 | 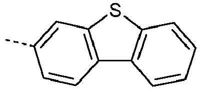 |
| R-34 | R-35 | R-36 |
| 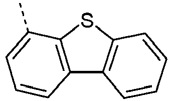 | 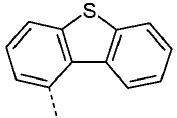 | 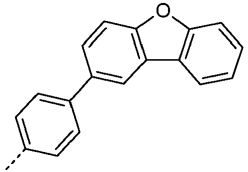 |
| R-37 | R-38 | R-39 |
| 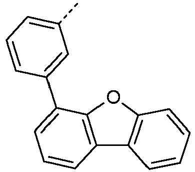 | 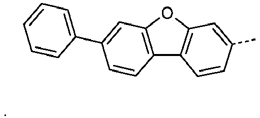 | 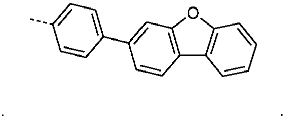 |
| R-40 | R-41 | R-42 |
| 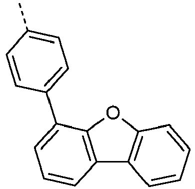 | 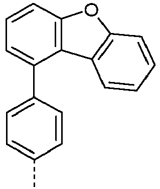 | 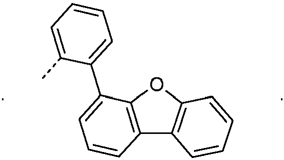 |
| R-43 | R-44 | R-45 |
| 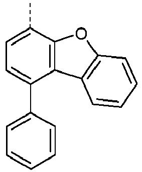 | 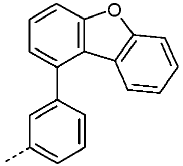 | 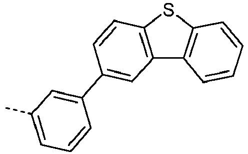 |
| R-46 | R-47 | R-48 |
| 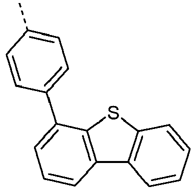 | 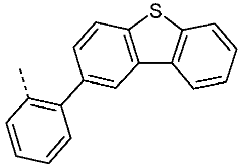 | 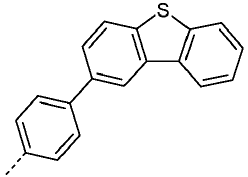 |
| R-49 | R-50 | R-51 |
| 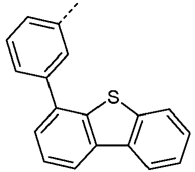 | 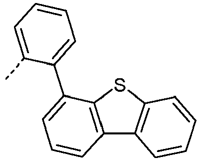 | 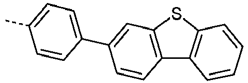 |
| R-52 | R-53 | R-54 |
| 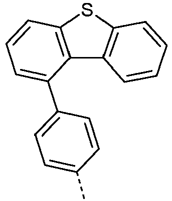 | 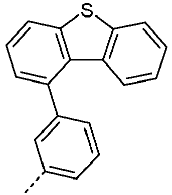 | 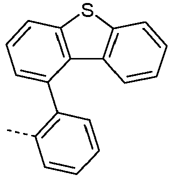 |
| R-55 | R-56 | R-57 |
| 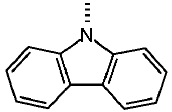 | 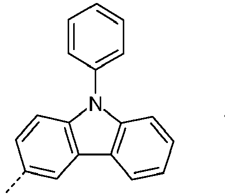 | 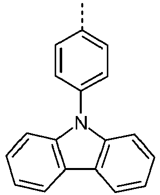 |
| R-58 | R-59 | R-60 |
| 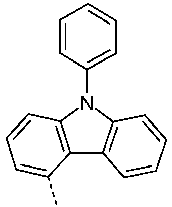 | 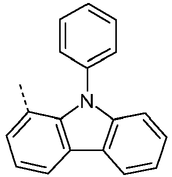 | 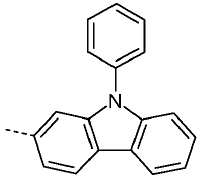 |
| R-61 | R-62 | R-63 |
| 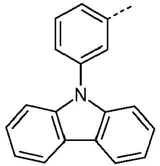 | 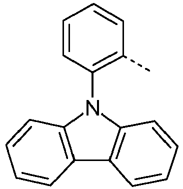 | 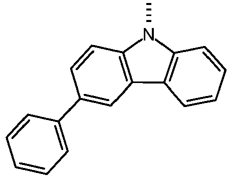 |
| R-64 | R-65 | R-66 |
| 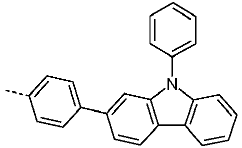 | 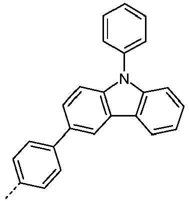 | 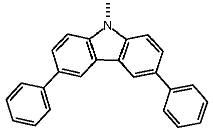 |
| R-67 | R-68 | R-69 |
| 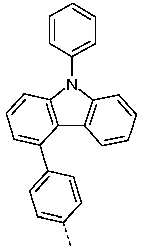 | 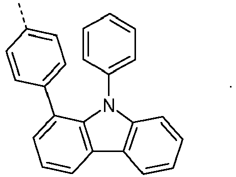 | 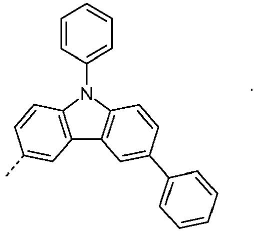 |
| R-70 | R-71 | R-72 |
| 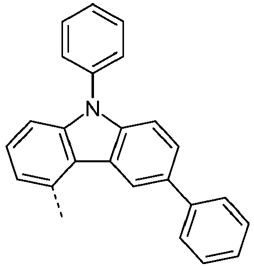 | 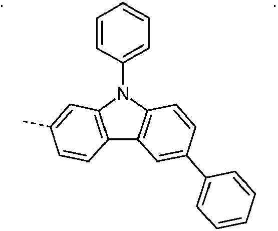 | 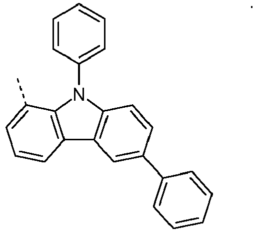 |
| R-73 | R-74 | R-75 |
| 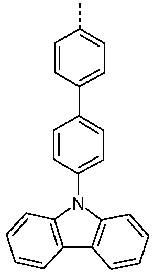 | 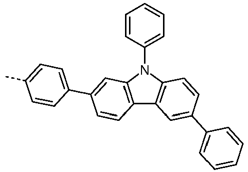 | 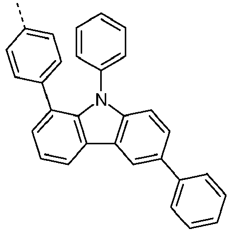 |
| R-76 | R-77 | R-78 |
| 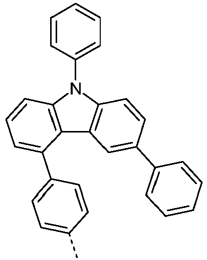 | 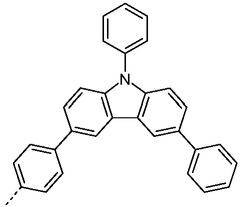 | 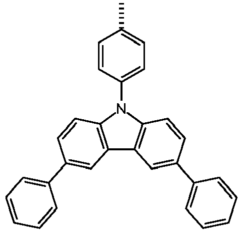 |
| R-79 | R-80 | R-81 |
| 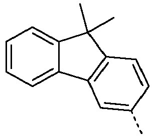 | 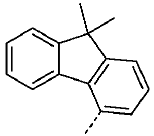 | 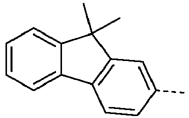 |
| R-82 | R-83 | R-84 |
| 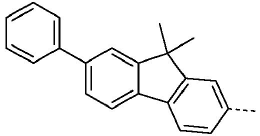 | 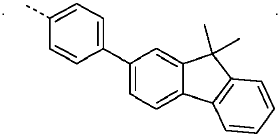 | 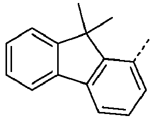 |
| R-85 | R-86 | R-87 |
| 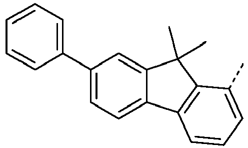 | 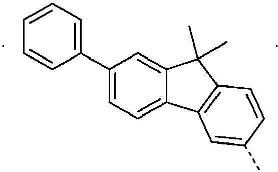 | 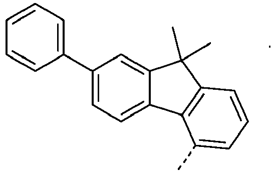 |
| R-88 | R-89 | R-90 |
| 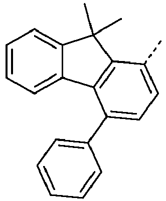 | 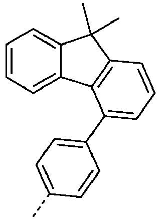 | 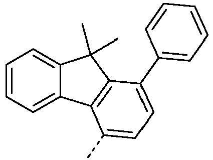 |
| R-91 | R-92 | R-93 |
| 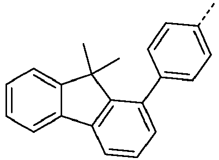 | 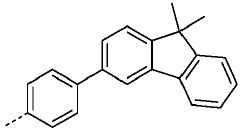 | 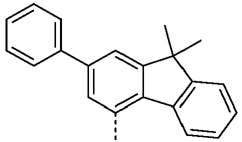 |
| R-94 | R-95 | R-96 |
| 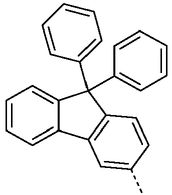 | 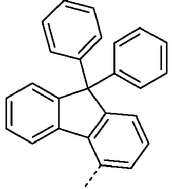 | 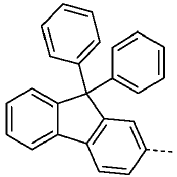 |
| R-97 | R-98 | R-99 |
| 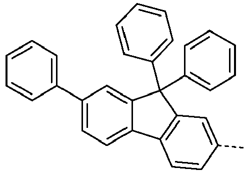 | 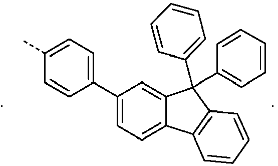 | 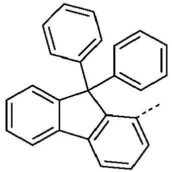 |
| R-100 | R-101 | R-102 |
| 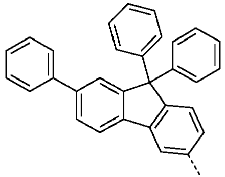 | 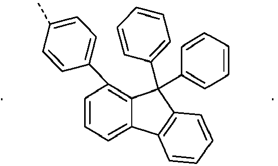 | 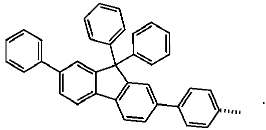 |
| R-103 | R-104 | R-105 |
| 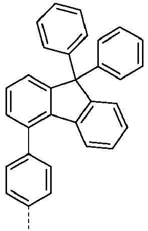 | 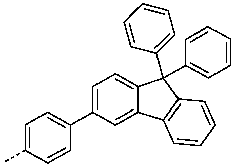 | 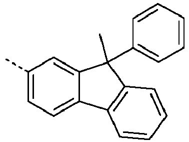 |
| R-106 | R-107 | R-108 |
| 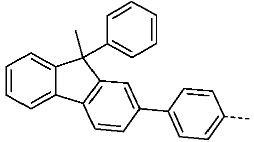 | 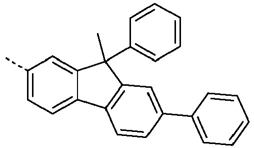 | 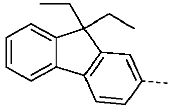 |
| R-109 | R-110 | R-111 |
| 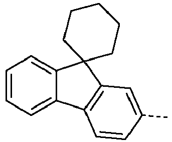 | 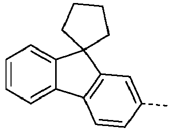 | 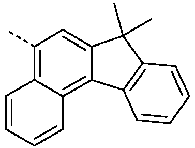 |
| R-112 | R-113 | R-114 |
| 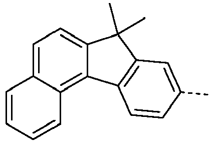 | 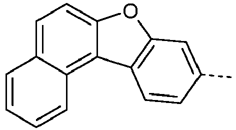 | 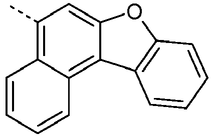 |
| R-115 | R-116 | R-117 |
| 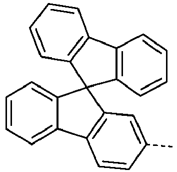 | 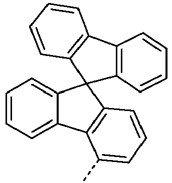 | 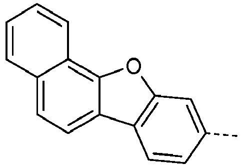 |
| R-118 | R-119 | R-120 |
| 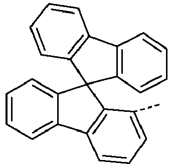 | 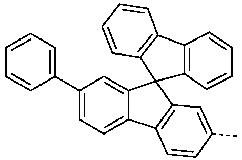 | 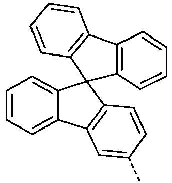 |
| R-121 | R-122 | R-123 |
| 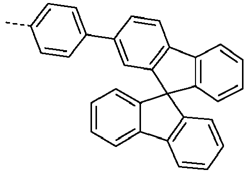 | 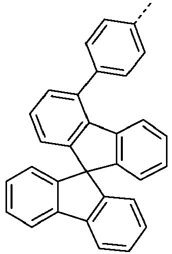 | 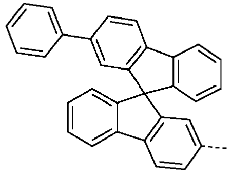 |
| R-124 | R-125 | R-126 |
| 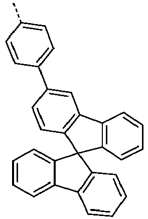 | 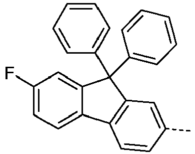 | 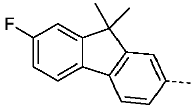 |
| R-127 | R-128 | R-129 |
| 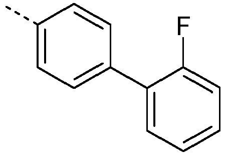 | 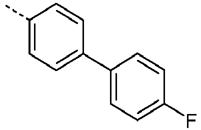 | 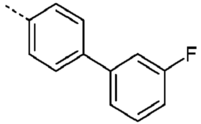 |
| R-130 | R-131 | R-132 |
| 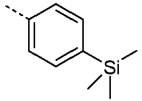 | 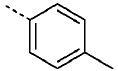 | 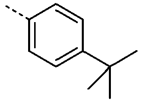 |
| R-133 | R-134 | R-135 |
| 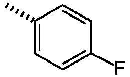 | 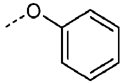 | 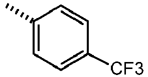 |
| R-136 | R-137 | R-138 |
| 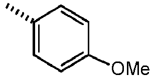 | 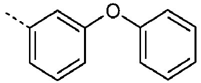 | 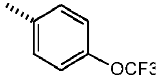 |
| R-139 | R-140 | R-141 |
| 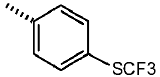 | -F | -Cl |
| R-142 | R-143 | R-144 |
| -Br | -I | -H |
| R-145 | R-146 | R-147 |
| -CH3 | 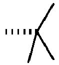 | -CH₂CH₃ |
| R-148 | R-149 | R-150 |
| 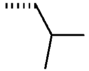 | -CF₃ | -CF₂CF₃ |
| R-151 | R-152 | R-153 |
| -OCF₃ | -SCF₃ | -SF₅ |
| R-154 | R-155 | R-156 |
| -OCF₂CF₃ | -SCF₂CF₃ | 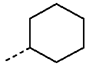 |
| R-157 | R-158 | R-159 |
| 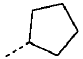 | 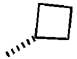 | 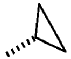 |
| R-160 | R-161 | R-162 |
| -CN | -SCN | -OCH₂CH₃ |
| R-163 | R-164 | R-165 |
| -OCH₃ | -SCH₃ | -Si(CH₃)₃ |
| R-166 | R-167 | R-168 |
| -Si(CH₃)₂t-Bu | -Si(iPr)₃ | -Si(CH₃)₂Ph |
| R-169 | R-170 | R-171 |
| Si-(Ph)₃ | C-(Ph)₃ | -D |
| R-172 | R-173 | R-174 |
| -CD₃ | -CD₂-CD₃ | -C(CD₃)₃ |
| R-175 | R-176 | R-177 |
| -CD₂-(CD₃)₂ | -OCD₃ | -SCD₃ |
| R-178 | R-179 | R-180 |
| -Si(CD₃)₃ | 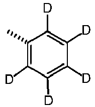 | 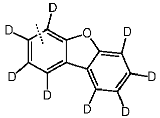 |
| R-181 | R-182 | R-183 |
| 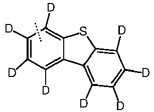 | 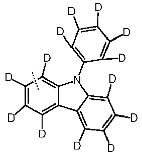 | -CD₂(CH₃)₂ |
| R-184 | R-185 | R-186 |
| -CD₂(CH₃)₃ | -[Ar^{L}]ₖ-N(Ar⁴)2 | |
| R-187 | R-188 | |

Besonders bevorzugt sind darunter die Formeln R-1, R-2, R-143, R-147, R-148, R-149, R-154.

Bevorzugt sind R², R³, R⁴, R⁵ und R⁶ bei jedem Auftreten gleich oder verschieden gewählt aus H, D, F, CN, Si(R⁷)₃, N(R⁷)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit Resten R⁷ substituiert sind; und wobei in den genannten Alkyl- oder Alkoxygruppen eine oder mehrere CH₂-Gruppen durch -C=C-, -R⁷C=CR⁷-, Si(R⁷)₂, C=O, C=NR⁷, -NR⁷-, -O-, -S-, -C(=O)O- oder -C(=O)NR⁷- ersetzt sein können.

Gemäß einer bevorzugten Ausführungsform enthält die Verbindung mindestens eine, bevorzugt eine Gruppe R⁴, die gewählt ist aus aromatischen Ringsystemen mit 6 bis 40 aromatischen Ringatomen, und heteroaromatischen Ringsystemen mit 5 bis 40 aromatischen Ringatome, die jeweils mit Resten R⁷ substituiert sind. Besonders bevorzugt sind Phenyl, Biphenyl, Terphenyl, Naphthyl, Phenanthrenyl, Fluorenyl, Carbazolyl, Dibenzofuranyl, Dibenzothiophenyl, die jeweils mit Resten R⁷ substituiert sind.

Bevorzugt ist R⁷ bei jedem Auftreten gleich oder verschieden gewählt aus H, D, F, CN, Si(R⁸)₃, N(R⁸)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit Resten R⁸ substituiert sind; und wobei in den genannten Alkyl- oder Alkoxygruppen eine oder mehrere CH₂-Gruppen durch -C=C-, -R⁸C=CR⁸-, Si(R⁸)₂, C=O, C=NR⁸, -NR⁸-, -O-, -S-, -C(=O)O- oder -C(=O)NR⁸- ersetzt sein können.

Index k ist bevorzugt gewählt aus 0, 1 und 2, besonders bevorzugt gewählt aus 0 und 1, ganz besonders bevorzugt ist Index k gleich 0.

Ar^{L} ist bevorzugt bei jedem Auftreten gleich oder verschieden gewählt aus aromatischen oder heteroaromatischen Ringen mit 6 aromatischen Ringatomen und aromatischen oder heteroaromatischen Ringsystemen mit 10 aromatischen Ringatomen, besonders bevorzugt gewählt aus Phenyl, Biphenyl, Naphthyl und Fluorenyl, die jeweils mit Resten R² substituiert sind; ganz besonders besonders bevorzugt gewählt aus Phenyl, das mit Resten R² substituiert ist.

Ar^{L} ist bevorzugt bei jedem Auftreten gleich oder verschieden gewählt aus Gruppen der folgenden Formeln:

| | | |
|---|---|---|
| 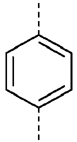 | 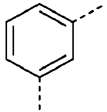 | 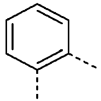 |
| Ar^{L}-23 | Ar^{L}-24 | Ar^{L}-25 |
| 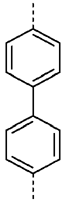 | 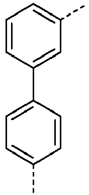 | 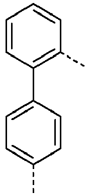 |
| Ar^{L}-26 | Ar^{L}-27 | Ar^{L}-28 |
| 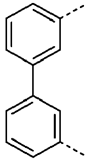 | 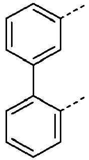 | 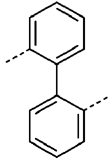 |
| Ar^{L}-29 | Ar^{L}-30 | Ar^{L}-31 |
| 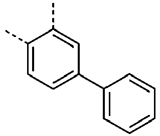 | 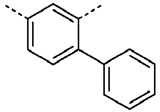 | 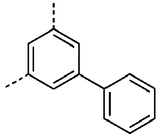 |
| Ar^{L}-32 | Ar^{L}-33 | Ar^{L}-34 |
| 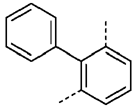 | 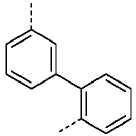 | 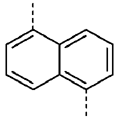 |
| Ar^{L}-35 | Ar^{L}-36 | Ar^{L}-37 |
| 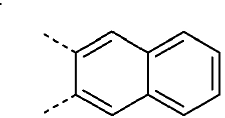 | 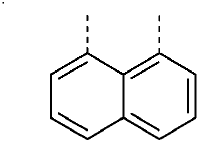 | 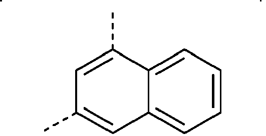 |
| Ar^{L}-38 | Ar^{L}-39 | Ar^{L}-40 |
| 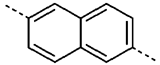 | 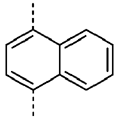 | 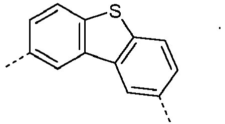 |
| Ar^{L}-41 | Ar^{L}-42 | Ar^{L}-43 |
| 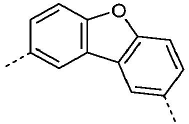 | 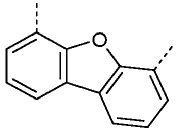 | 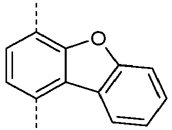 |
| Ar^{L}-44 | Ar^{L}-45 | Ar^{L}-46 |
| 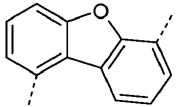 | 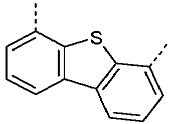 | 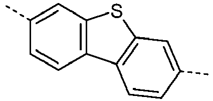 |
| Ar^{L}-47 | Ar^{L}-48 | Ar^{L}-49 |
| 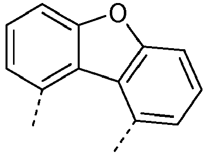 | 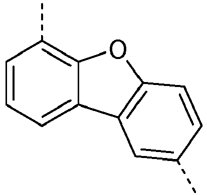 | 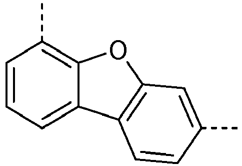 |
| Ar^{L}-50 | Ar^{L}-51 | Ar^{L}-52 |
| 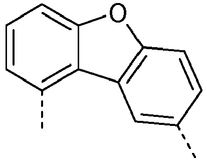 | 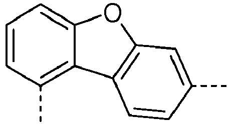 | 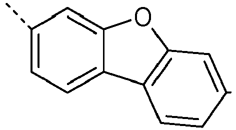 |
| Ar^{L}-53 | Ar^{L}-54 | Ar^{L}-55 |
| 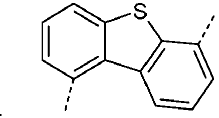 | 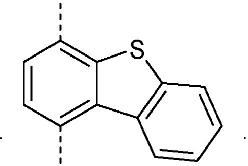 | 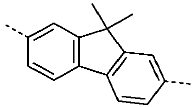 |
| Ar^{L}-56 | Ar^{L}-57 | Ar^{L}-58 |
| 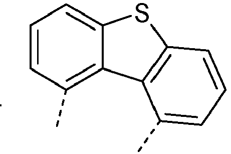 | 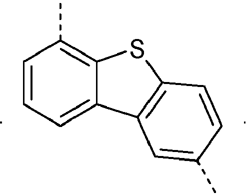 | 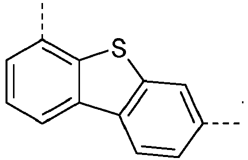 |
| Ar^{L}-59 | Ar^{L}-60 | Ar^{L}-61 |
| 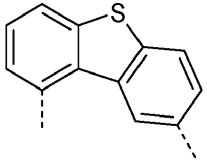 | 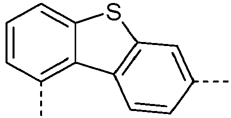 | 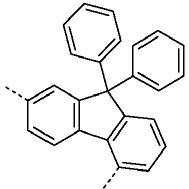 |
| Ar^{L}-62 | Ar^{L}-63 | Ar^{L}-64 |
| 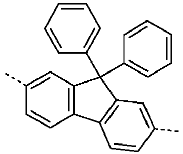 | 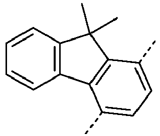 | 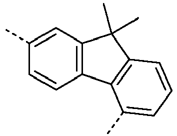 |
| Ar^{L}-65 | Ar^{L}-66 | Ar^{L}-67 |
| 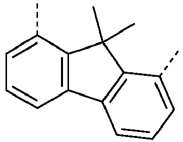 | 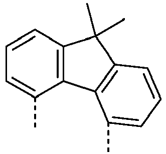 | 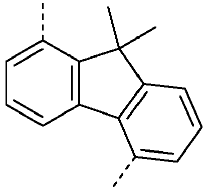 |
| Ar^{L}-68 | Ar^{L}-69 | Ar^{L}-70 |
| 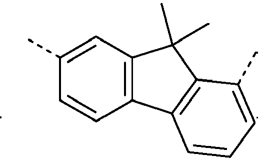 | 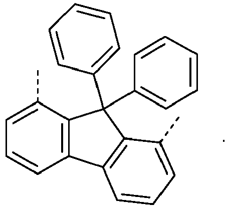 | 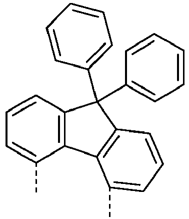 |
| Ar^{L}-71 | Ar^{L}-72 | Ar^{L}-73 |
| 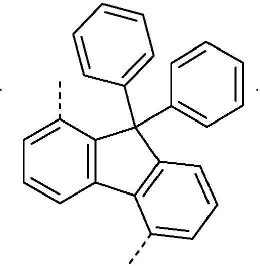 | 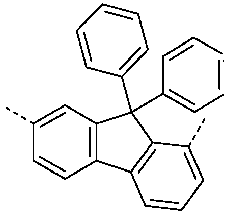 | 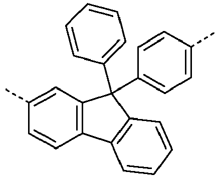 |
| Ar^{L}-74 | Ar^{L}-75 | Ar^{L}-76 |
| 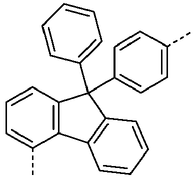 | 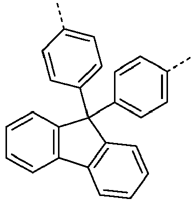 | 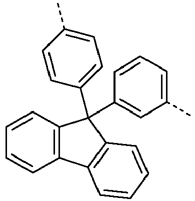 |
| Ar^{L}-77 | Ar^{L}-78 | Ar^{L}-79 |
| 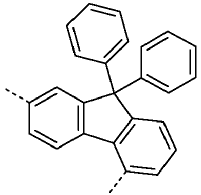 | 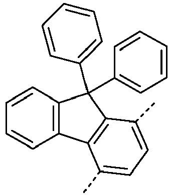 | 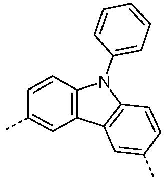 |
| Ar^{L}-80 | Ar^{L}-81 | Ar^{L}-82 |
| 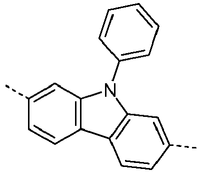 | 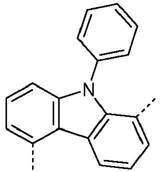 | 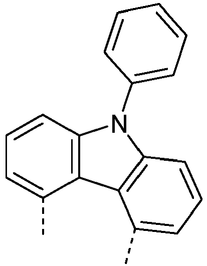 |
| Ar^{L}-83 | Ar^{L}-84 | Ar^{L}-85 |
| 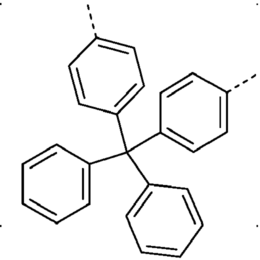 | 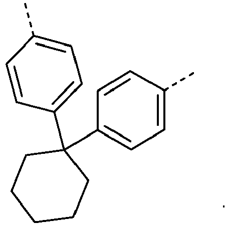 | 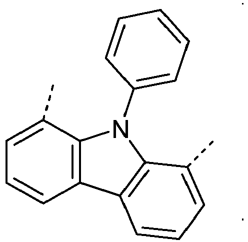 |
| Ar^{L}-86 | Ar^{L}-87 | Ar^{L}-88 |
| 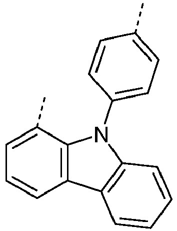 | 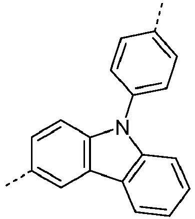 | 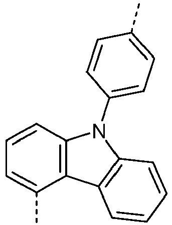 |
| Ar^{L}-89 | Ar^{L}-90 | Ar^{L}-91 |
| 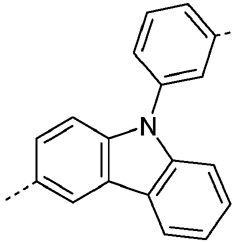 | 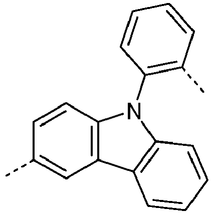 | 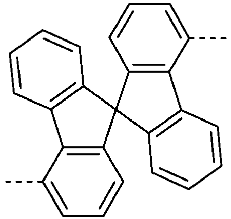 |
| Ar^{L}-92 | Ar^{L}-93 | Ar^{L}-94 |
| 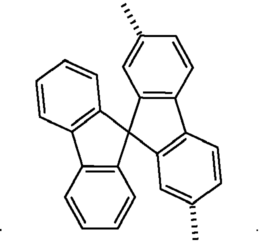 | 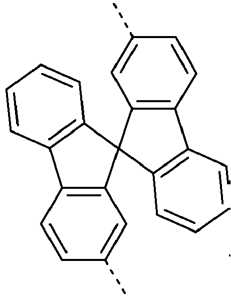 | 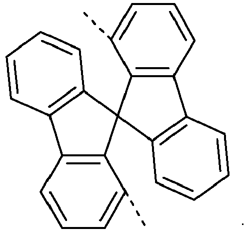 |
| Ar^{L}-95 | Ar^{L}-96 | Ar^{L}-97 |
| 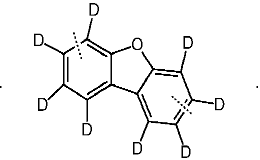 | 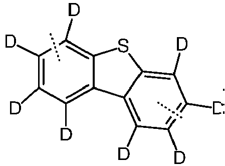 | 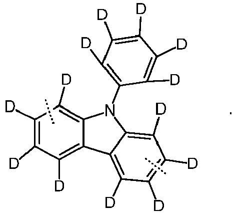 |
| Ar^{L}-98 | Ar^{L}-99 | Ar^{L}-100 |
| 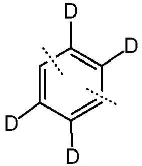 | 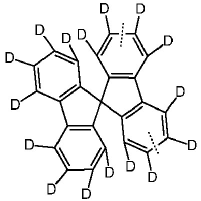 | 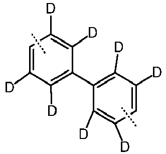 |
| Ar^{L}-101 | Ar^{L}-102 | Ar^{L}-103 |
| 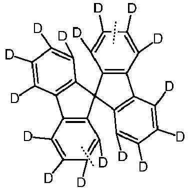 | | |
| Ar^{L}-104 | | |

wobei die gestrichelten Linien die Bindungen an den Rest der Formel darstellen. Unter den oben genannten Formeln sind die Formeln Ar^{L}-23, Ar^{L}-24, Ar^{L}-25, Ar^{L}-26, Ar^{L}-37, Ar^{L}-42, Ar^{L}-47, Ar^{L}-58 besonders bevorzugt.

Bevorzugt ist Index m gleich 0 oder 1. Gemäß einer alternativen bevorzugten Ausführungsform ist Index m gleich 2 oder 3, besonders bevorzugt gleich 2.

Bevorzugt ist Ar¹ bei jedem Auftreten gleich oder verschieden gewählt aus aromatischen oder heteroaromatischen Ringsystemen mit 6 aromatischen Ringatomen, die mit Resten R³ substituiert sind, aus aromatischen oder heteroaromatischen Ringsystemen mit 10 aromatischen Ringatomen, die mit Resten R³ substituiert sind, und aus Fluorenyl, das mit Resten R³ substituiert ist. Besonders bevorzugt ist Ar¹ bei jedem Auftreten gleich oder verschieden gewählt aus Phenyl, Naphthyl, Fluorenyl, und Biphenyl, die jeweils mit Resten R³ substituiert sind. Ganz besonders bevorzugt ist Ar³ gleich Phenyl, das mit Resten R³ substituiert ist.

Die Einheit 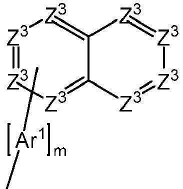 ist bevorzugt eine Einheit 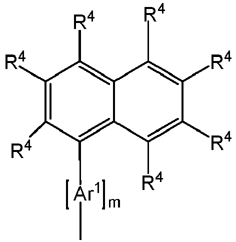 , so dass die Verbindungen der Formel (I) einer bevorzugten Formel (I-sub) entsprechen:

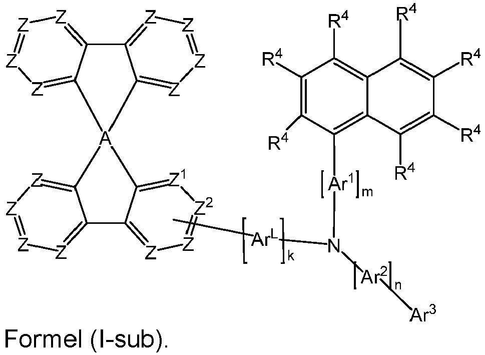

Die Einheit 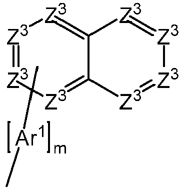 ist besonders bevorzugt gewählt aus Einheiten einer Formel

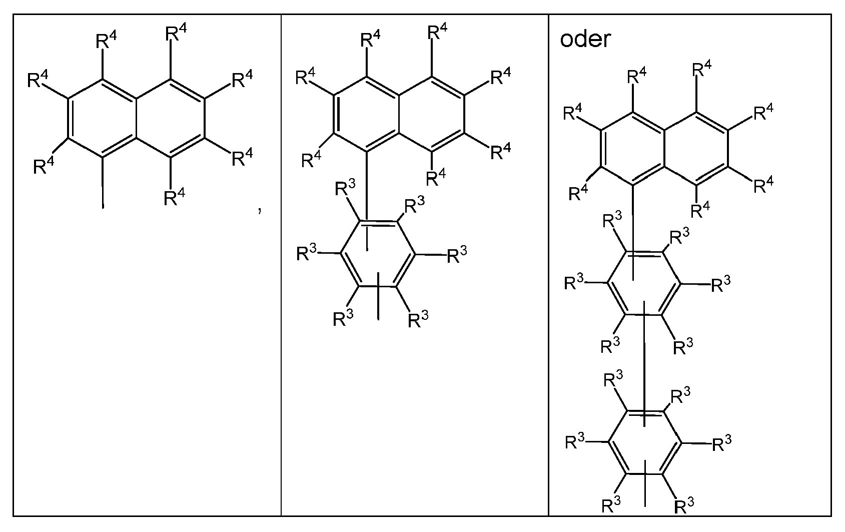

, so dass die Verbindungen der Formel (I) einer bevorzugten Formel (I-sub-1), (I-sub-2) oder (I-sub-3) entsprechen:

| |
|---|
| 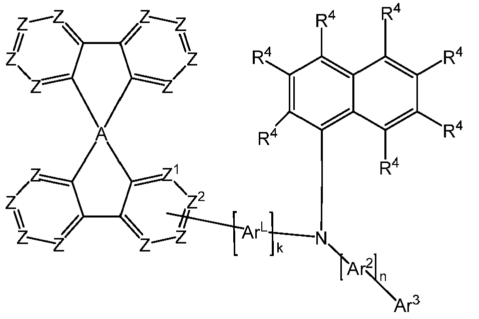 |
| Formel (I-sub-1) |
| 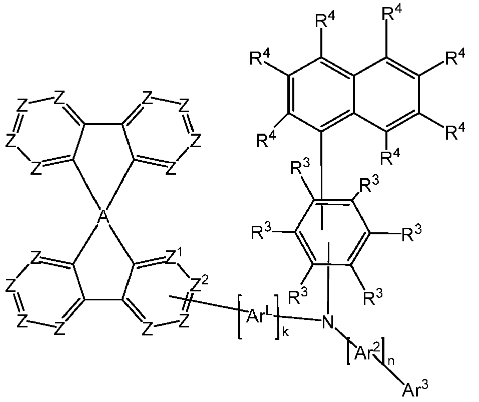 |
| Formel (I-sub-2) |
| 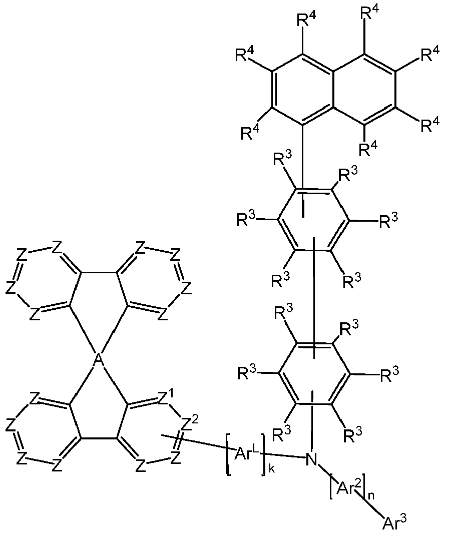 |
| Formel (I-sub-3). |

Bevorzugte Einheiten 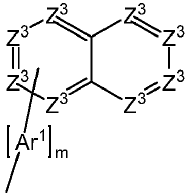 sind gewählt aus den folgenden:

| | | |
|---|---|---|
| 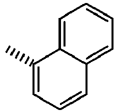 | 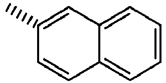 | 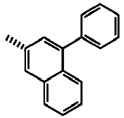 |
| Naph-1 | Naph-2 | Naph-3 |
| 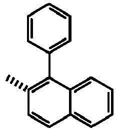 | 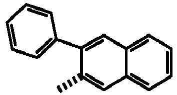 | 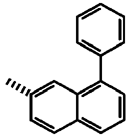 |
| Naph-4 | Naph-5 | Naph-6 |
| 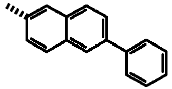 | 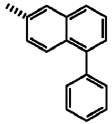 | 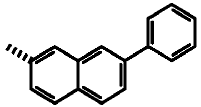 |
| Naph-7 | Naph-8 | Naph-9 |
| 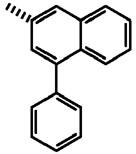 | 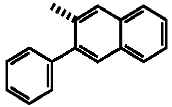 | 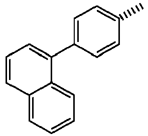 |
| Naph-10 | Naph-11 | Naph-12 |
| 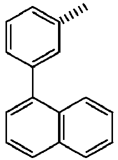 | 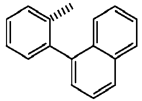 | 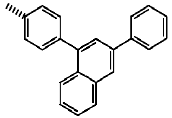 |
| Naph-13 | Naph-14 | Naph-15 |
| 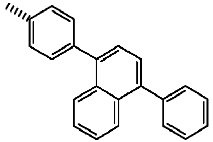 | 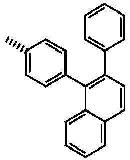 | 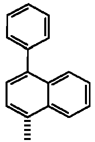 |
| Naph-16 | Naph-17 | Naph-18 |
| 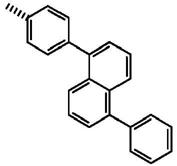 | 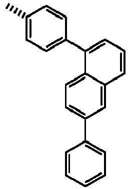 | 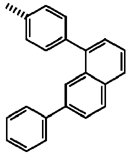 |
| Naph-19 | Naph-20 | Naph-21 |
| 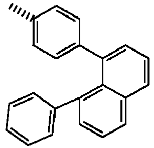 | 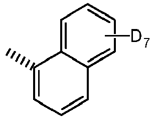 | 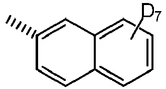 |
| Naph-22 | Naph-23 | Naph-24 |
| 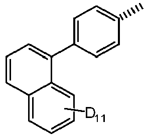 | 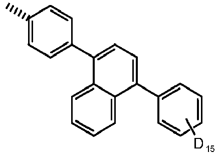 | 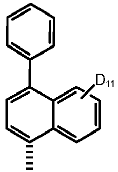 |
| Naph-25 | Naph-26 | Naph-27 |
| 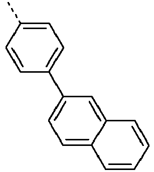 | 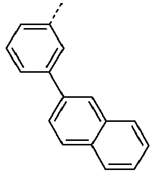 | 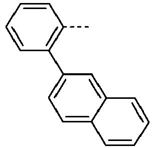 |
| Naph-28 | Naph-29 | Naph-30 |
| 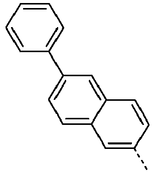 | 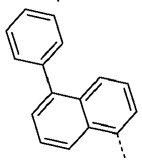 | 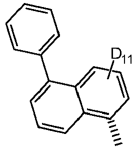 |
| Naph-31 | Naph-32 | Naph-33 |
| 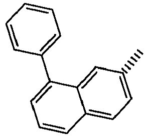 | 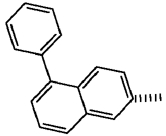 | 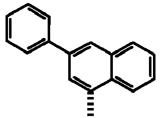 |
| Naph-34 | Naph-35 | Naph-36 |
| 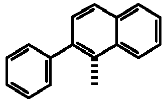 | 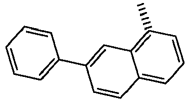 | 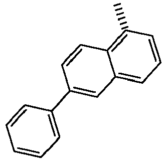 |
| Naph-37 | Naph-38 | Naph-39 |
| 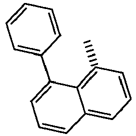 | 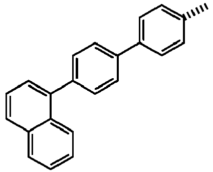 | 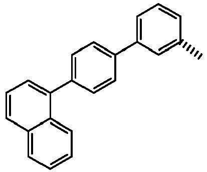 |
| Naph-40 | Naph-41 | Naph-42 |
| 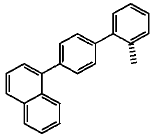 | 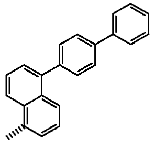 | 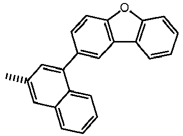 |
| Naph-43 | Naph-44 | Naph-45 |
| 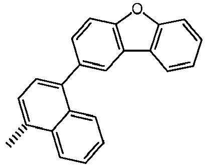 | 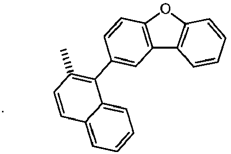 | 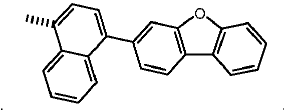 |
| Naph-46 | Naph-47 | Naph-48 |
| 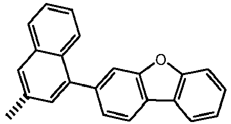 | 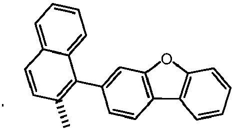 | 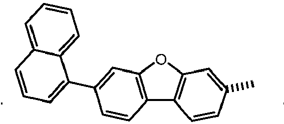 |
| Naph-49 | Naph-50 | Naph-51 |
| 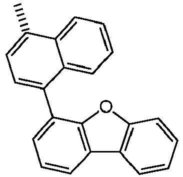 | 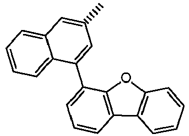 | 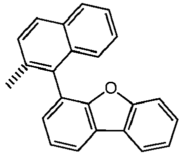 |
| Naph-52 | Naph-53 | Naph-54 |
| 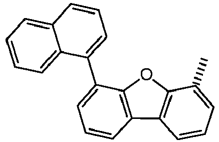 | 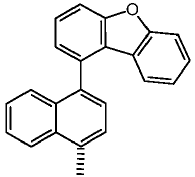 | 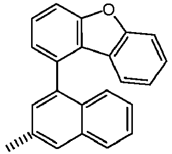 |
| Naph-55 | Naph-56 | Naph-57 |
| 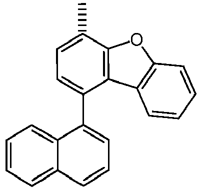 | 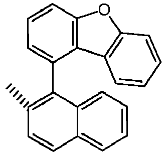 | 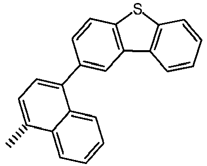 |
| Naph-58 | Naph-59 | Naph-60 |
| 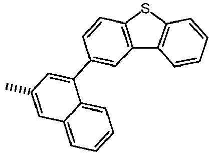 | 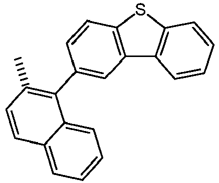 | 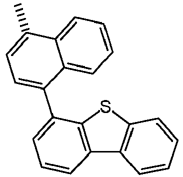 |
| Naph-61 | Naph-62 | Naph-63 |
| 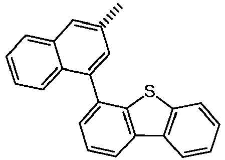 | 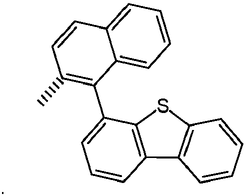 | 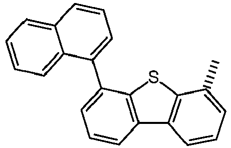 |
| Naph-64 | Naph-65 | Naph-66 |
| 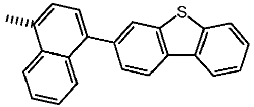 | 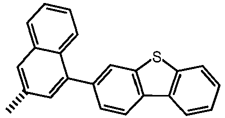 | 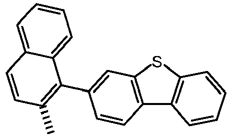 |
| Naph-67 | Naph-68 | Naph-69 |
| 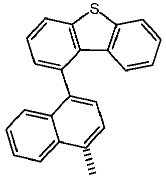 | 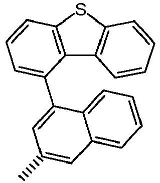 | 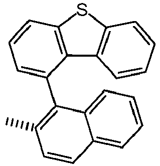 |
| Naph-70 | Naph-71 | Naph-72 |
| 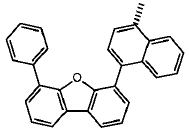 | 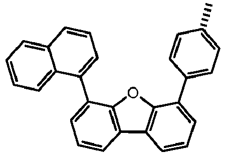 | 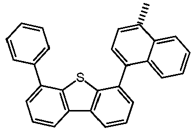 |
| Naph-73 | Naph-74 | Naph-75 |
| 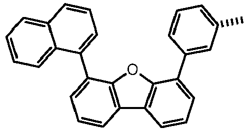 | 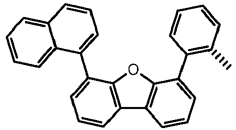 | 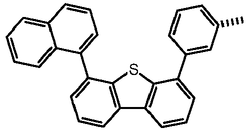 |
| Naph-76 | Naph-77 | Naph-78 |
| 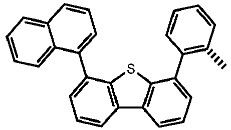 | 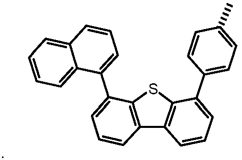 | 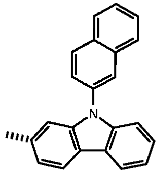 |
| Naph-79 | Naph-80 | Naph-81 |
| 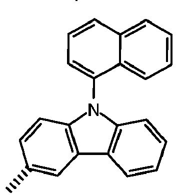 | 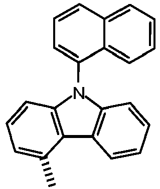 | 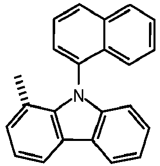 |
| Naph-82 | Naph-83 | Naph-84 |
| 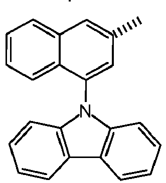 | 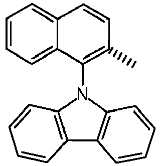 | 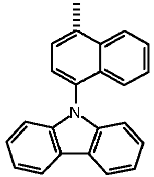 |
| Naph-85 | Naph-86 | Naph-87 |
| 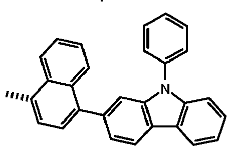 | 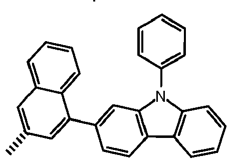 | 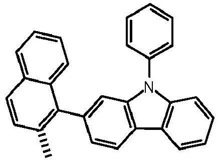 |
| Naph-88 | Naph-89 | Naph-90 |
| 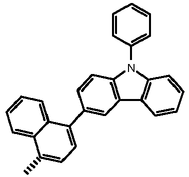 | 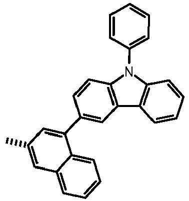 | 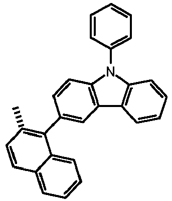 |
| Naph-91 | Naph-92 | Naph-93 |
| 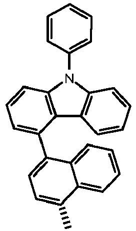 | 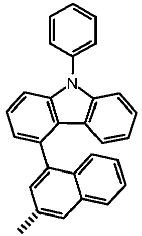 | 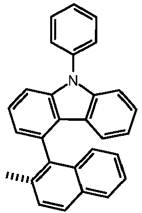 |
| Naph-94 | Naph-95 | Naph-96 |
| 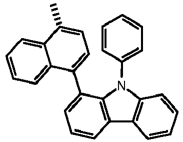 | 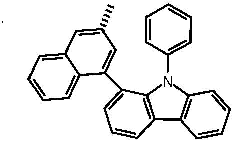 | 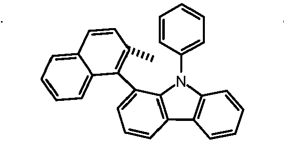 |
| Naph-97 | Naph-98 | Naph-99 |
| 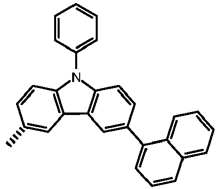 | 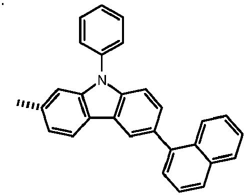 | 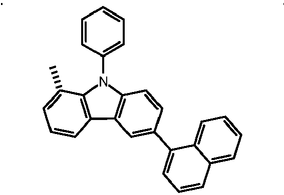 |
| Naph-100 | Naph-101 | Naph-102 |
| 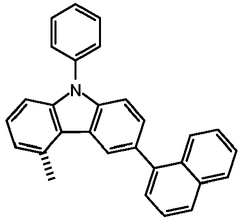 | 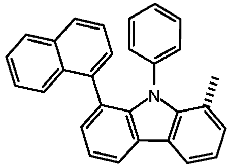 | 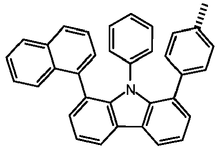 |
| Naph-103 | Naph-104 | Naph-105 |
| 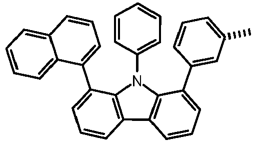 | 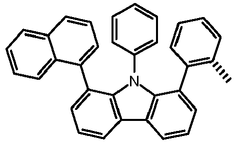 | 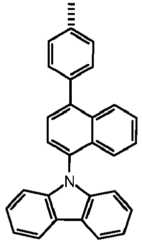 |
| Naph-106 | Naph-107 | Naph-108 |
| 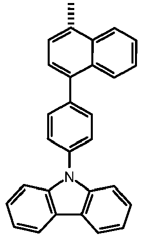 | 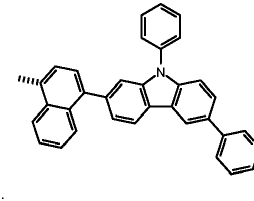 | 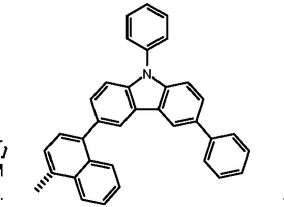 |
| Naph-109 | Naph-110 | Naph-111 |
| 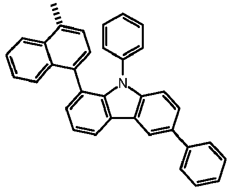 | 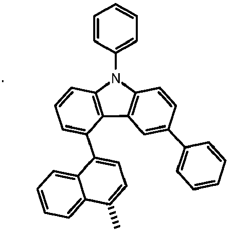 | 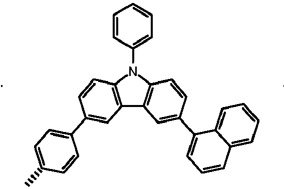 |
| Naph-112 | Naph-113 | Naph-114 |
| 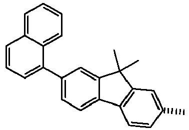 | 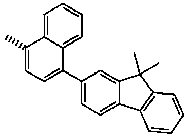 | 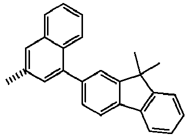 |
| Naph-115 | Naph-116 | Naph-117 |
| 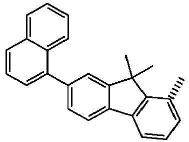 | 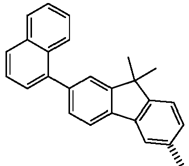 | 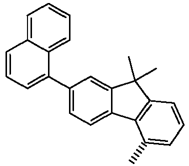 |
| Naph-118 | Naph-119 | Naph-120 |
| 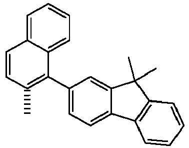 | 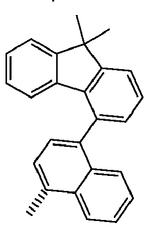 | 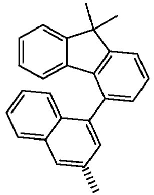 |
| Naph-121 | Naph-122 | Naph-123 |
| 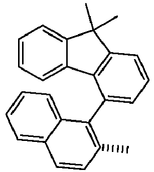 | 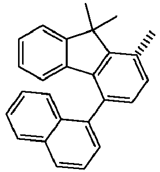 | 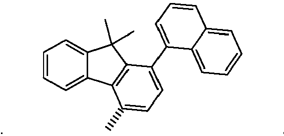 |
| Naph-124 | Naph-125 | Naph-126 |
| 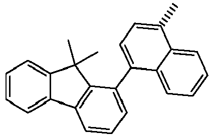 | 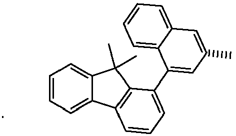 | 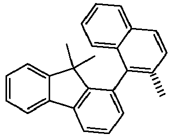 |
| Naph-127 | Naph-128 | Naph-129 |
| 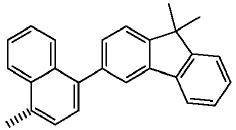 | 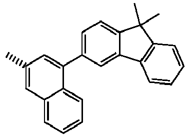 | 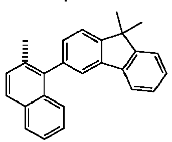 |
| Naph-130 | Naph-131 | Naph-132 |
| 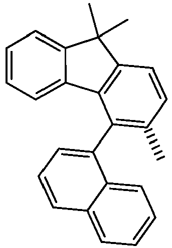 | 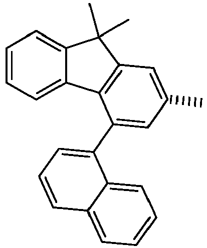 | 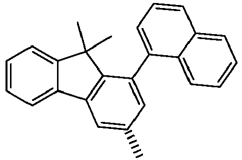 |
| Naph-133 | Naph-134 | Naph-135 |
| 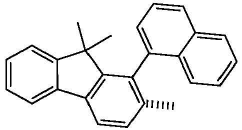 | 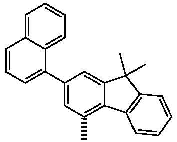 | 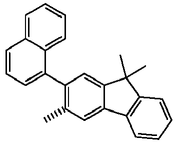 |
| Naph-136 | Naph-137 | Naph-138 |
| 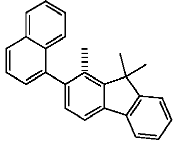 | 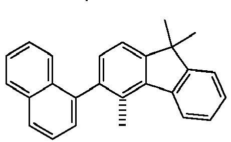 | 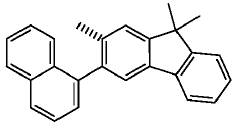 |
| Naph-139 | Naph-140 | Naph-141 |
| 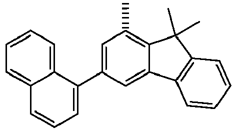 | 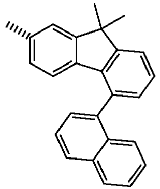 | 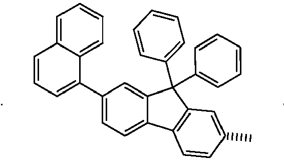 |
| Naph-142 | Naph-143 | Naph-144 |
| 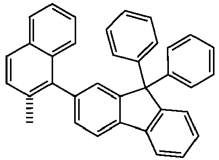 | 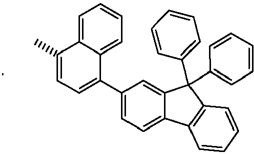 | 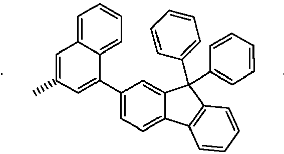 |
| Naph-145 | Naph-146 | Naph-147 |
| 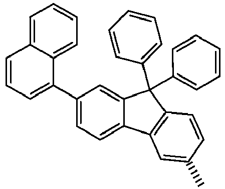 | 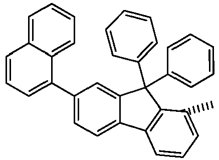 | 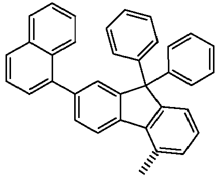 |
| Naph-148 | Naph-149 | Naph-150 |
| 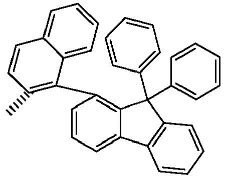 | 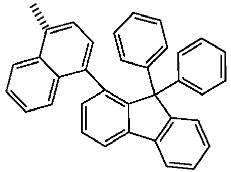 | 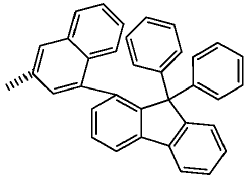 |
| Naph-151 | Naph-152 | Naph-153 |
| 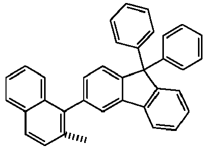 | 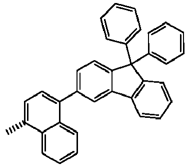 | 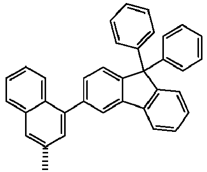 |
| Naph-154 | Naph-155 | Naph-156 |
| 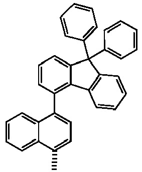 | 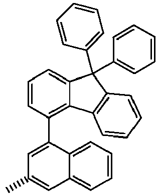 | 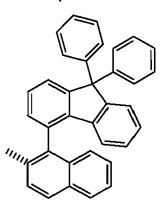 |
| Naph-157 | Naph-158 | Naph-159 |
| 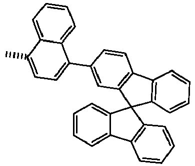 | 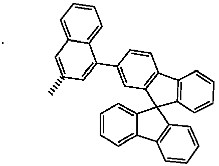 | 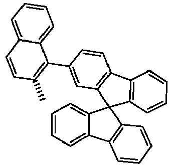 |
| Naph-160 | Naph-161 | Naph-162 |
| 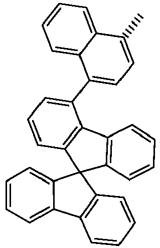 | 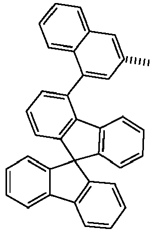 | 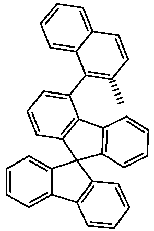 |
| Naph-163 | Naph-164 | Naph-165 |
| 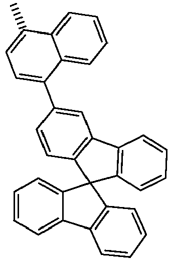 | 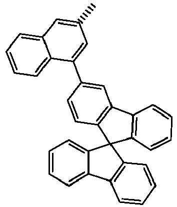 | 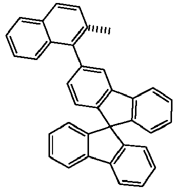 |
| Naph-166 | Naph-167 | Naph-168 |
| 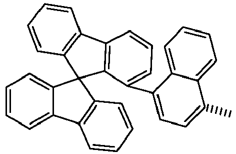 | 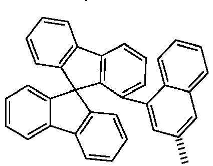 | 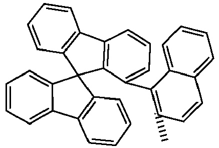 |
| Naph-169 | Naph-170 | Naph-171 |
| 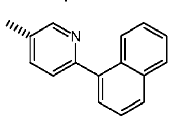 | 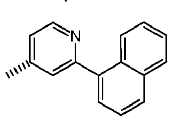 | 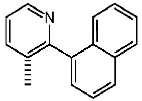 |
| Naph-172 | Naph-173 | Naph-174 |
| 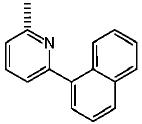 | 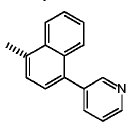 | 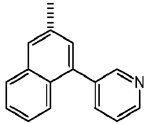 |
| Naph-175 | Naph-176 | Naph-177 |
| 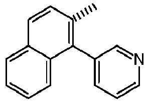 | 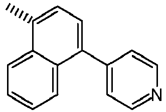 | 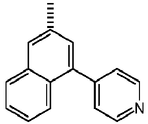 |
| Naph-178 | Naph-179 | Naph-180 |
| 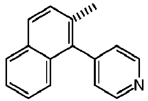 | 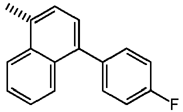 | 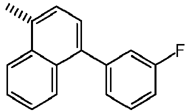 |
| Naph-181 | Naph-182 | Naph-183 |
| 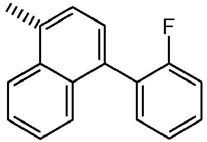 | 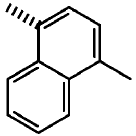 | 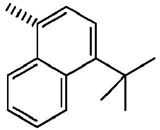 |
| Naph-184 | Naph-185 | Naph-186 |
| 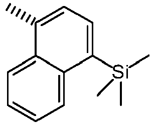 | 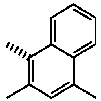 | 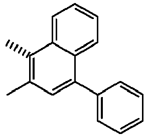 |
| Naph-187 | Naph-188 | Naph-189 |
| 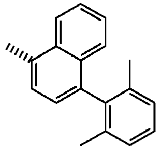 | 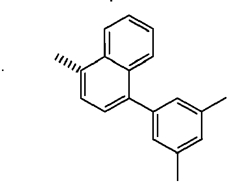 | 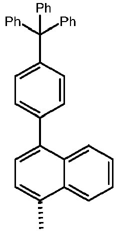 |
| Naph-190 | Naph-191 | Naph-192 |
| 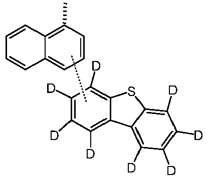 | 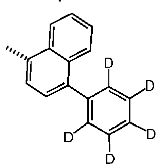 | 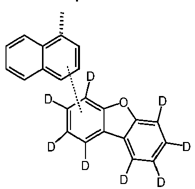 |
| Naph-193 | Naph-194 | Naph-195 |
| 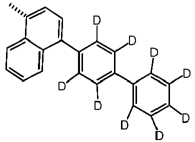 | 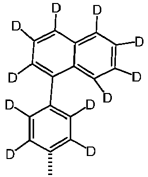 | |
| Naph-196 | Naph-197 | |

Unter diesen Gruppen sind die Gruppen Naph-1, Naph-12, Naph-18, Naph-23, Naph-25, Naph-26, Naph-27, Naph-41, Naph-52, Naph-56, Naph-82, Naph-87, Naph-108, Naph-109, Naph-116, Naph-122, Naph-194, Naph-195, Naph-197 besonders bevorzugt.

Bevorzugt ist Ar² bei jedem Auftreten gleich oder verschieden gewählt aus Phenyl und Naphthyl, die jeweils mit Resten R⁵ substituiert sind, ganz besonders bevorzugt Phenyl, das mit Resten R⁵ substituiert ist. Phenyl kann dabei ortho-Phenylen, meta-Phenylen oder para-Phenylen sein, wobei para-Phenylen bevorzugt ist.

Bevorzugt ist n gleich 1 oder 2.

Ganz besonders bevorzugt ist die Einheit -[Ar²]ₙ- gleich Phenylen, das mit Resten R⁵ substituiert ist, oder gleich Biphenylen, das mit Resten R⁵ substituiert. Dabei sind para-Phenyleneinheiten bevorzugt gegenüber meta- oder ortho-Phenyleneinheiten.

Bevorzugt ist Ar³ gewählt aus Gruppen der Formeln (Ar³-2), (Ar³-3) und (Ar³-4), besonders bevorzugt Gruppen der Formeln (Ar³-2) und (Ar³-3). In diesen Fällen ist bevorzugt Z⁴ gleich CR⁶, bzw. C, wenn die Bindung an den Rest der Formel über dieses Z⁴ erfolgt.

Bevorzugte Gruppen Ar³ sind gewählt aus den folgenden Gruppen:

| | | |
|---|---|---|
| 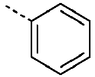 | 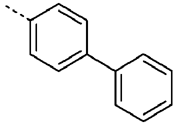 | 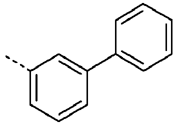 |
| Ar³-1 | Ar³-2 | Ar³-3 |
| 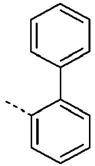 | 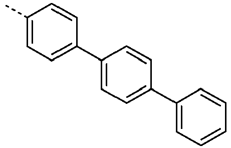 | 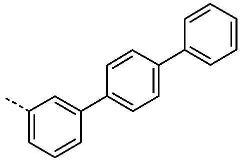 |
| Ar³-4 | Ar³-5 | Ar³-6 |
| 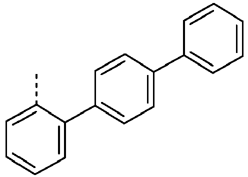 | 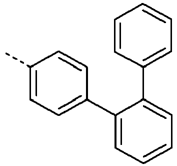 | 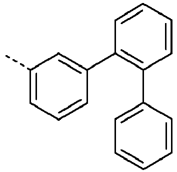 |
| Ar³-7 | Ar³-8 | Ar³-9 |
| 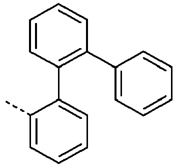 | 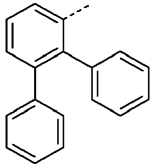 | 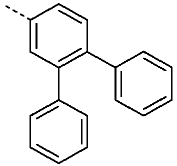 |
| Ar³-10 | Ar³-11 | Ar³-12 |
| 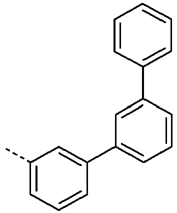 | 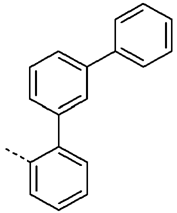 | 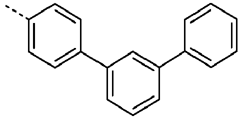 |
| Ar³-13 | Ar³-14 | Ar³-15 |
| 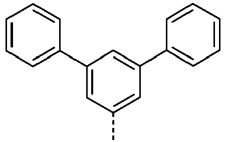 | 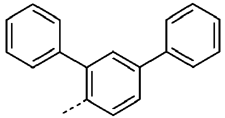 | 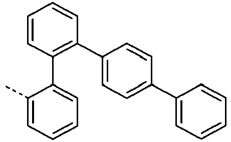 |
| Ar³-16 | Ar³-17 | Ar³-18 |
| 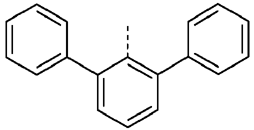 | 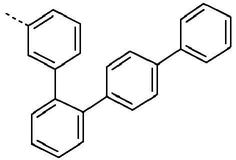 | 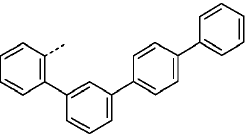 |
| Ar³-19 | Ar³-20 | Ar³-21 |
| 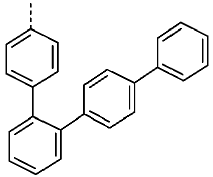 | 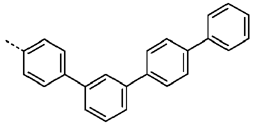 | 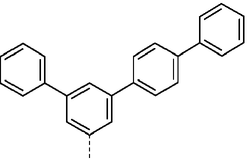 |
| Ar³-22 | Ar³-23 | Ar³-24 |
| 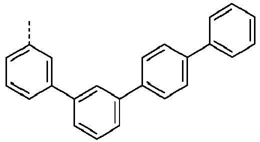 | 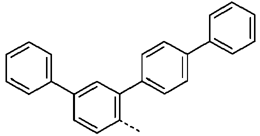 | 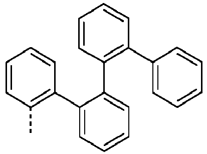 |
| Ar³-25 | Ar³-26 | Ar³-27 |
| 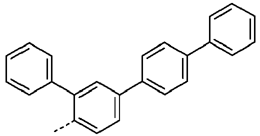 | 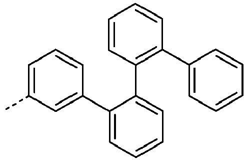 | 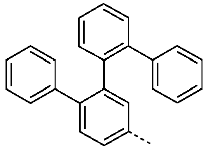 |
| Ar³-28 | Ar³-29 | Ar³-30 |
| 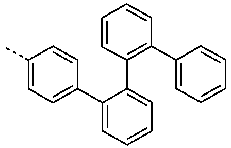 | 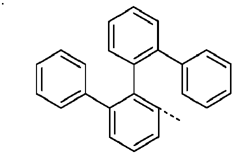 | 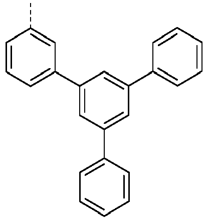 |
| Ar³-31 | Ar³-32 | Ar³-33 |
| 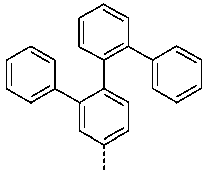 | 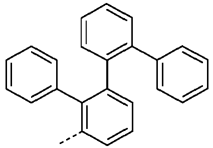 | 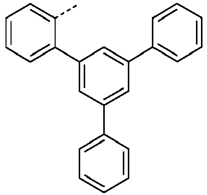 |
| Ar³-34 | Ar³-35 | Ar³-36 |
| 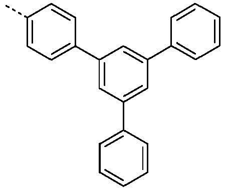 | 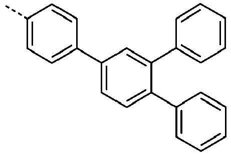 | 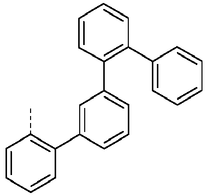 |
| Ar³-37 | Ar³-38 | Ar³-39 |
| 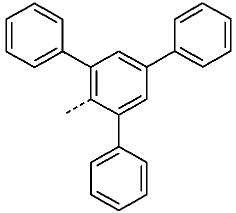 | 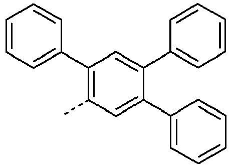 | 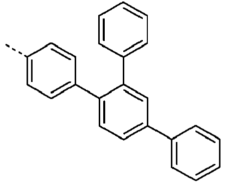 |
| Ar³-40 | Ar³-41 | Ar³-42 |
| 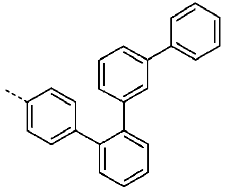 | 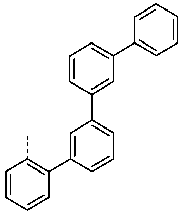 | 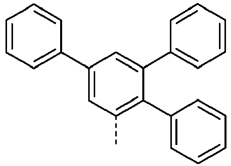 |
| Ar³-43 | Ar³-44 | Ar³-45 |
| 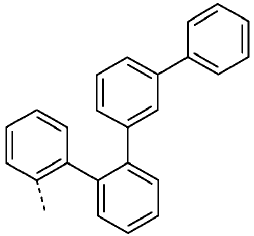 | 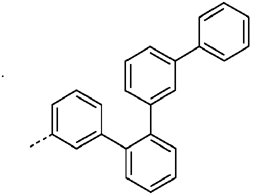 | 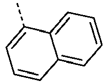 |
| Ar³-46 | Ar³-47 | Ar³-48 |
| 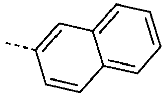 | 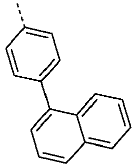 | 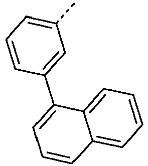 |
| Ar³-49 | Ar³-50 | Ar³-51 |
| 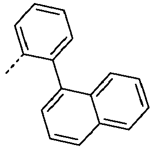 | 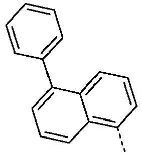 | 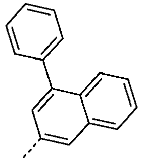 |
| Ar³-52 | Ar³-53 | Ar³-54 |
| 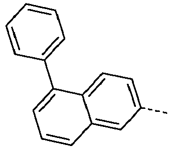 | 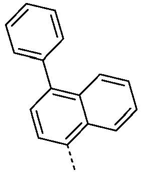 | 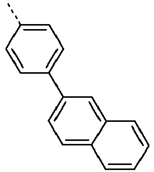 |
| Ar³-55 | Ar³-56 | Ar³-57 |
| 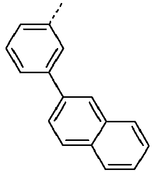 | 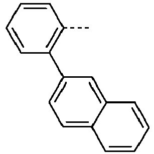 | 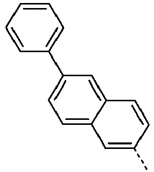 |
| Ar³-58 | Ar³-59 | Ar³-60 |
| 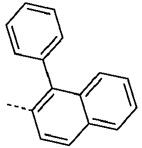 | 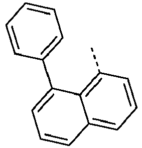 | 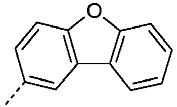 |
| Ar³-61 | Ar³-62 | Ar³-63 |
| 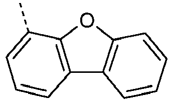 | 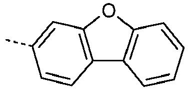 | 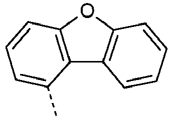 |
| Ar³-64 | Ar³-65 | Ar³-66 |
| 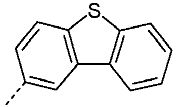 | 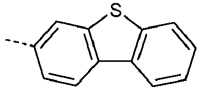 | 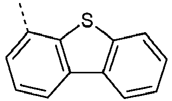 |
| Ar³-67 | Ar³-68 | Ar³-69 |
| 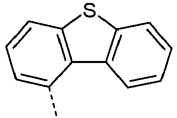 | 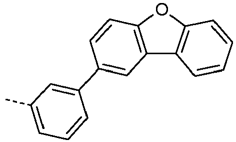 | 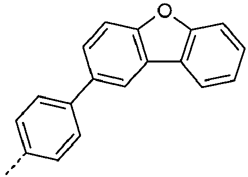 |
| Ar³-70 | Ar³-71 | Ar³-72 |
| 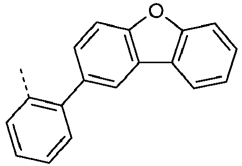 | 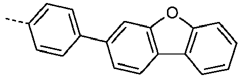 | 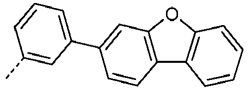 |
| Ar³-73 | Ar³-74 | Ar³-75 |
| 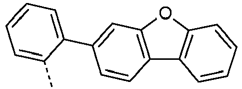 | 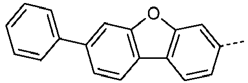 | 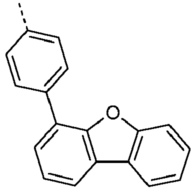 |
| Ar³-76 | Ar³-77 | Ar³-78 |
| 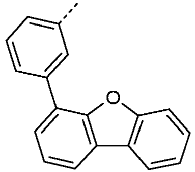 | 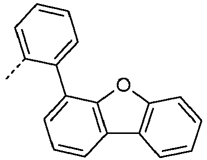 | 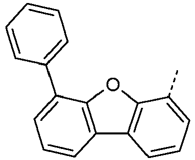 |
| Ar³-79 | Ar³-80 | Ar³-81 |
| 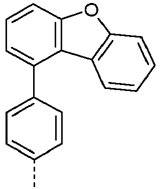 | 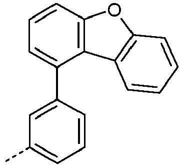 | 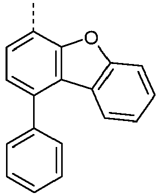 |
| Ar³-82 | Ar³-83 | Ar³-84 |
| 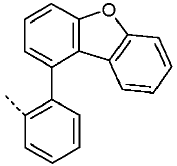 | 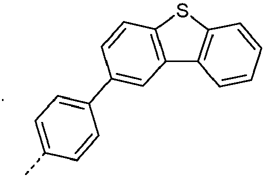 | 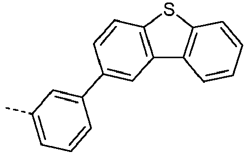 |
| Ar³-85 | Ar³-86 | Ar³-87 |
| 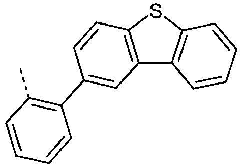 | 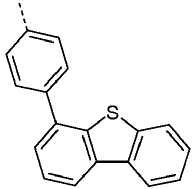 | 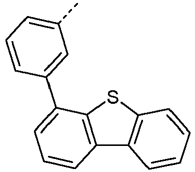 |
| Ar³-88 | Ar³-89 | Ar³-90 |
| 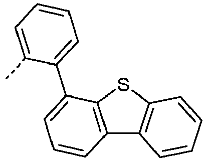 | 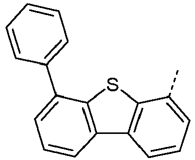 | 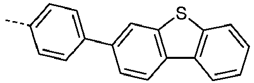 |
| Ar³-91 | Ar³-92 | Ar³-93 |
| 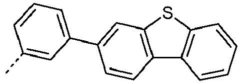 | 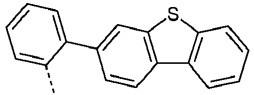 | 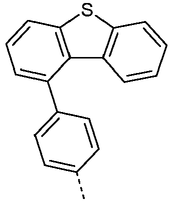 |
| Ar³-94 | Ar³-95 | Ar³-96 |
| 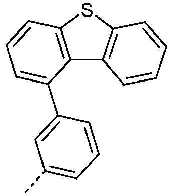 | 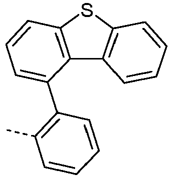 | 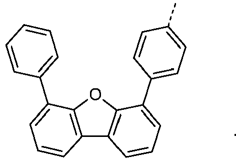 |
| Ar³-97 | Ar³-98 | Ar³-99 |
| 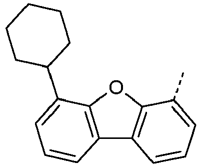 | 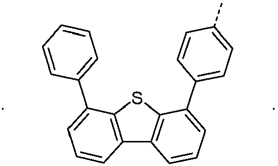 | 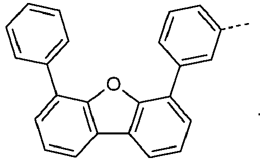 |
| Ar³-100 | Ar³-101 | Ar³-102 |
| 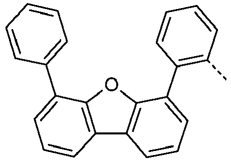 | 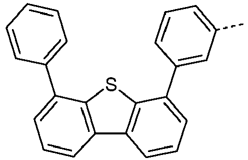 | 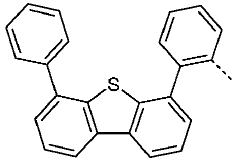 |
| Ar³-103 | Ar³-104 | Ar³-105 |
| 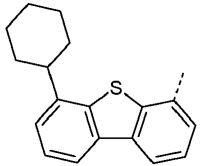 | 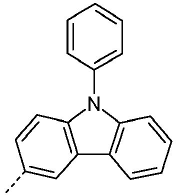 | 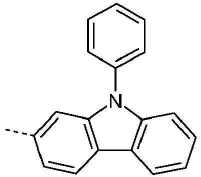 |
| Ar³-106 | Ar³-107 | Ar³-108 |
| 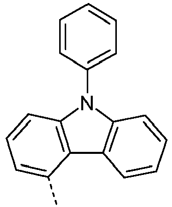 | 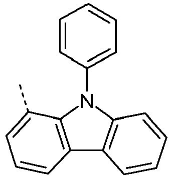 | 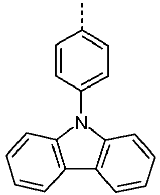 |
| Ar³-109 | Ar³-110 | Ar³-111 |
| 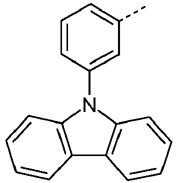 | 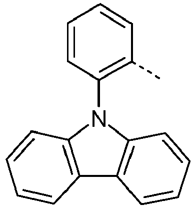 | 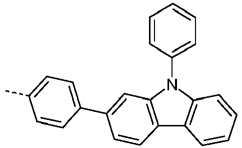 |
| Ar³-112 | Ar³-113 | Ar³-114 |
| 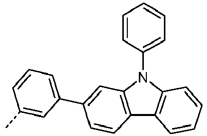 | 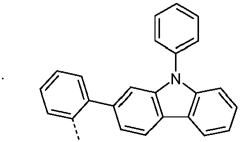 | 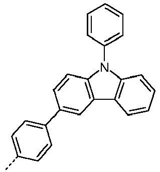 |
| Ar³-115 | Ar³-116 | Ar³-117 |
| 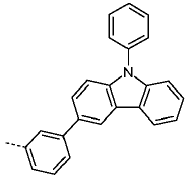 | 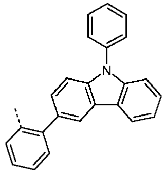 | 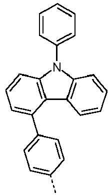 |
| Ar³-118 | Ar³-119 | Ar³-120 |
| 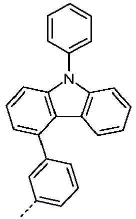 | 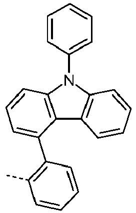 | 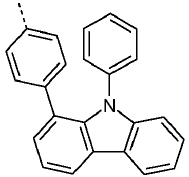 |
| Ar³-121 | Ar³-122 | Ar³-123 |
| 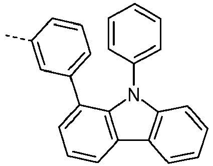 | 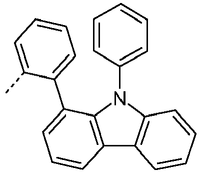 | 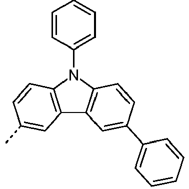 |
| Ar³-124 | Ar³-125 | Ar³-126 |
| 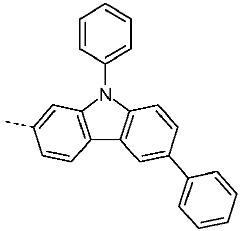 | 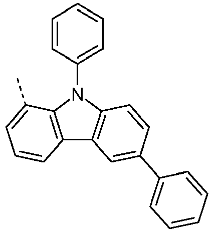 | 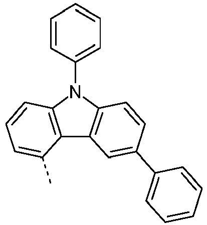 |
| Ar³-127 | Ar³-128 | Ar³-129 |
| 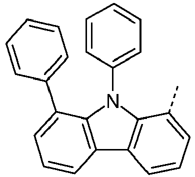 | 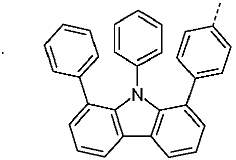 | 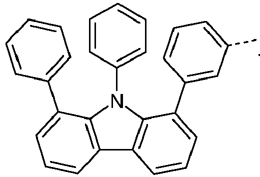 |
| Ar³-130 | Ar³-131 | Ar³-132 |
| 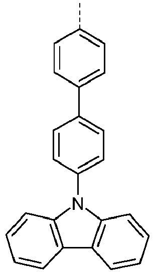 | 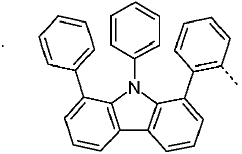 | 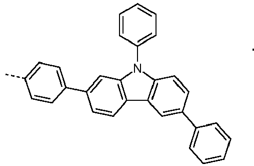 |
| Ar³-133 | Ar³-134 | Ar³-135 |
| 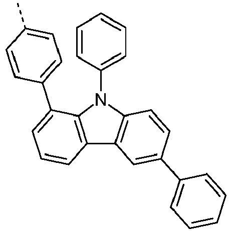 | 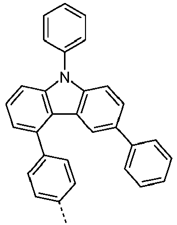 | 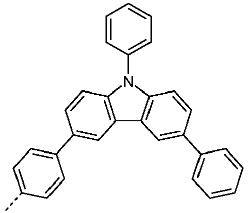 |
| Ar³-136 | Ar³-137 | Ar³-138 |
| 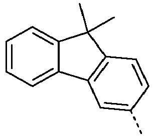 | 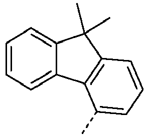 | 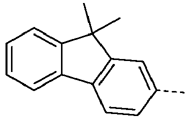 |
| Ar³-139 | Ar³-140 | Ar³-141 |
| 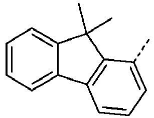 | 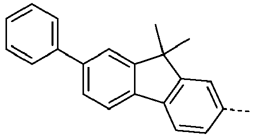 | 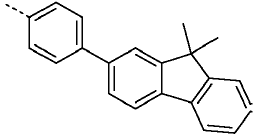 |
| Ar³-142 | Ar³-143 | Ar³-144 |
| 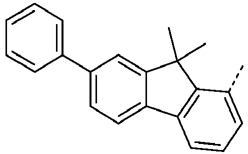 | 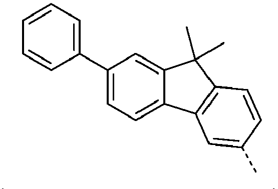 | 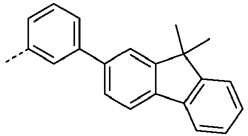 |
| Ar³-145 | Ar³-146 | Ar³-147 |
| 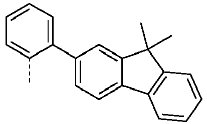 | 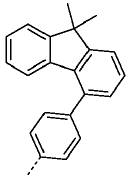 | 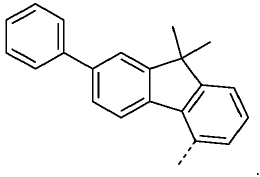 |
| Ar³-148 | Ar³-149 | Ar³-150 |
| 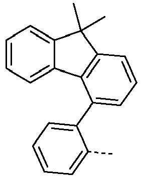 | 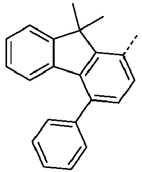 | 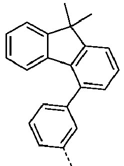 |
| Ar³-151 | Ar³-152 | Ar³-153 |
| 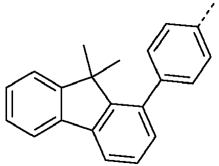 | 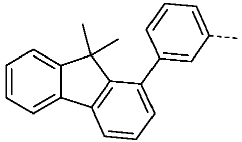 | 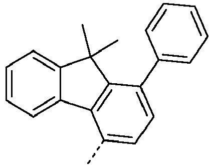 |
| Ar³-154 | Ar³-155 | Ar³-156 |
| 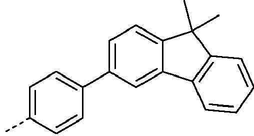 | 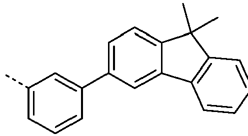 > | 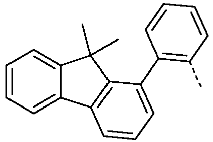 |
| Ar³-157 | Ar³-158 | Ar³-159 |
| 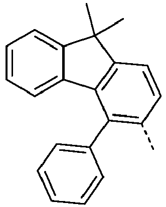 | 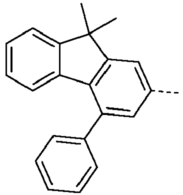 | 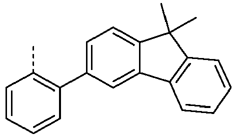 |
| Ar³-160 | Ar³-161 | Ar³-162 |
| 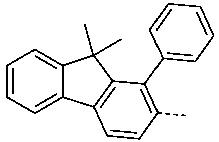 | 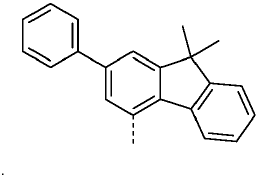 | 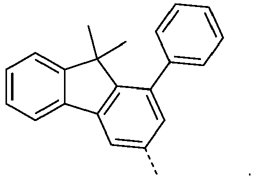 |
| Ar³-163 | Ar³-164 | Ar³-165 |
| 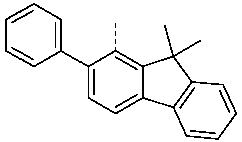 | 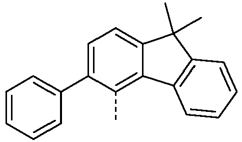 | 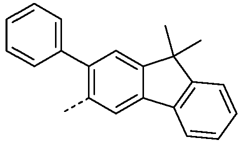 |
| Ar³-166 | Ar³-167 | Ar³-168 |
| 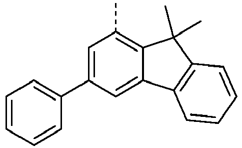 | 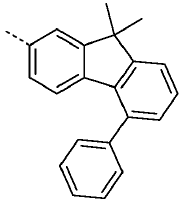 | 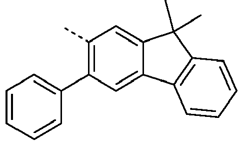 |
| Ar³-169 | Ar³-170 | Ar³-171 |
| 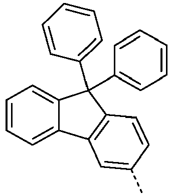 | 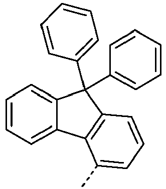 | 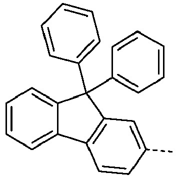 |
| Ar³-172 | Ar³-173 | Ar³-174 |
| 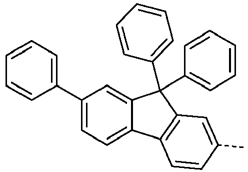 | 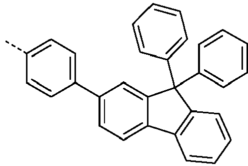 | 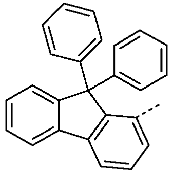 |
| Ar³-175 | Ar³-176 | Ar³-177 |
| 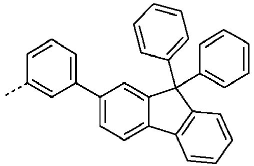 | 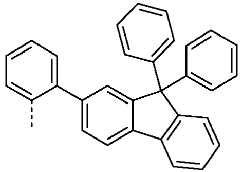 | 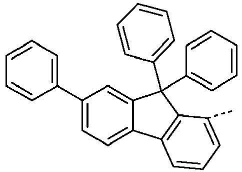 |
| Ar³-178 | Ar³-179 | Ar³-180 |
| 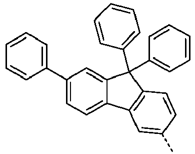 | 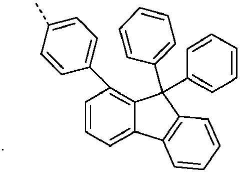 | 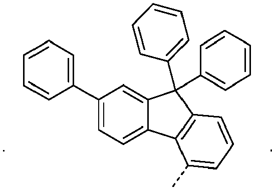 |
| Ar³-181 | Ar³-182 | Ar³-183 |
| 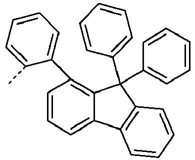 | 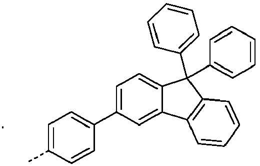 | 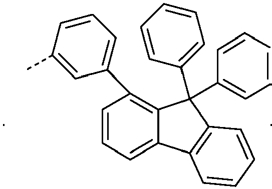 |
| Ar³-184 | Ar³-185 | Ar³-186 |
| 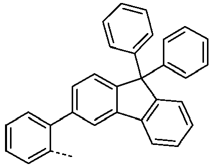 | 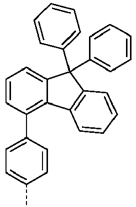 | 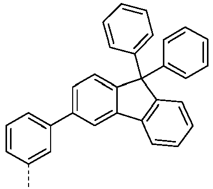 |
| Ar³-187 | Ar³-188 | Ar³-189 |
| 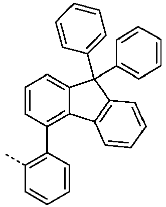 | 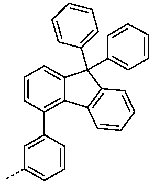 | 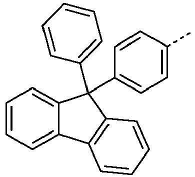 |
| Ar³-190 | Ar³-191 | Ar³-192 |
| 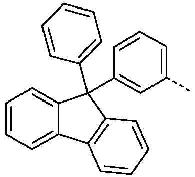 | 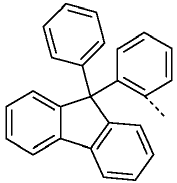 | 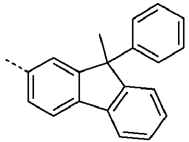 |
| Ar³-193 | Ar³-194 | Ar³-195 |
| 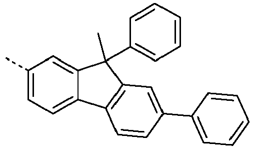 | 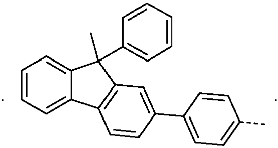 | 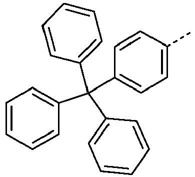 |
| Ar³-196 | Ar³-197 | Ar³-198 |
| 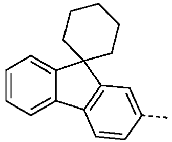 | 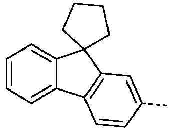 | 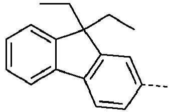 |
| Ar³-199 | Ar³-200 | Ar³-201 |
| 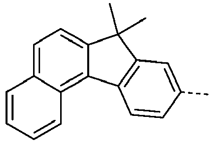 | 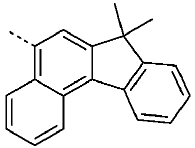 | 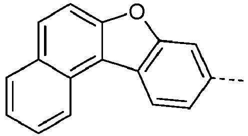 |
| Ar³-202 | Ar³-203 | Ar³-204 |
| 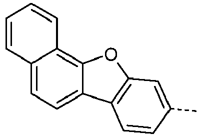 | 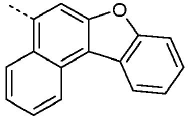 | 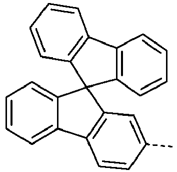 |
| Ar³-205 | Ar³-206 | Ar³-207 |
| 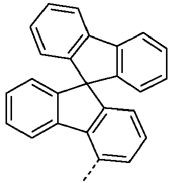 | 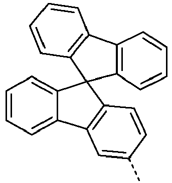 | 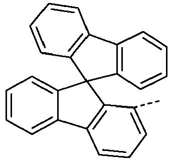 |
| Ar³-208 | Ar³-209 | Ar³-210 |
| 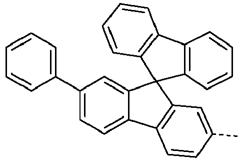 | 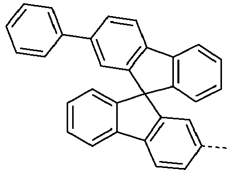 | 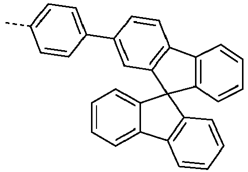 |
| Ar³-211 | Ar³-212 | Ar³-213 |
| 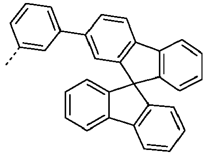 | 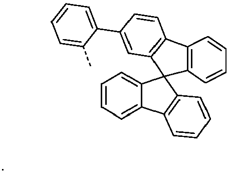 | 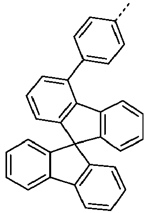 |
| Ar³-214 | Ar³-215 | Ar³-216 |
| 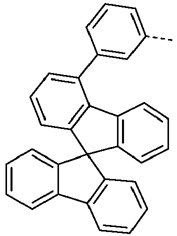 | 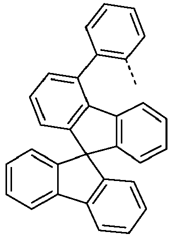 | 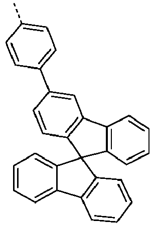 |
| Ar³-217 | Ar³-218 | Ar³-219 |
| 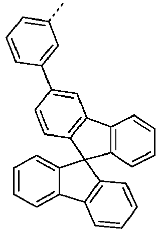 | 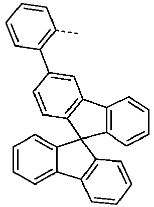 | 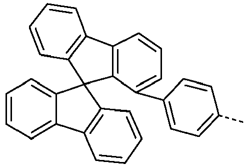 |
| Ar³-220 | Ar³-221 | Ar³-222 |
| 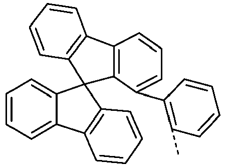 | 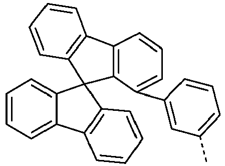 | 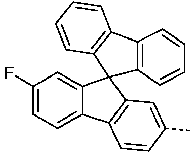 |
| Ar³-223 | Ar³-224 | Ar³-225 |
| 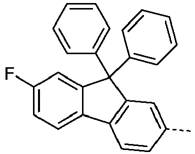 | 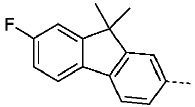 | 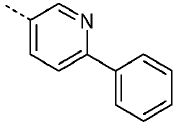 |
| Ar³-226 | Ar³-227 | Ar³-228 |
| 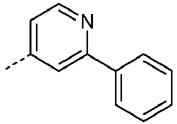 | 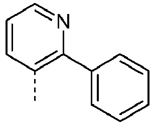 | 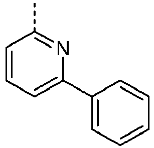 |
| Ar³-229 | Ar³-230 | Ar³-231 |
| 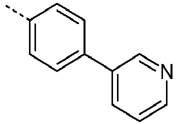 | 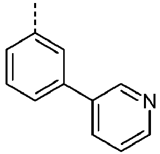 | 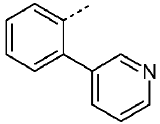 |
| Ar³-232 | Ar³-233 | Ar³-234 |
| 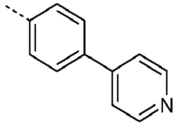 | 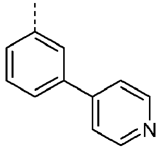 | 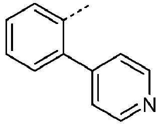 |
| Ar³-235 | Ar³-236 | Ar³-237 |
| 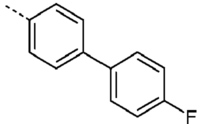 | 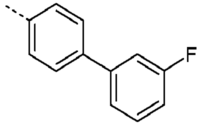 | 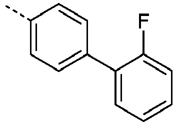 |
| Ar³-238 | Ar³-239 | Ar³-240 |
| 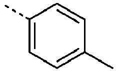 | 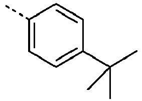 | 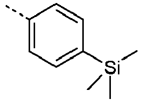 |
| Ar³-241 | Ar³-242 | Ar³-243 |
| 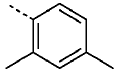 | 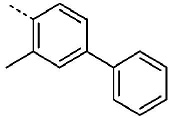 | 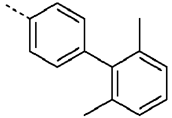 |
| Ar³-244 | Ar³-245 | Ar³-246 |
| 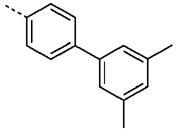 | 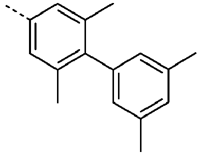 | 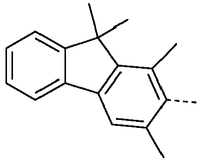 |
| Ar³-247 | Ar³-248 | Ar³-249 |
| 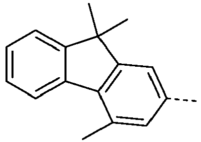 | 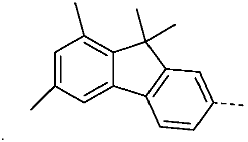 | 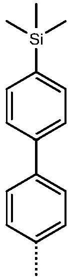 |
| Ar³-250 | Ar³-251 | Ar³-252 |
| 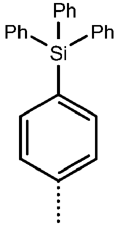 | 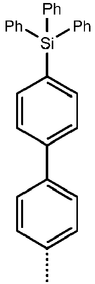 | 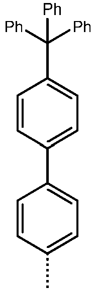 |
| Ar³-253 | Ar³-254 | Ar³-255 |
| 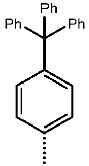 | 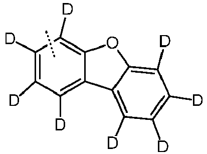 | 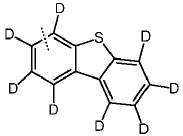 |
| Ar³-256 | Ar³-257 | Ar³-258 |
| 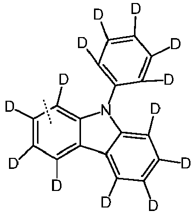 | 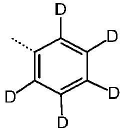 | 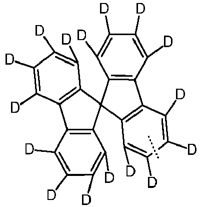 |
| Ar³-259 | Ar³-260 | Ar³-261 |
| 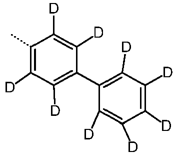 | 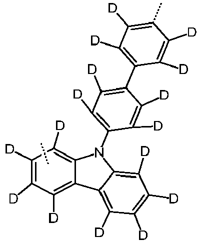 | 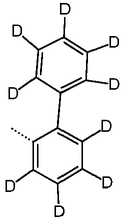 |
| Ar³-262 | Ar³-263 | Ar³-264 |
| 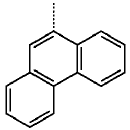 | 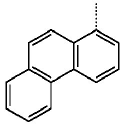 | 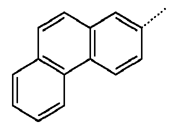 |
| Ar³-265 | Ar³-266 | 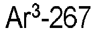 |
| 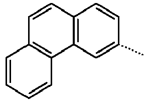 | 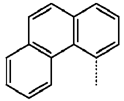 | 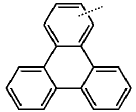 |
| Ar³-268 | Ar³-269 | Ar³-270 |

Darunter sind die Gruppen Ar³-1, Ar³-2, Ar³-3, Ar³-5, Ar³-50, Ar³-56, Ar³-78, Ar³-82, Ar³-111, Ar³-114, Ar³-140, Ar³-141, Ar³-257, Ar³-262, Ar³-263 besonders bevorzugt.

Einheiten -[Ar²]ₙ-Ar³ sind bevorzugt gewählt aus den folgenden Einheiten

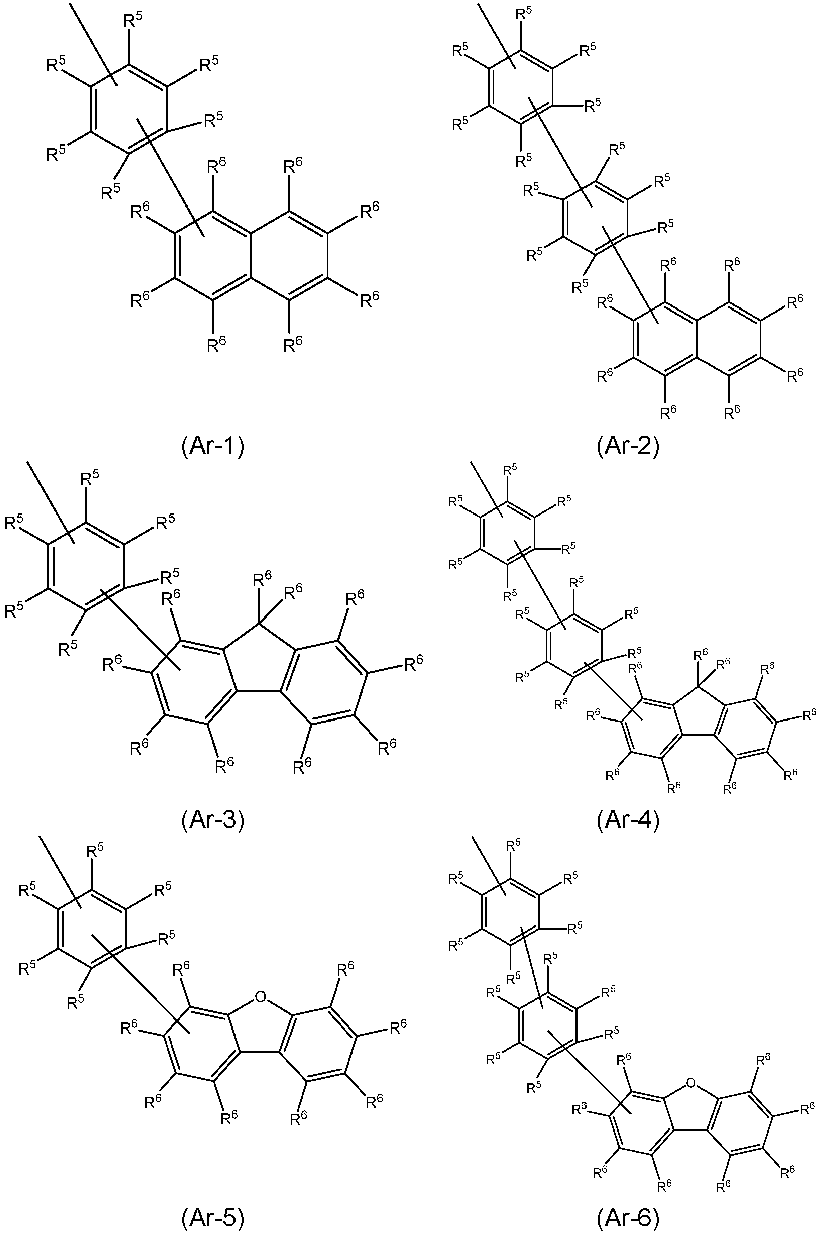

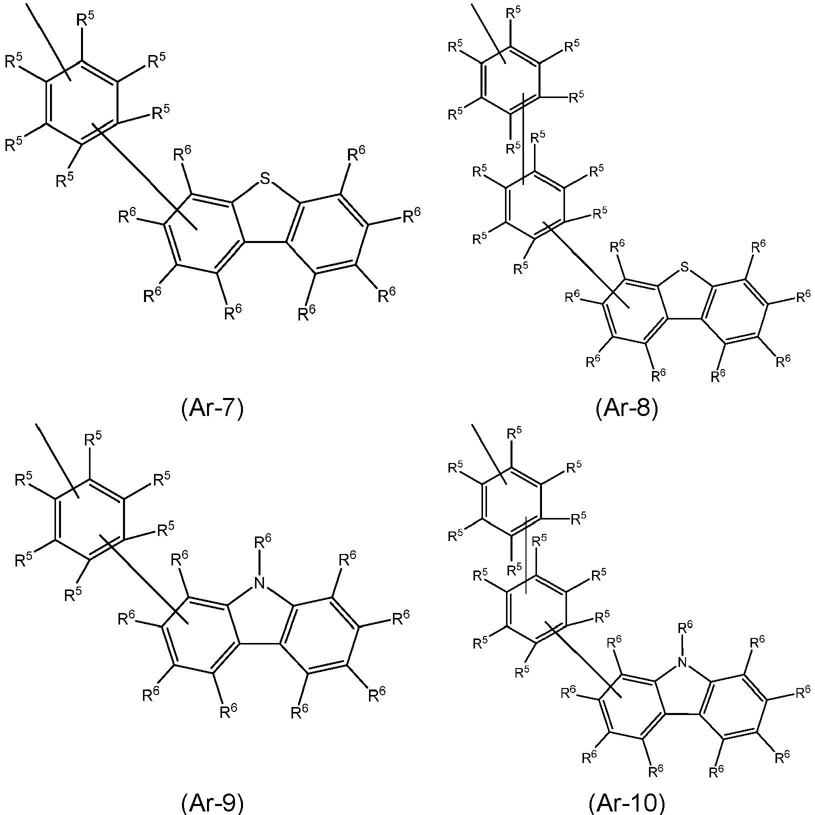

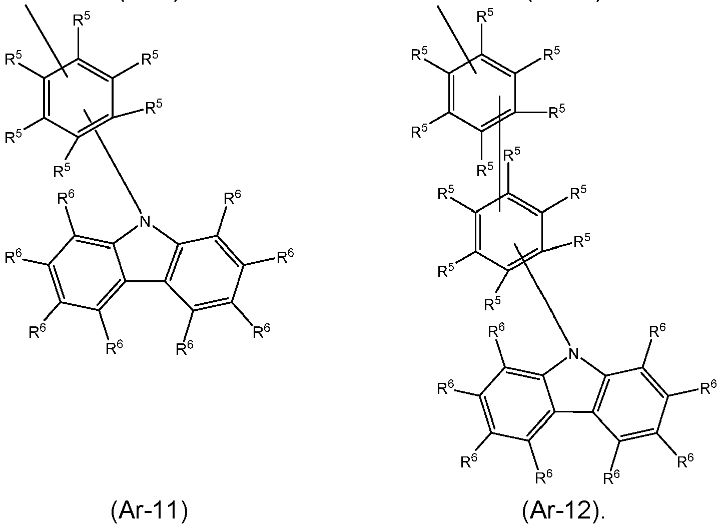

Bevorzugte Ausführungsformen der Formel (I) sind aus den folgenden Formeln gewählt:

| |
|---|
| 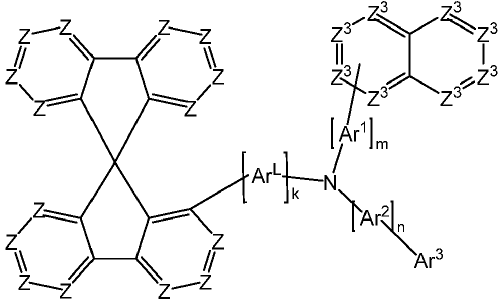 |
| (I-1) |
| 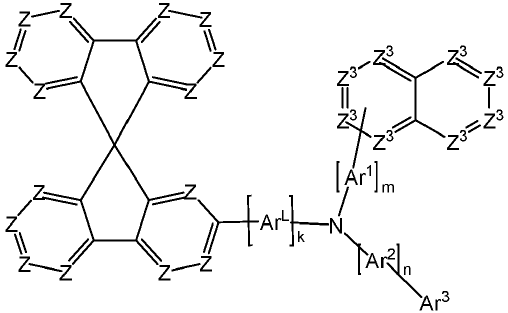 |
| (I-2) |
| 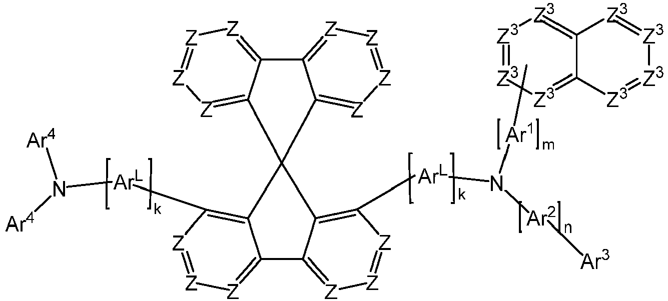 |
| (I-3) |
| 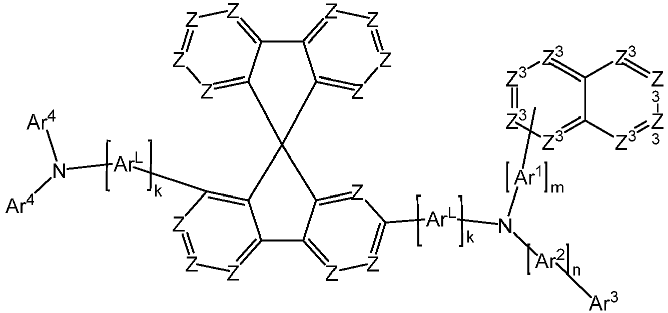 |
| (I-4) |
| 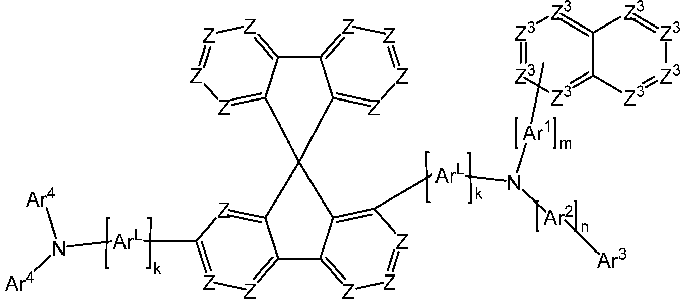 |
| (I-5) |
| 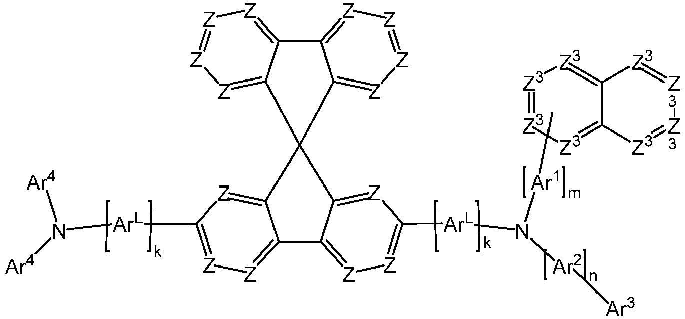 |
| (I-6) |
| 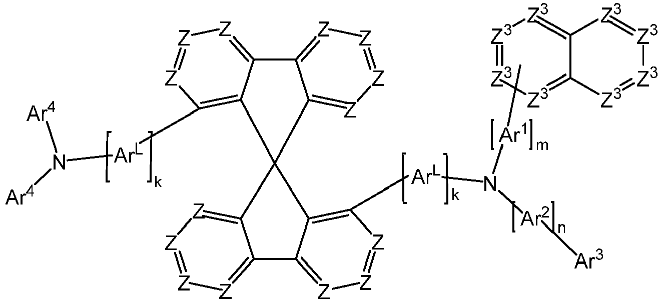 |
| (I-7) |
| 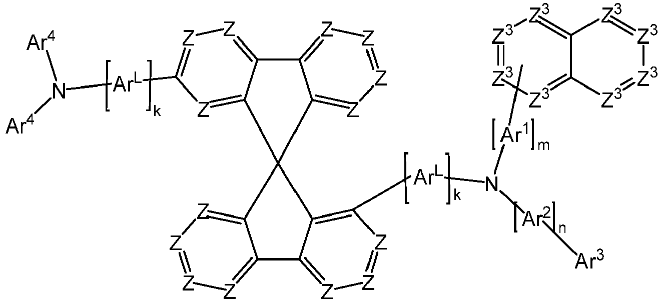 |
| (I-8) |
| 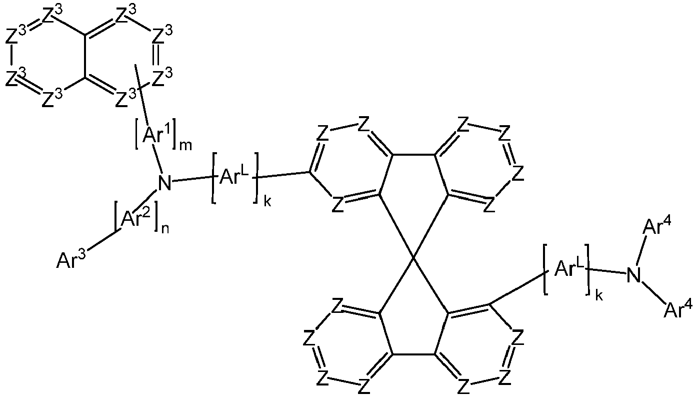 |
| (I-9) |
| 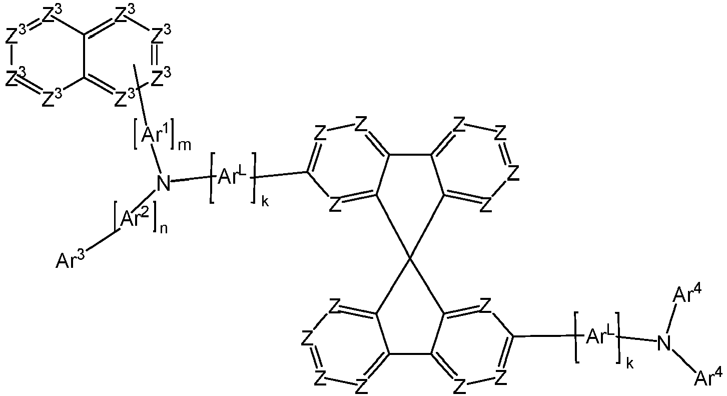 |
| (I-10) |

wobei die auftretenden Variablen definiert sind wie oben, und wobei Formeln (I-1) und (I-2) bevorzugt sind, besonders bevorzugt Formel (I-1). Bevorzugt gelten in den Formeln die bevorzugten Ausführungsformen der variablen Gruppen und Indices, insbesondere der Gruppen Z, Z³, Ar^{L}, Ar¹, Ar², Ar³, k, m und n. Insbesondere bevorzugt ist Z gleich CR¹.

Bevorzugt ist in den Formeln (I-3) bis (I-10) die Gruppe -[Ar^{L}]ₖ-N(Ar⁴)₂ definiert als eine Gruppe der Formel

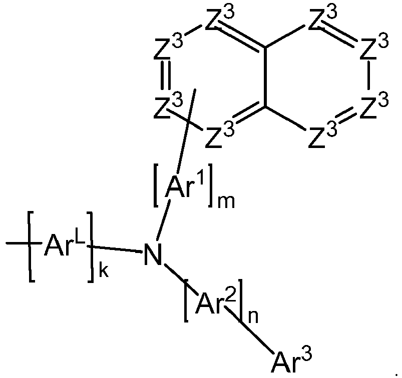

Entsprechende bevorzugte Formeln für die anmeldungsgemäßen Verbindungen sind:

| |
|---|
| 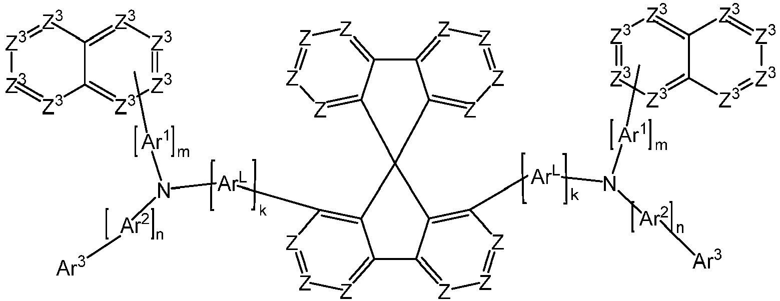 |
| (I-3-1) |
| 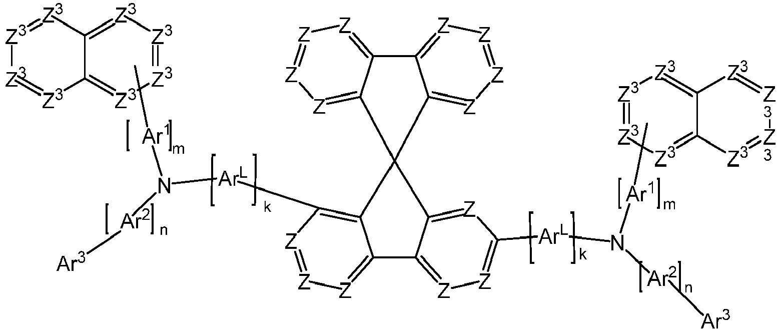 |
| (I-4-1) |
| 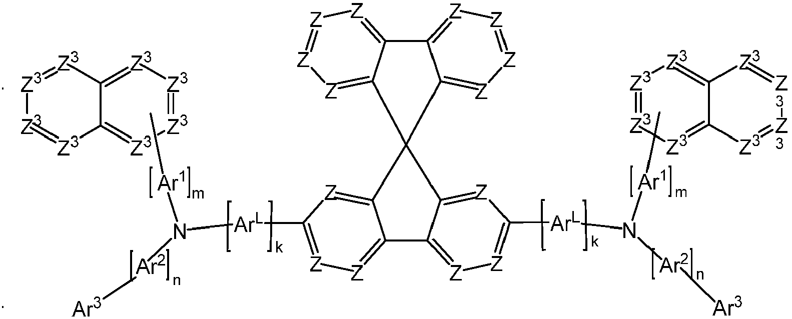 |
| (I-6-1) |
| 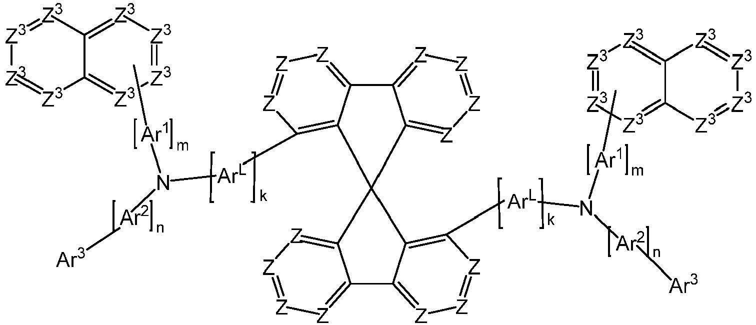 |
| (I-7-1) |
| 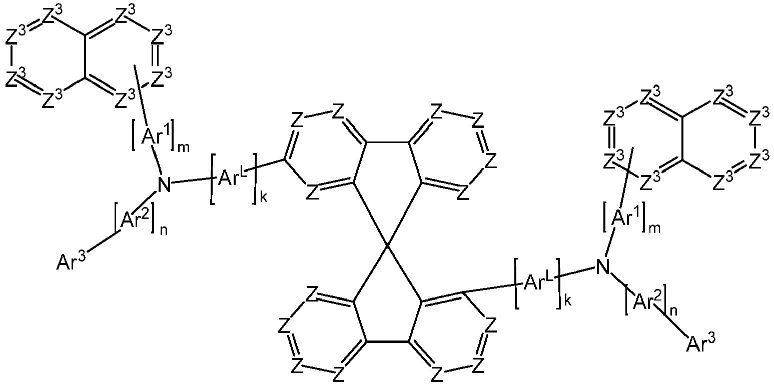 |
| (I-8-1) |
| 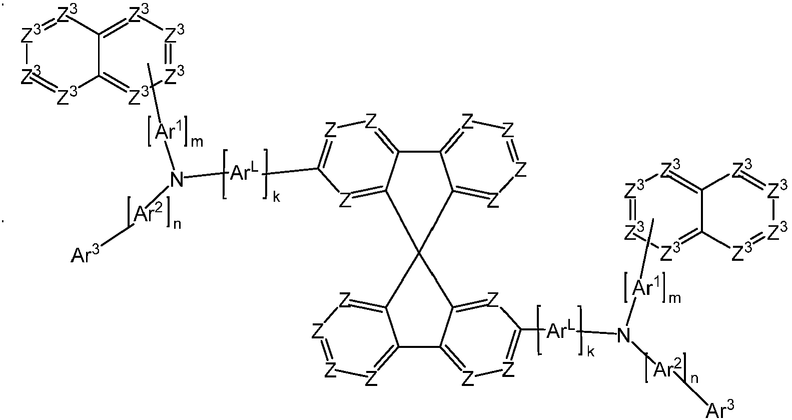 |
| (I-10-1) |

wobei die auftretenden Gruppen definiert sind wie oben. Bevorzugt gelten in den Formeln die bevorzugten Ausführungsformen der variablen Gruppen und Indices, insbesondere der Gruppen Z, Z³, Ar^{L}, Ar¹, Ar², Ar³, k, m und n. Insbesondere bevorzugt ist Z gleich CR¹ und Z³ ist gleich CR⁴.

Besonders bevorzugte Ausführungsformen der Formel (I) sind aus den folgenden Formeln gewählt:

| | | |
|---|---|---|
| 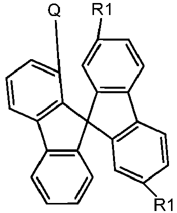 | 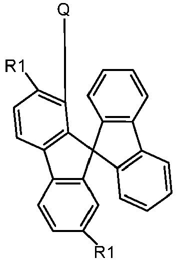 | 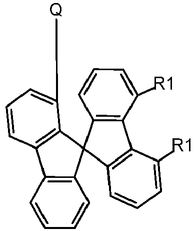 |
| (I-A-1) | (I-A-2) | (I-A-3) |
| 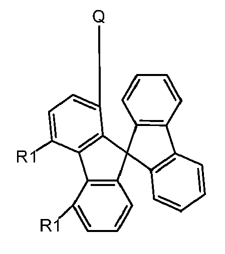 | 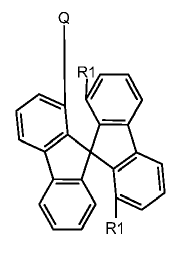 | 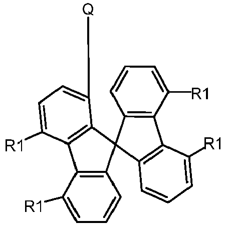 |
| (I-A-4) | (I-A-5) | (I-A-6) |
| 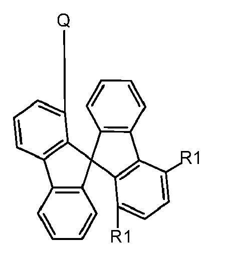 | 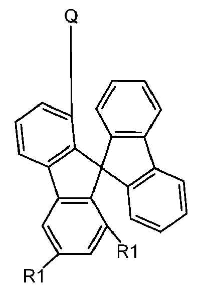 | 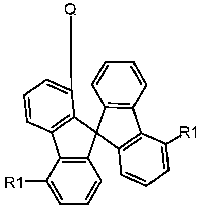 |
| (I-A-7) | (I-A-8) | (I-A-9) |
| 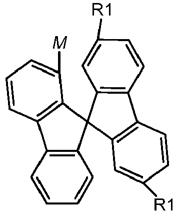 | 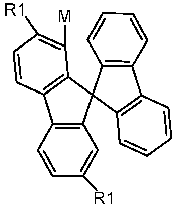 | 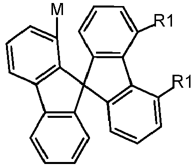 |
| (I-B-1) | (I-B-2) | (I-B-3) |
| 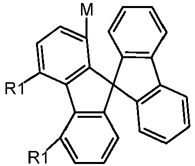 | 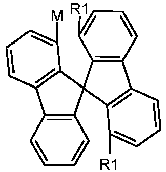 | 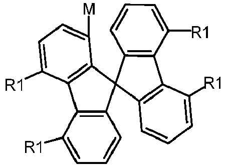 |
| (I-B-4) | (I-B-5) | (I-B-6) |
| 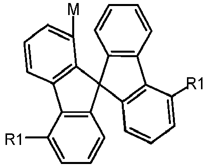 | 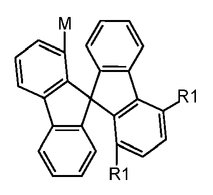 | 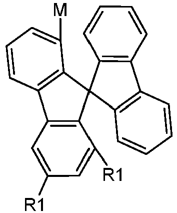 |
| (I-B-7) | (I-B-8) | (I-B-9) |
| 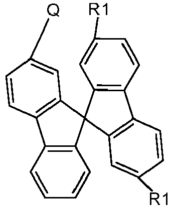 | 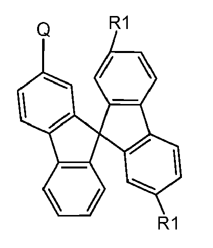 | 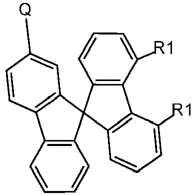 |
| (I-C-1) | (I-C-2) | (I-C-3) |
| 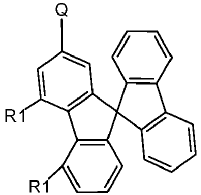 | 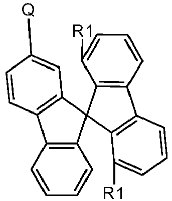 | 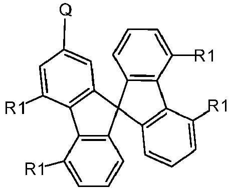 |
| (I-C-4) | (I-C-5) | (I-C-6) |
| 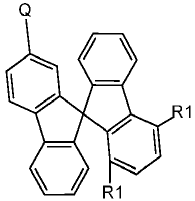 | 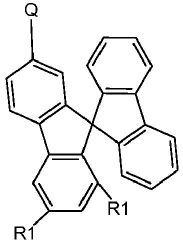 | 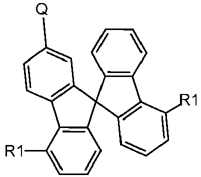 |
| (I-C-7) | (I-C-8) | (I-C-9) |
| 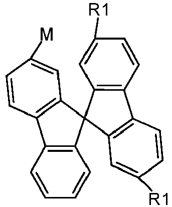 | 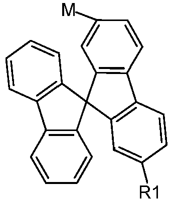 | 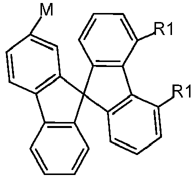 |
| (I-D-1) | (I-D-2) | (I-D-3) |
| 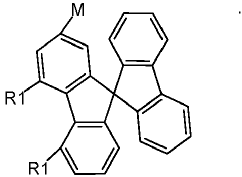 | 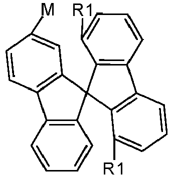 | 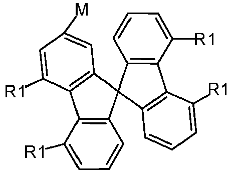 |
| (I-D-4) | (I-D-5) | (I-D-6) |
| 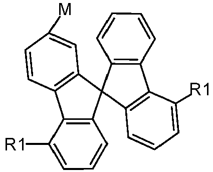 | 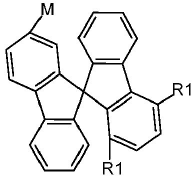 | 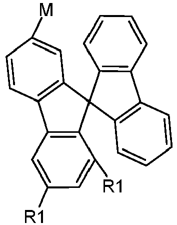 |
| (I-D-7) | (I-D-8) | (I-D-9) |
| 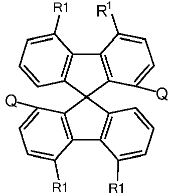 | 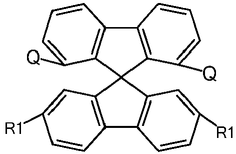 | 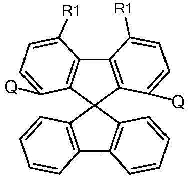 |
| (I-E-1) | (I-E-2) | (I-E-3) |
| 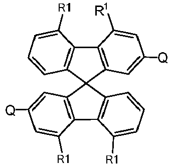 | 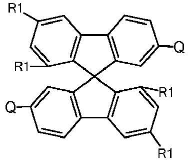 | 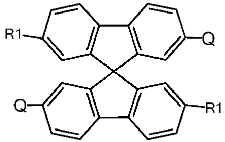 |
| (I-F-1) | (I-F-2) | (I-F-3) |
| 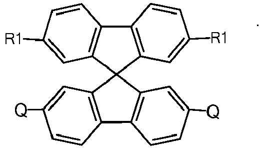 | 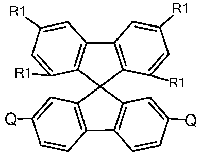 | 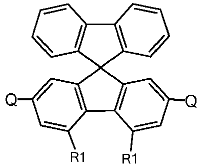 |
| (I-F-4) | (I-F-5) | (I-F-6) |
| 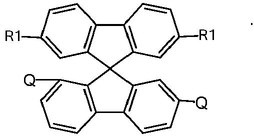 | 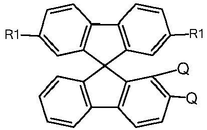 | 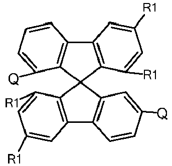 |
| (I-G-1) | (I-G-2) | (I-G-3) |
| 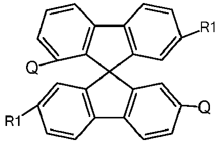 | 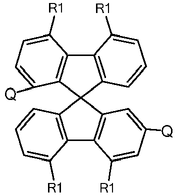 | 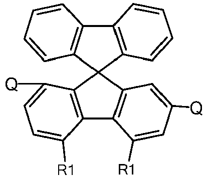 |
| (I-G-4) | (I-G-5) | (I-G-6) |
| 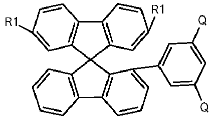 | 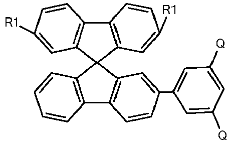 | |
| (I-H-1) | (I-H-2) | |
| 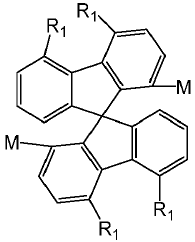 | 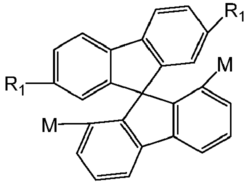 | 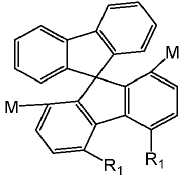 |
| (I-I-1) | (I-I-2) | (I-I-3) |
| 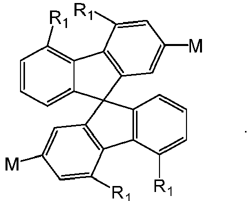 | 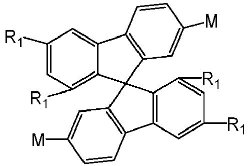 | 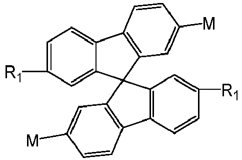 |
| (I-J-1) | (I-J-2) | (I-J-3) |
| 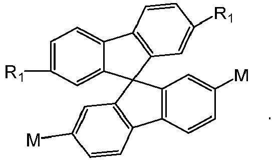 | 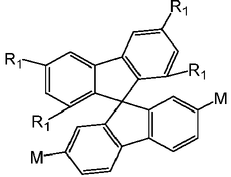 | 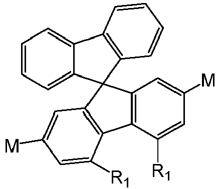 |
| (I-J-4) | (I-J-5) | (I-J-6) |
| 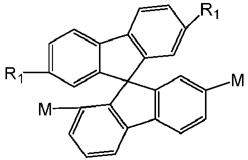 | 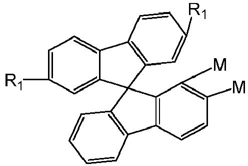 | 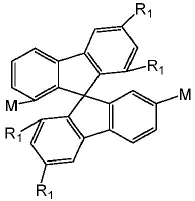 |
| (I-K-1) | (I-K-2) | (I-K-3) |
| 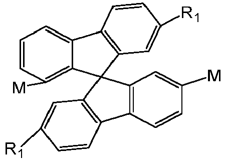 | 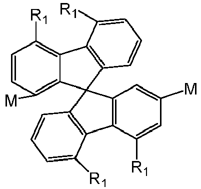 | 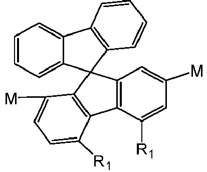 |
| (I-K-4) | (I-K-5) | (I-K-6) |
| 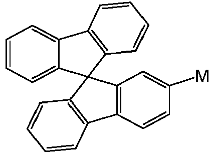 | 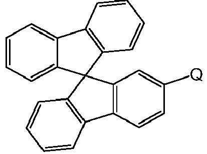 | 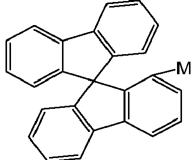 |
| (I-L-1) | (I-L-2) | (I-M-1) |
| 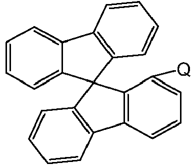 | | |
| (I-M-2) | | |

wobei Gruppen M und Q wie folgt definiert sind:

| | |
|---|---|
| 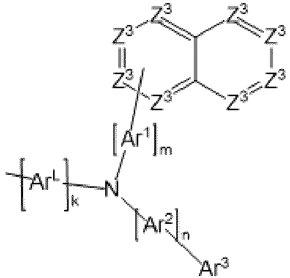 | 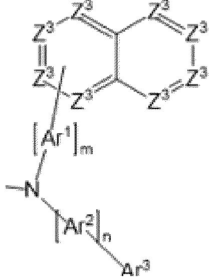 |
| Gruppe M | Gruppe Q |

und wobei die sonstigen Variablen definiert sind wie oben, und wobei R1 definiert ist wie R¹.

Bevorzugte Verbindungen sind in der folgenden Tabelle gezeigt:

| | | |
|---|---|---|
| 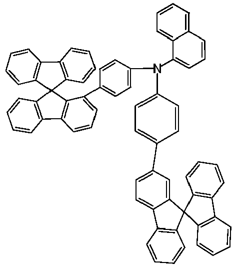 | 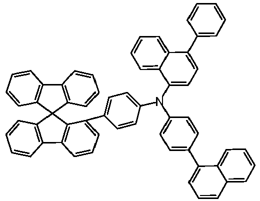 | 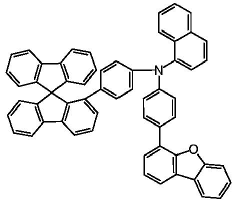 |
| 1 | 2 | 3 |
| 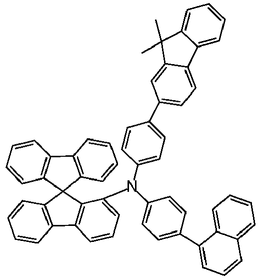 | 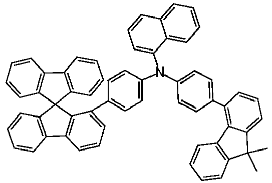 | 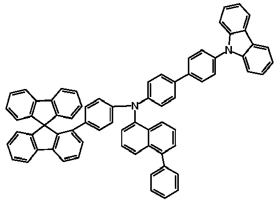 |
| 4 | 5 | 6 |
| 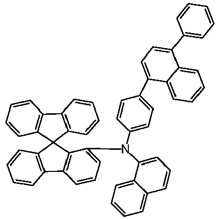 | 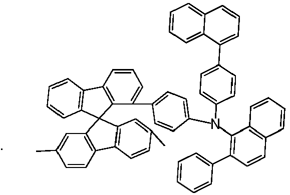 | 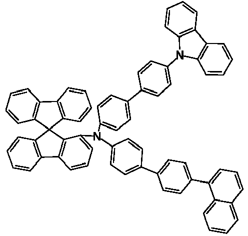 |
| 7 | 8 | 9 |
| 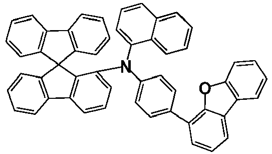 | 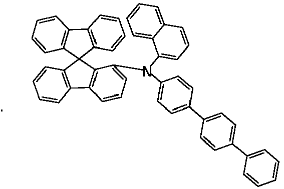 | 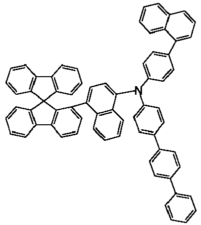 |
| 10 | 11 | 12 |
| 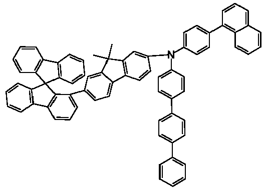 | 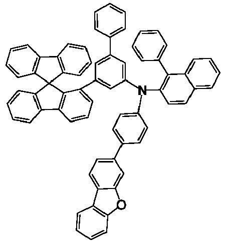 | 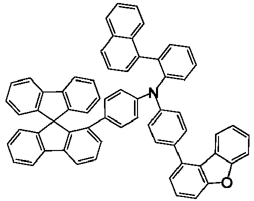 |
| 13 | 14 | 15 |
| 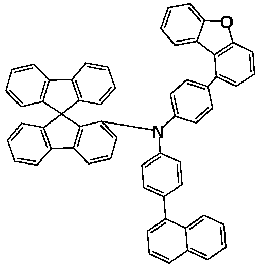 | 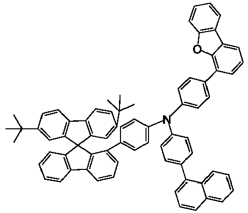 | 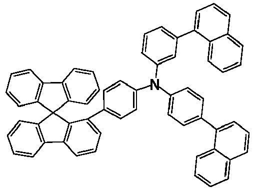 |
| 16 | 17 | 18 |
| 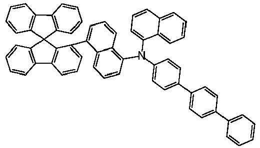 | 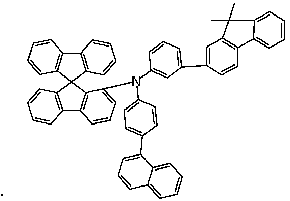 | 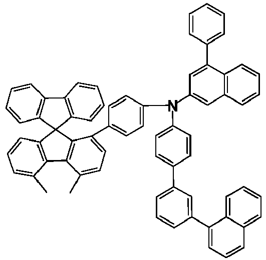 |
| 19 | 20 | 21 |
| 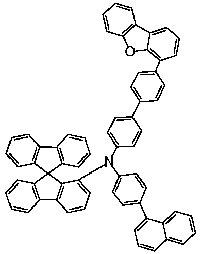 | 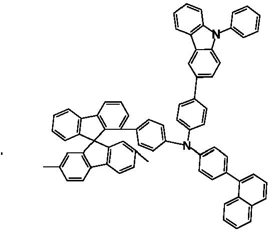 | 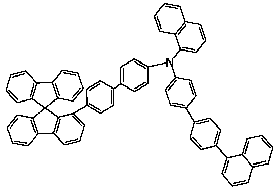 |
| 22 | 23 | 24 |
| 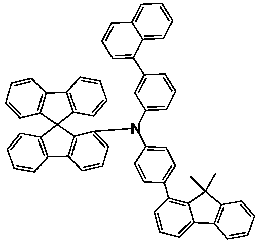 | 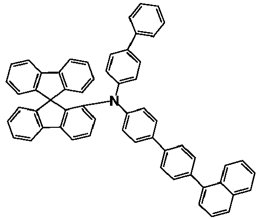 | 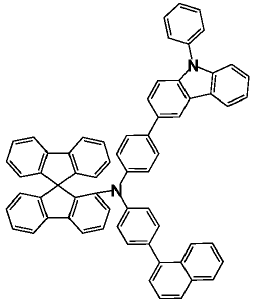 |
| 25 | 26 | 27 |
| 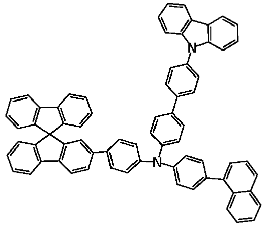 | 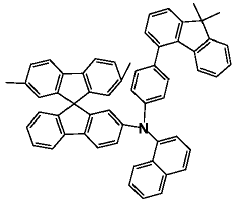 | 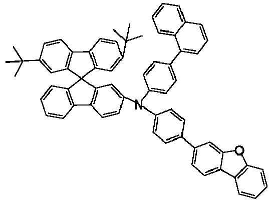 |
| 28 | 29 | 30 |
| 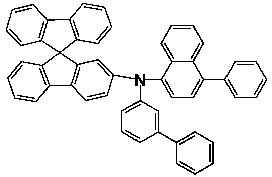 | 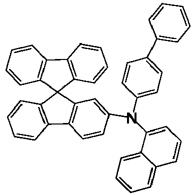 | 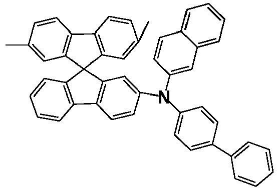 |
| 31 | 32 | 33 |
| 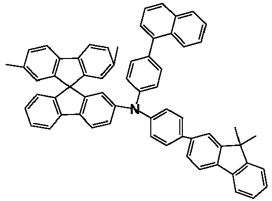 | 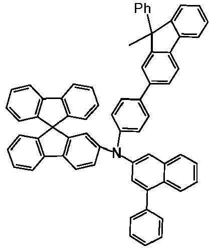 | 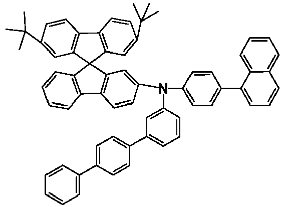 |
| 34 | 35 | 36 |
| 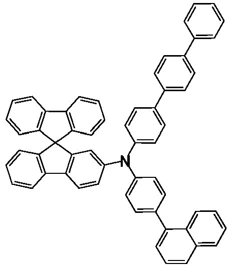 | 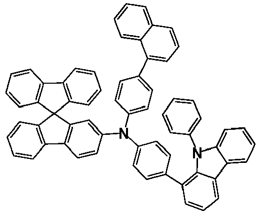 | 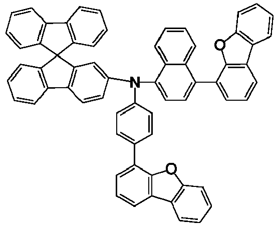 |
| 37 | 38 | 39 |
| 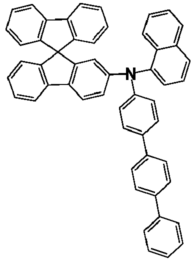 | 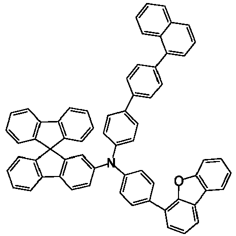 | 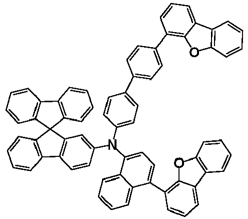 |
| 40 | 41 | 42 |
| 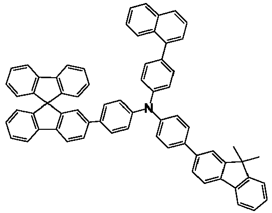 | 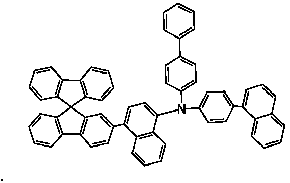 | 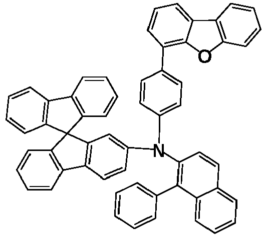 |
| 43 | 44 | 45 |
| 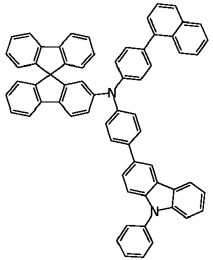 | 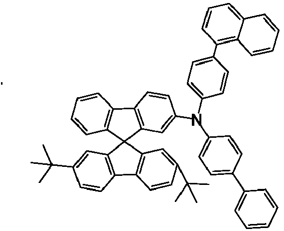 | 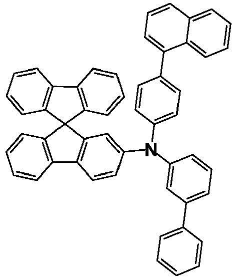 |
| 46 | 47 | 48 |
| 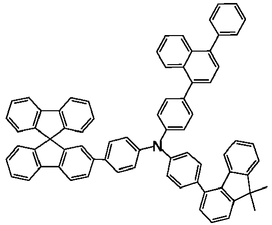 | 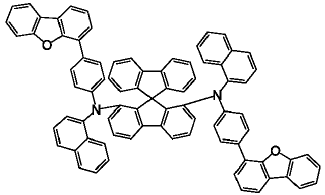 | |
| 49 | 50 | |
| 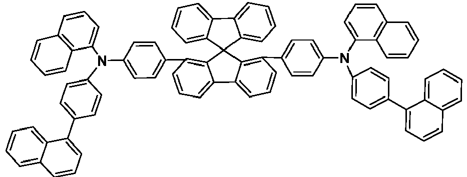 | | 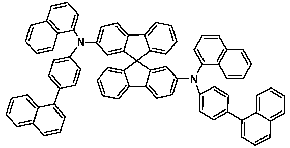 |
| 51 | | 52 |
| 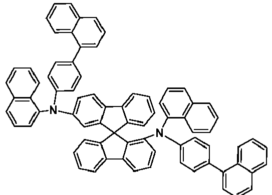 | 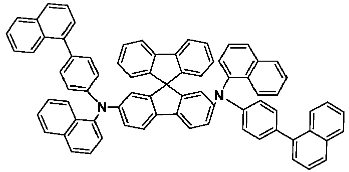 | |
| 53 | 54 | |
| 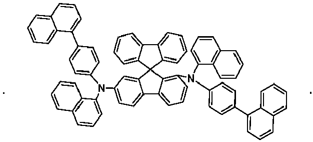 | 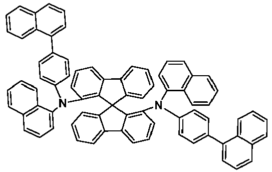 | 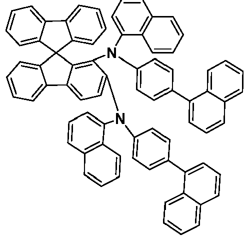 |
| 55 | 56 | 57 |
| 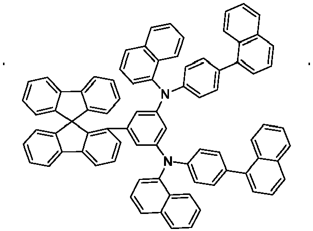 | 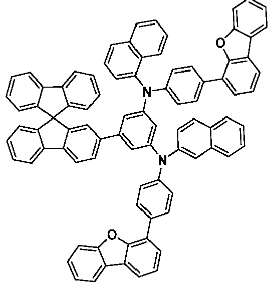 | 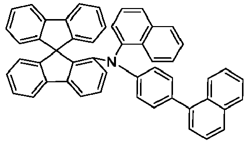 |
| 58 | 59 | 60 |
| 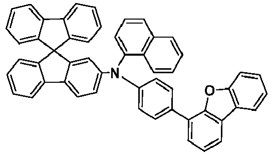 | 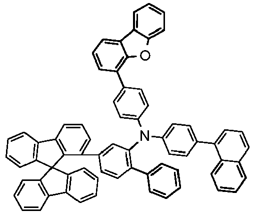 | 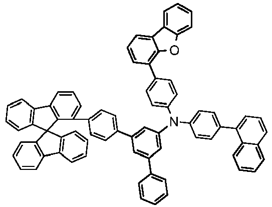 |
| 61 | 62 | 63 |

Im Folgenden sind bevorzugte Verfahren zur Herstellung von Verbindungen gemäß Formel (I) gezeigt:

Gemäß dem in Schema 1 gezeigten Verfahren wird ausgehend von einem halogensubstituierten Spirobifluoren über eine Buchwald-Kupplungsreaktion mit einem Diarylamin eine Verbindung der Formel (I) hergestellt. 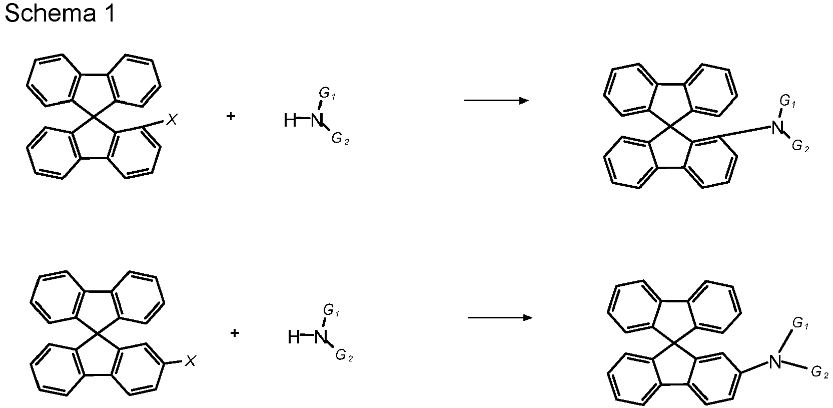
X: Halogen
G₁, G₂: aromatische oder heteroaromatische Gruppe

Alternativ kann gemäß dem in Schema 2 gezeigten Verfahren zunächst eine Buchwald-Kupplung zwischen einem halogensubstituierten Fluorenon und einem Diarylamin durchgeführt werden, und anschließend über Addition von ortho-Halogenylbiphenyl mit Metallorganyl und Ringschluss das Spirobifluoren-Gerüst hergestellt werden. 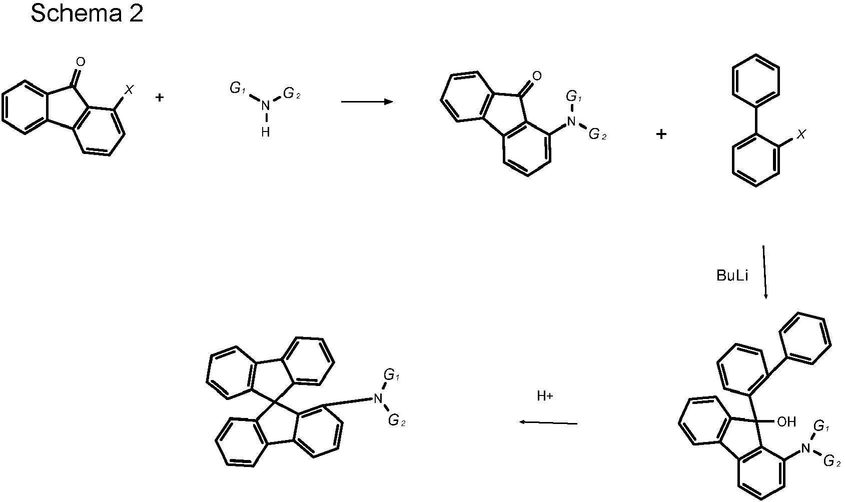
X: Halogen
G₁, G₂: aromatische oder heteroaromatische Gruppe

Alternativ können über die in Schema 3 gezeigte Synthese Verbindungen gemäß Formel (I) hergestellt werden, die eine Spacergruppe zwischen der Spirobifluorenylgruppe und der Aminogruppe enthalten. Dazu wird ein Halogenyl-substituiertes Fluorenon in einer Suzuki-Kupplung mit einer Boronsäure-substituierten Arylgruppe umgesetzt, die eine Halogenylgruppe trägt. Das Kupplungsprodukt wird anschließend mit Diarylamin in einer Buchwald-Reaktion umgesetzt. Daraufhin wird analog zu der in Schema 2 gezeigten Synthese über Addition von ortho-Halogenylbiphenyl mit Metallorganyl und Ringschluss das Spirobifluoren-Gerüst hergestellt. 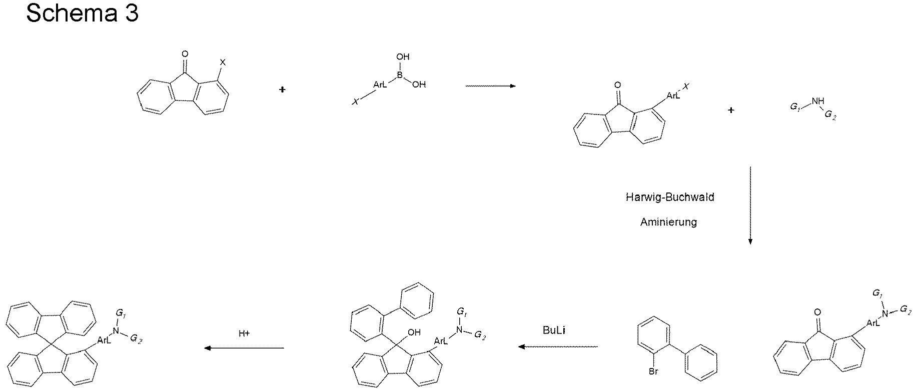
X: Halogen
G₁, G₂: aromatische oder heteroaromatische Gruppe
An_: aromatische oder heteroaromatische Gruppe

Ein weiteres bevorzugtes Verfahren zur Herstellung von Verbindungen gemäß Formel (I), die eine Spacergruppe zwischen der Spirobifluorenylgruppe und der Aminogruppe aufweisen, ist in Schema 4 gezeigt. Dazu wird ein halogensubstituiertes Spirobifluoren in einer Suzuki-Kupplung mit einem Amin umgesetzt, das drei aromatische bzw. heteroaromatische Reste aufweist, wobei einer dieser Reste eine Boronsäuregruppe aufweist. 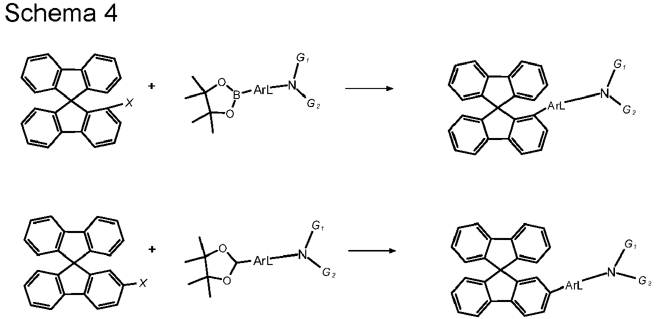
X: Halogen
G₁, G₂: aromatische oder heteroaromatische Gruppe
An_: aromatische oder heteroaromatische Gruppe

Ein weiteres bevorzugtes Verfahren zur Herstellung von Verbindungen gemäß Formel (I), die eine Spacergruppe zwischen der Spirobifluorenylgruppe und der Aminogruppe aufweisen, ist in Schema 5 gezeigt.

Dazu wird ein Spirobifluoren, das eine Halogengruppe trägt, in einer Suzuki-Kupplung mit einer boronsäuresubstituierten Arylgruppe umgesetzt, die eine Halogengruppe trägt. Anschließend wird eine Diarylaminogruppe in einer Buchwald-Kupplung eingeführt. 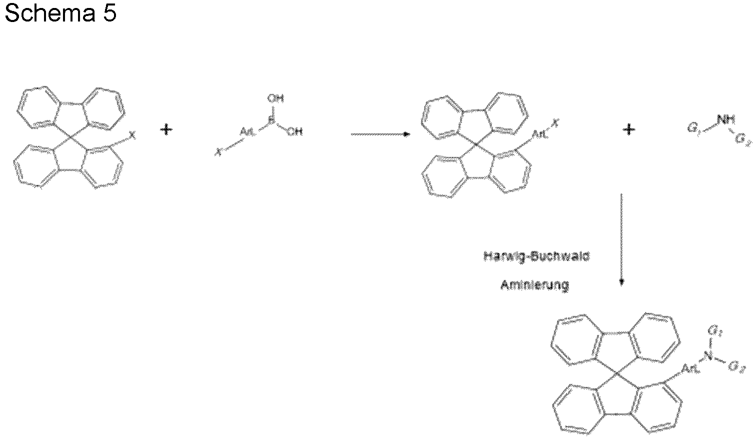
X: Halogen
G₁, G₂: aromatische oder heteroaromatische Gruppe
An_: aromatische oder heteroaromatische Gruppe

Gegenstand der vorliegenden Anmeldung ist damit ein Verfahren zur Herstellung einer Verbindung der Formel (I), dadurch gekennzeichnet, dass mittels einer metallorganischen Kupplungsreaktion eine Aminogruppe eingeführt wird. Bevorzugt ist die metallorganische Kupplungsreaktion ausgewählt aus einer Suzuki-Kupplung und einer Buchwald-Kupplung. Weiterhin bevorzugt erfolgt die metallorganische Kupplungsreaktion zwischen einem Spirobifluorenyl-Derivat, das einen Halogensubstituenten aufweist, oder einem Fluorenon-Derivat, das einen Halogensubstituenten aufweist, und der Aminogruppe.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹, R², R³, R⁴, R⁵ oder R⁶ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen, Spirobifluorenen, Paraphenylenen, Carbazolen, Thiophenen, Dihydrophenanthrenen, cis- und trans-Indenofluorenen, Ketonen, Phenanthrenen oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine oder phosphoreszierende Metallkomplexe, und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, alpha-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt.

Die Verbindung gemäß Formel (I) eignet sich für den Einsatz in einer elektronischen Vorrichtung, insbesondere einer organischen Elektrolumineszenzvorrichtung (OLED). Abhängig von der Substitution kann die Verbindung der Formel (I) in unterschiedlichen Funktionen und Schichten eingesetzt werden. Bevorzugt ist die Verwendung als lochtransportierendes Material in einer lochtransportierenden Schicht und/oder als Matrixmaterial in einer emittierenden Schicht, besonders bevorzugt in Kombination mit einem phosphoreszierenden Emitter.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Gegenstand der Erfindung ist weiterhin eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist eine organische Elektrolumineszenzvorrichtung, enthaltend Anode, Kathode und mindestens eine emittierende Schicht, dadurch gekennzeichnet, dass mindestens eine organische Schicht in der Vorrichtung enthalten ist, welche mindestens eine Verbindung gemäß Formel (I) enthält. Bevorzugt ist eine organische Elektrolumineszenzvorrichtung, enthaltend Anode, Kathode und mindestens eine emittierende Schicht, dadurch gekennzeichnet, dass mindestens eine organische Schicht in der Vorrichtung, gewählt aus lochtransportierenden und emittierenden Schichten, mindestens eine Verbindung gemäß Formel (I) enthält.

Unter einer lochtransportierenden Schicht werden dabei alle Schichten verstanden, die zwischen Anode und emittierender Schicht angeordnet sind, bevorzugt Lochinjektionsschicht, Lochtransportschicht, und Elektronenblockierschicht. Unter eine Lochinjektionsschicht wird dabei eine Schicht verstanden, die direkt an die Anode angrenzt. Unter einer Lochtransportschicht wird dabei eine Schicht verstanden, die zwischen Anode und emittierender Schicht vorliegt, aber nicht direkt an die Anode angrenzt, bevorzugt auch nicht direkt an die emittierende Schicht angrenzt. Unter einer Elektronenblockierschicht wird dabei eine Schicht verstanden, die zwischen Anode und emittierender Schicht vorliegt und direkt an die emittierende Schicht angrenzt. Eine Elektronenblockierschicht weist bevorzugt ein energetisch hoch liegendes LUMO auf und hält dadurch Elektronen von Austritt aus der emittierenden Schicht ab.

Außer Kathode, Anode und emittierender Schicht kann die elektronische Vorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der elektronischen Vorrichtung ist bevorzugt wie folgt:
- Anode-
- Lochinjektionsschicht-
- Lochtransportschicht-
- optional weitere Lochtransportschichten-
- emittierende Schicht-
- optional Lochblockierschicht-
- Elektronentransportschicht-
- Elektroneninjektionsschicht-
- Kathode-.

Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei jeweils eine der drei Schichten blaue, jeweils eine der drei Schichten grüne und jeweils eine der drei Schichten orangefarbene oder rote Emission zeigt. Die erfindungsgemäßen Verbindungen sind dabei bevorzugt in einer lochtransportierenden Schicht oder in der emittierenden Schicht vorhanden. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist bevorzugt, dass die Verbindung der Formel (I) als Lochtransportmaterial verwendet wird. Dabei kann die emittierende Schicht eine fluoreszierende emittierende Schicht sein, oder sie kann eine phosphoreszierende emittierende Schicht sein. Bevorzugt ist die emittierende Schicht eine blau fluoreszierende Schicht oder eine grün phosphoreszierende Schicht.

Wenn die Vorrichtung enthaltend die Verbindung der Formel (I) eine phosphoreszierende emittierende Schicht enthält, ist es bevorzugt, dass diese Schicht zwei oder mehr, bevorzugt genau zwei, verschiedene Matrixmaterialien enthält (mixed-Matrix-System). Bevorzugte Ausführungsformen von mixed-Matrix-Systemen sind weiter unten näher beschrieben.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden.

Gemäß einer bevorzugten Ausführungsform enthält eine lochtransportierende Schicht enthaltend die Verbindung der Formel (I) zusätzlich eine oder mehrere weitere lochtransportierende Verbindungen. Diese weiteren lochtransportierenden Verbindungen sind bevorzugt gewählt aus Triarylamin-Verbindungen, besonders bevorzugt aus Mono-Triarylaminverbindungen. Ganz besonders bevorzugt sind sie gewählt aus den weiter unten angegebenen bevorzugten Ausführungsformen von Lochtransportmaterialien. In der beschriebenen bevorzugten Ausführungsform sind die Verbindung der Formel (I) und die eine oder mehrere weiteren lochtransportierenden Verbindungen bevorzugt jeweils in einem Anteil von mindestens 10% vorhanden, besonders bevorzugt jeweils in einem Anteil von mindestens 20% vorhanden.

Gemäß einer bevorzugten Ausführungsform enthält eine lochtransportierende Schicht enthaltend die Verbindung der Formel (I) zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReOs. Nochmals weiterhin bevorzugt sind Komplexe von Bismut in der Oxidationsstufe (III), insbesondere Bismut(III)-Komplexe mit elektronenarmen Liganden, insbesondere Carboxylat-Liganden.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden. Der p-Dotand liegt bevorzugt in einem Anteil von 1 bis 10 % in der p-dotierten Schicht vor.

Bevorzugt sind als p-Dotanden insbesondere die folgenden Verbindungen:

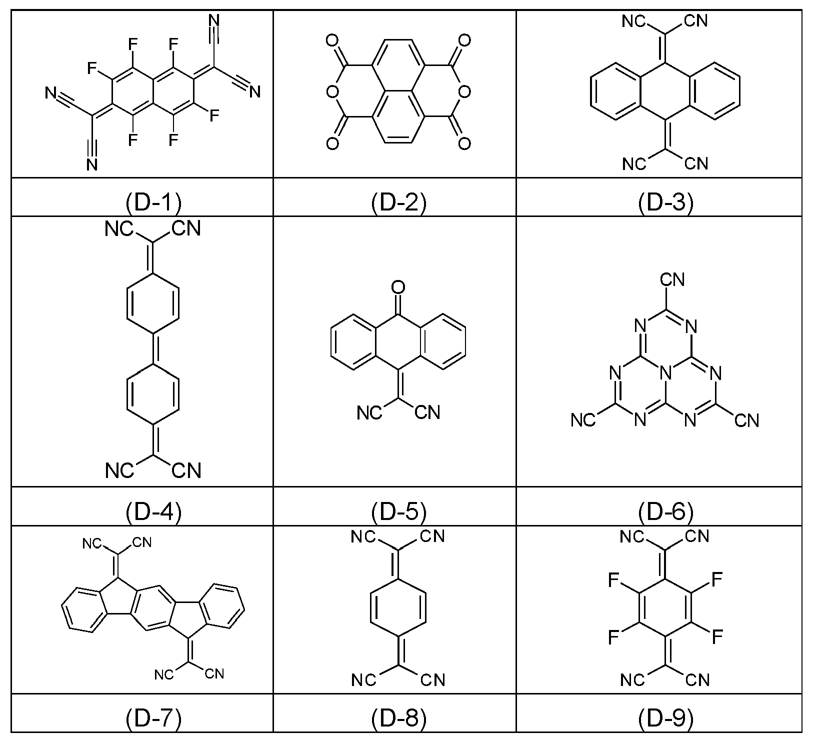

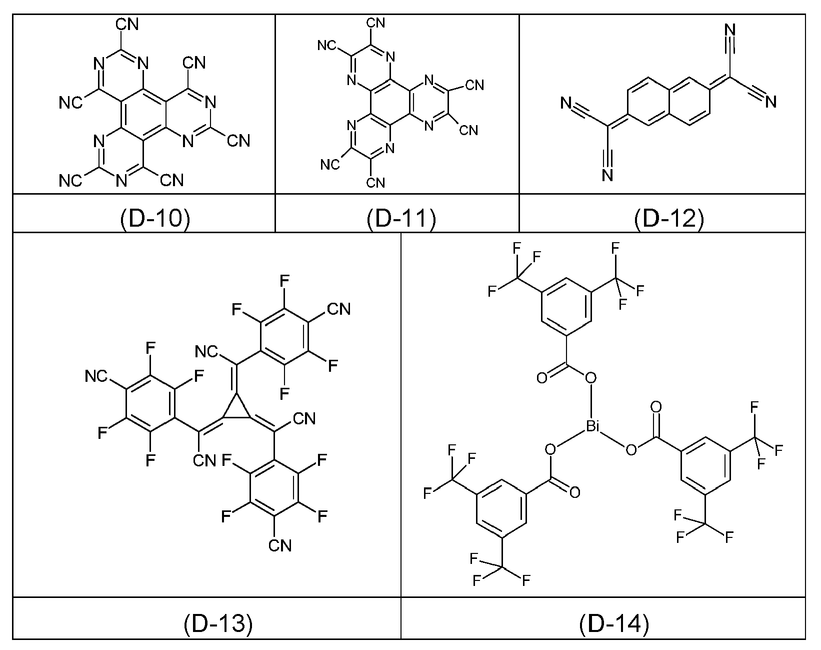

Gemäß einer bevorzugten Ausführungsform ist in der Vorrichtung eine Lochinjektionsschicht vorhanden, die einer der folgenden Ausführungsformen entspricht: a) sie enthält ein Triarylamin und einen p-Dotanden; oder b) sie enthält ein einzelnes elektronenarmes Material (Elektronenakzeptor). Gemäß einer bevorzugten Ausführungsform der Ausführungsform a) ist das Triarylamin ein Mono-Triarylamin, insbesondere eines der weiter unten genannten bevorzugten Triarylamin-Derivate. Gemäß einer bevorzugten Ausführungsform der Ausführungsform b) ist das elektronenarme Material ein Hexaazatriphenylenderivat, wie in US 2007/0092755 beschrieben.

Die Verbindung der Formel (I) kann in einer Lochinjektionsschicht, in einer Lochtransportschicht, und/oder in einer Elektronenblockierschicht der Vorrichtung enthalten sein. Wenn die Verbindung in einer Lochinjektionschicht oder in einer Lochtransportschicht vorliegt, ist sie bevorzugt p-dotiert, das heißt sie liegt gemischt mit einem p-Dotanden, wie oben beschrieben, in der Schicht vor.

Bevorzugt ist die Verbindung der Formel (I) in einer Elektronenblockierschicht enthalten. Bevorzugt ist sie in diesem Fall nicht p-dotiert. Weiterhin bevorzugt liegt sie in diesem Fall bevorzugt als Einzelverbindung in der Schicht vor, ohne Beimischung einer weiteren Verbindung.

Gemäß einer alternativen bevorzugten Ausführungsform liegt die Verbindung der Formel (I) in einer emittierenden Schicht als Matrixmaterial in Kombination mit einer oder mehreren emittierenden Verbindungen, vorzugsweise phosphoreszierenden emittierenden Verbindungen, eingesetzt. Die phosphoreszierenden emittierenden Verbindungen sind dabei bevorzugt gewählt aus rot phosphoreszierenden und grün phosphoreszierenden Verbindungen.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) als eine Komponente von Mixed-Matrix-Systemen, bevorzugt für phosphoreszierende Emitter, verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Bevorzugt ist weiterhin, wenn eines der Materialien gewählt ist aus Verbindungen mit großer Energiedifferenz zwischen HOMO und LUMO (Wide-Bandgap-Materialien). Die Verbindung der Formel (I) stellt in einem mixed-Matrix-System bevorzugt das Matrixmaterial mit lochtransportierenden Eigenschaften dar. Entsprechend ist, wenn die Verbindung der Formel (I) als Matrixmaterial für einen phosphoreszierenden Emitter in der emittierenden Schicht einer OLED eingesetzt wird, eine zweite Matrixverbindung in der emittierenden Schicht vorhanden, die elektronentransportierende Eigenschaften aufweist. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen.

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen.

In den oben genannten Schichten der Vorrichtung werden bevorzugt die folgenden Materialklassen eingesetzt:

### Phosphoreszierende Emitter:

Vom Begriff phosphoreszierende Emitter sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Emitter eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Weitere Beispiele für geeignete phosphoreszierende Emitter sind in der folgenden Tabelle gezeigt:

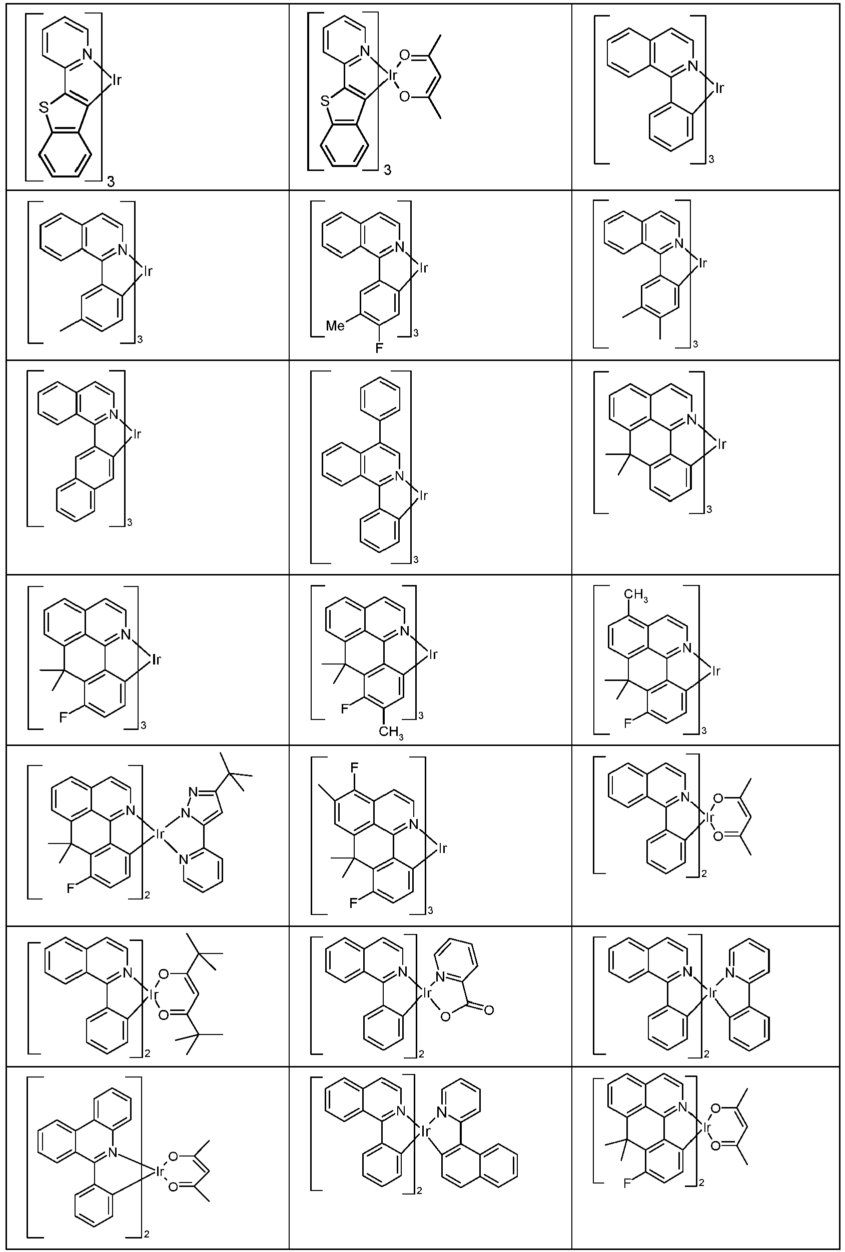

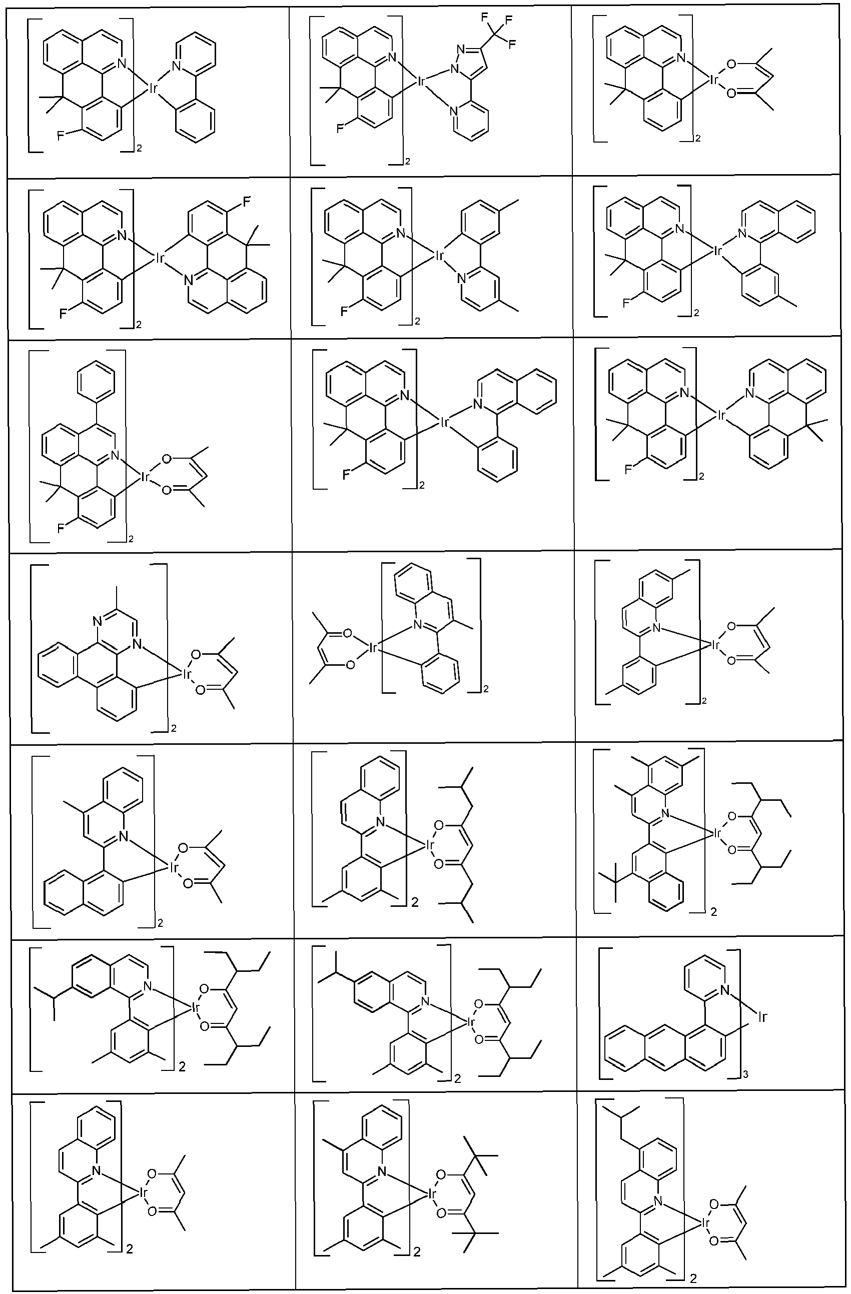

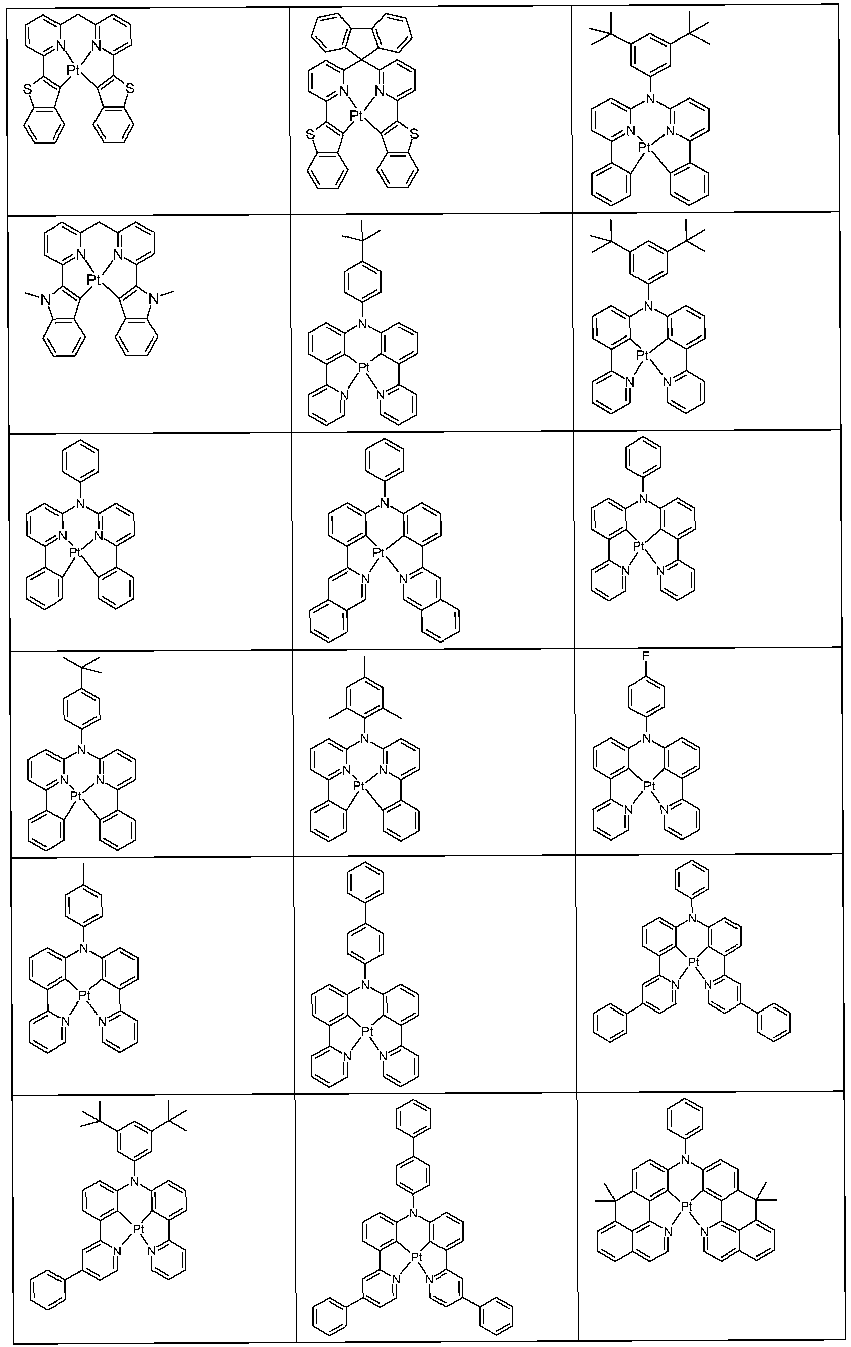

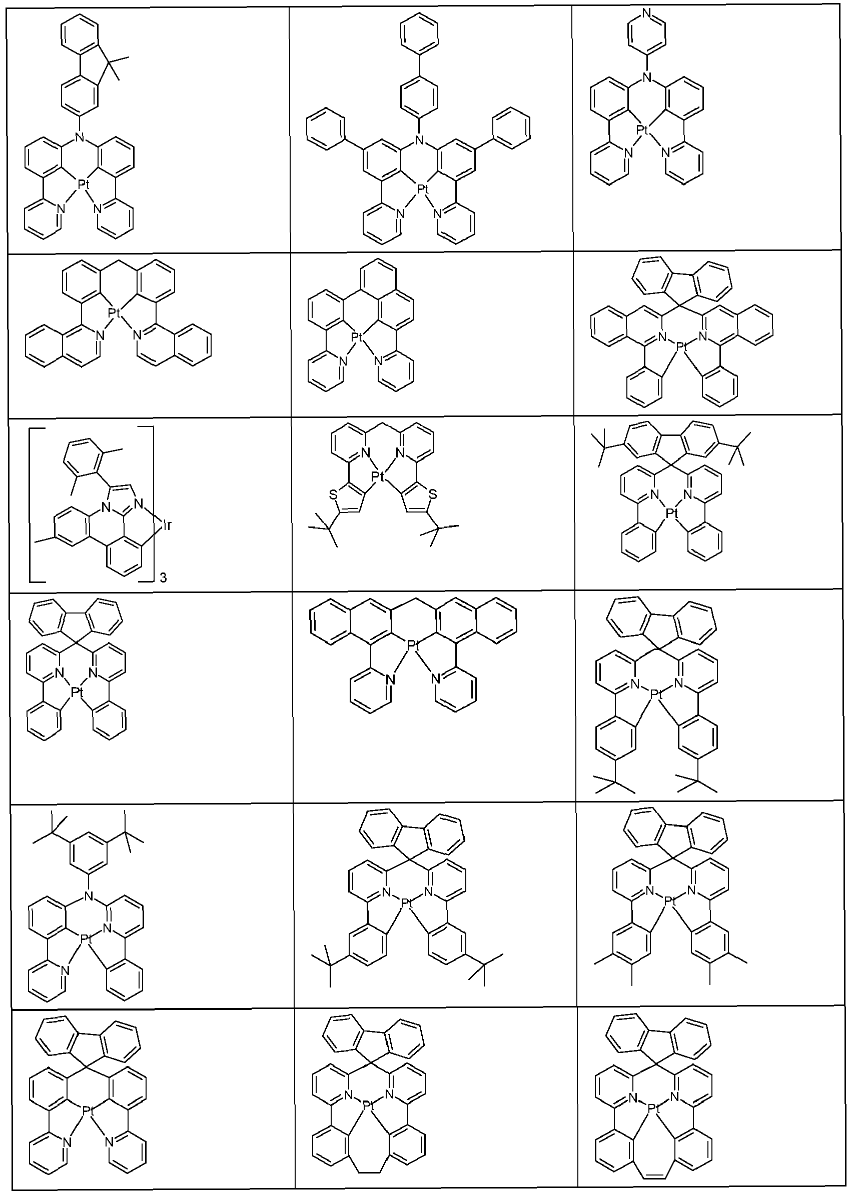

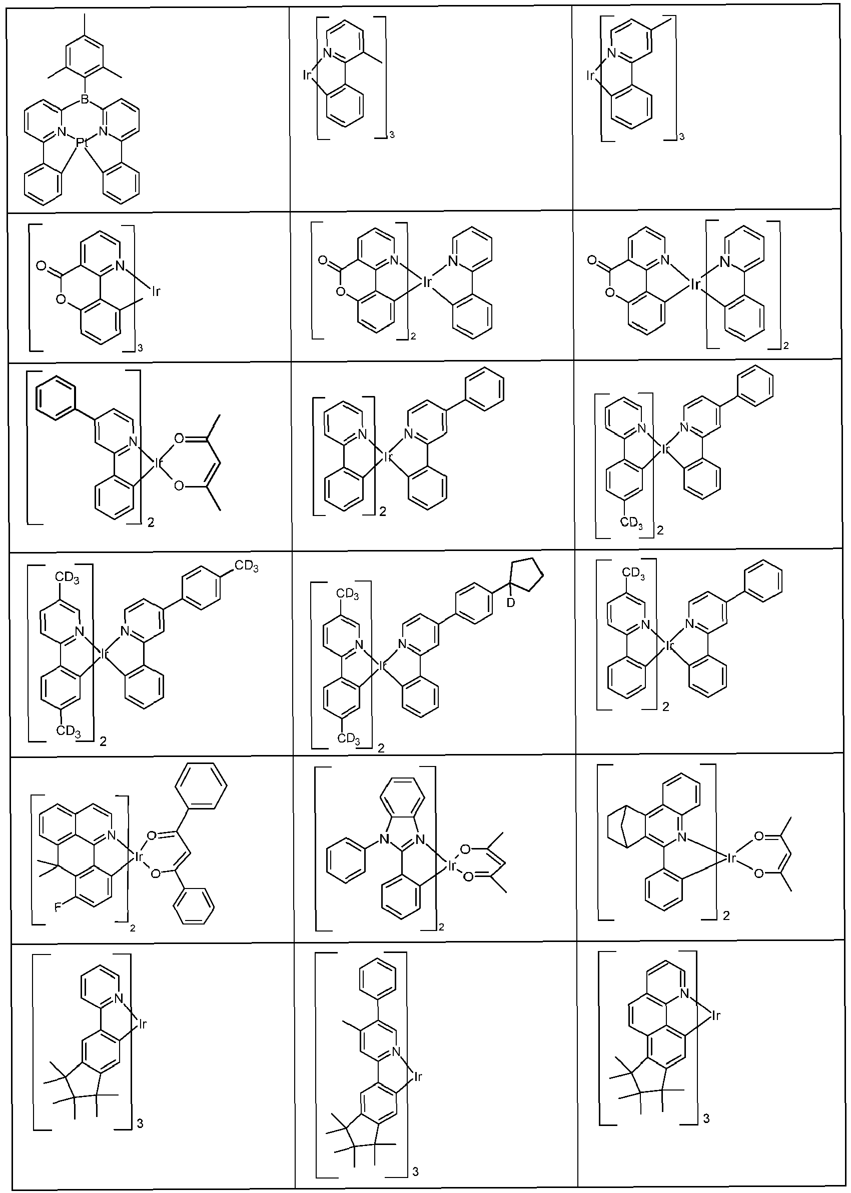

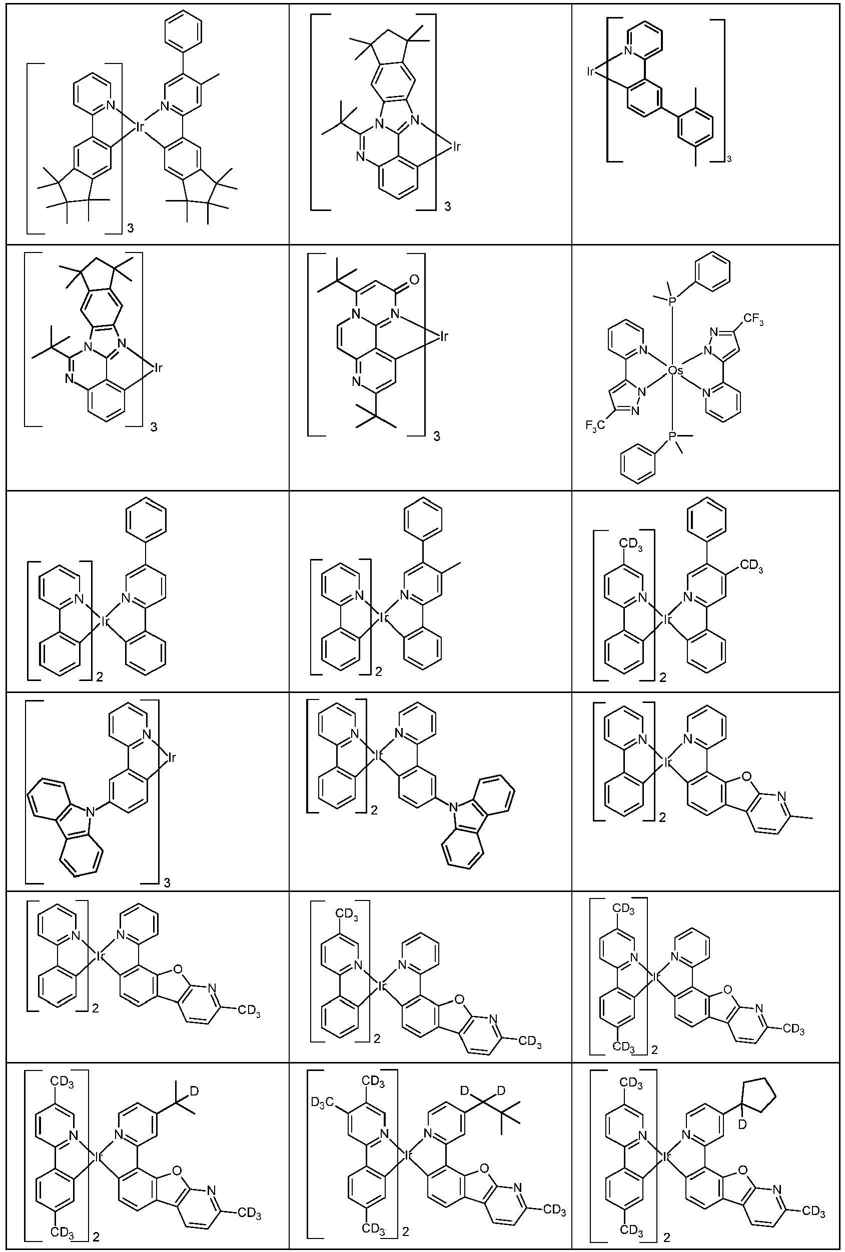

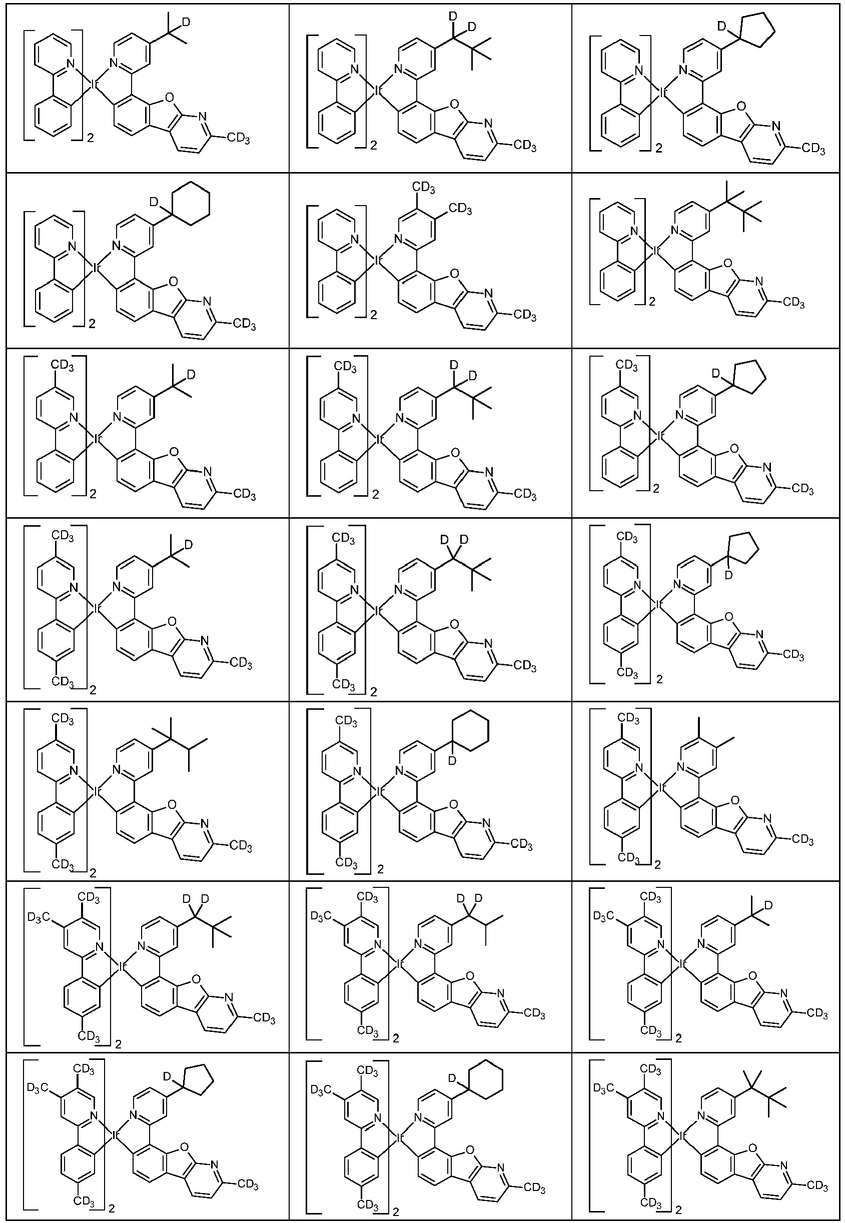

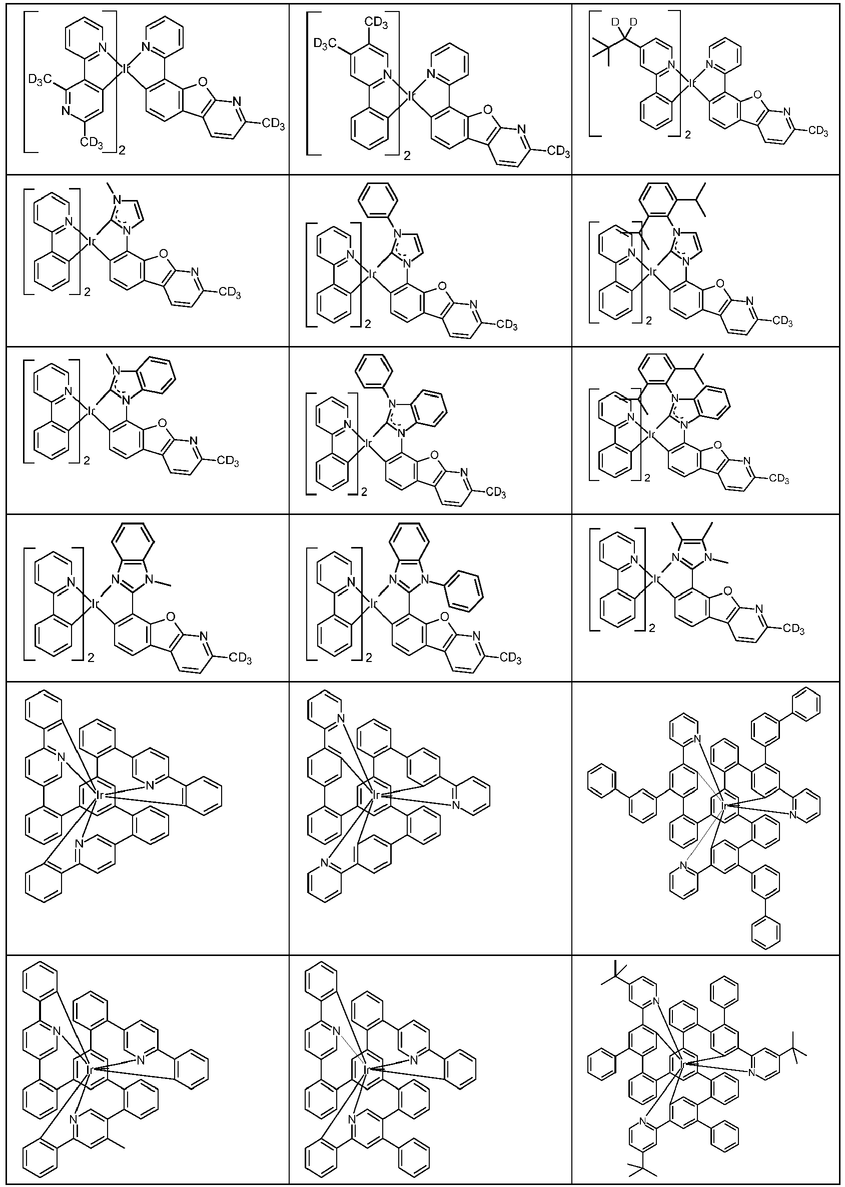

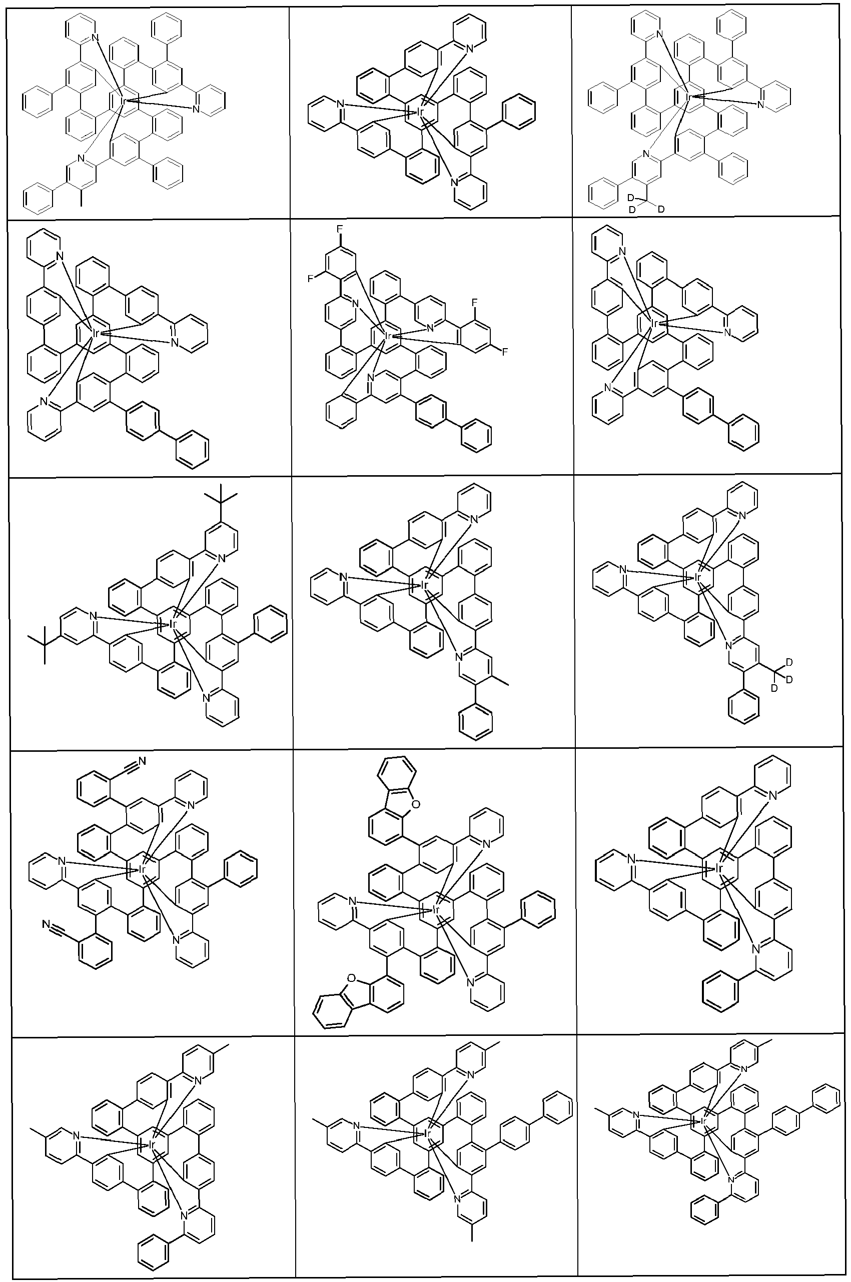

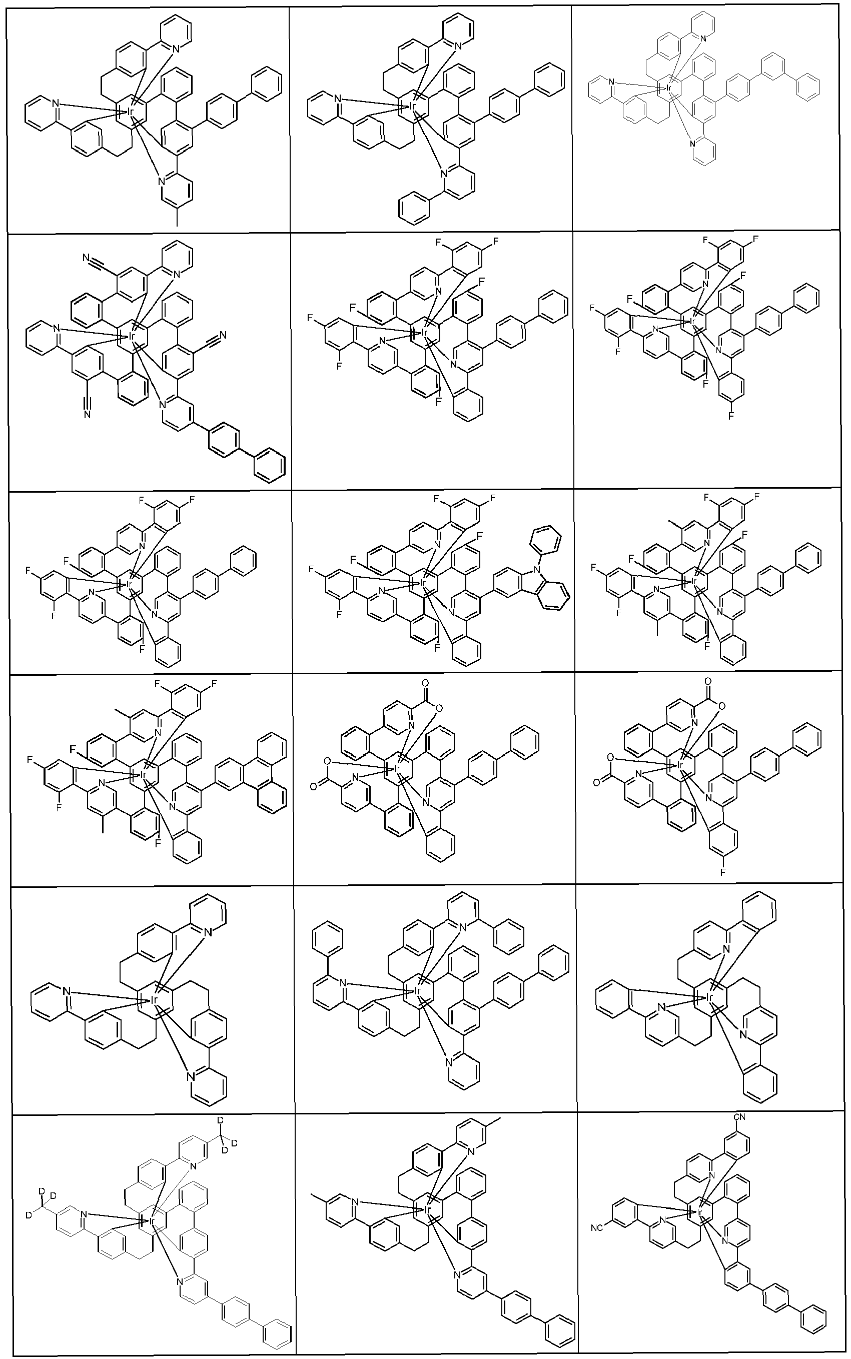

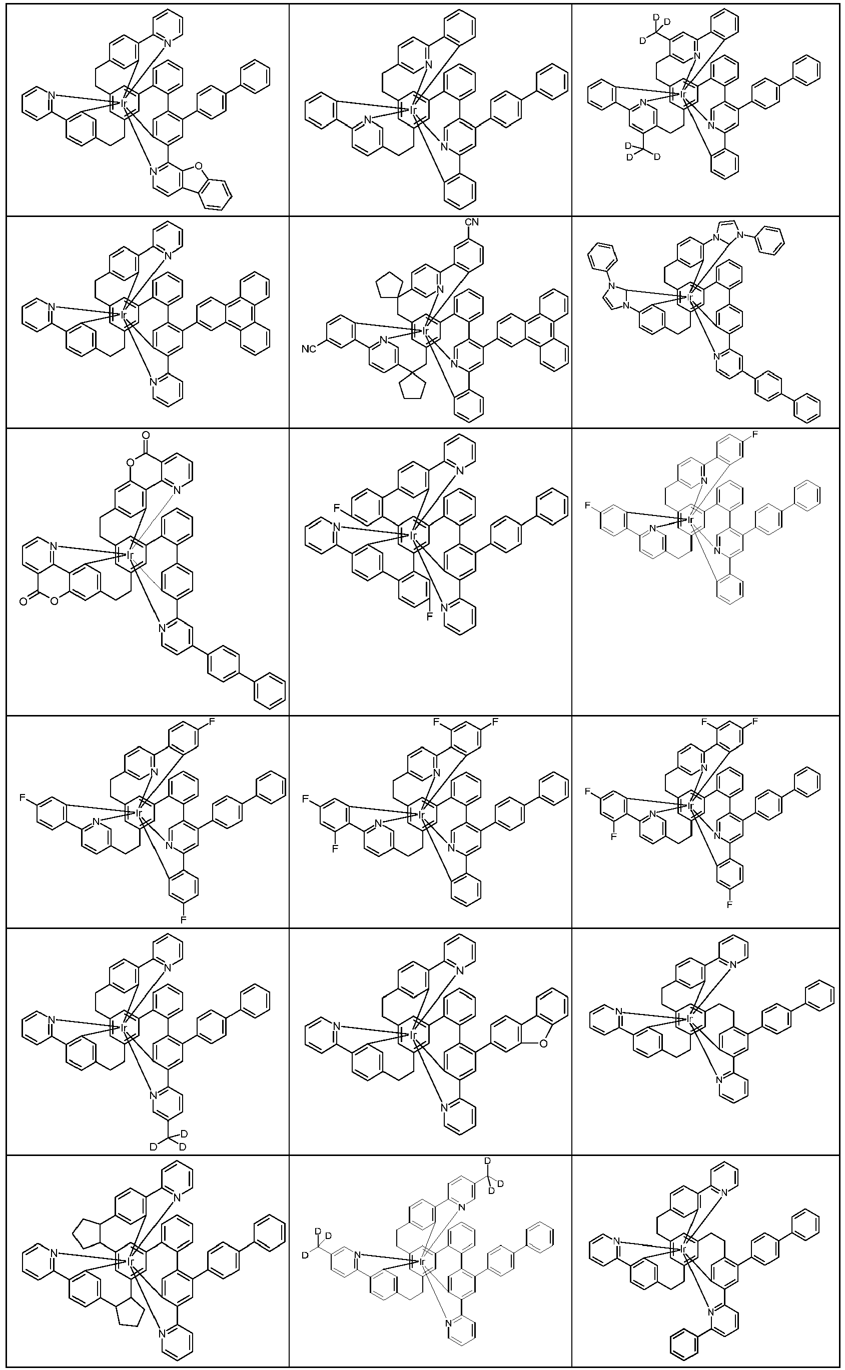

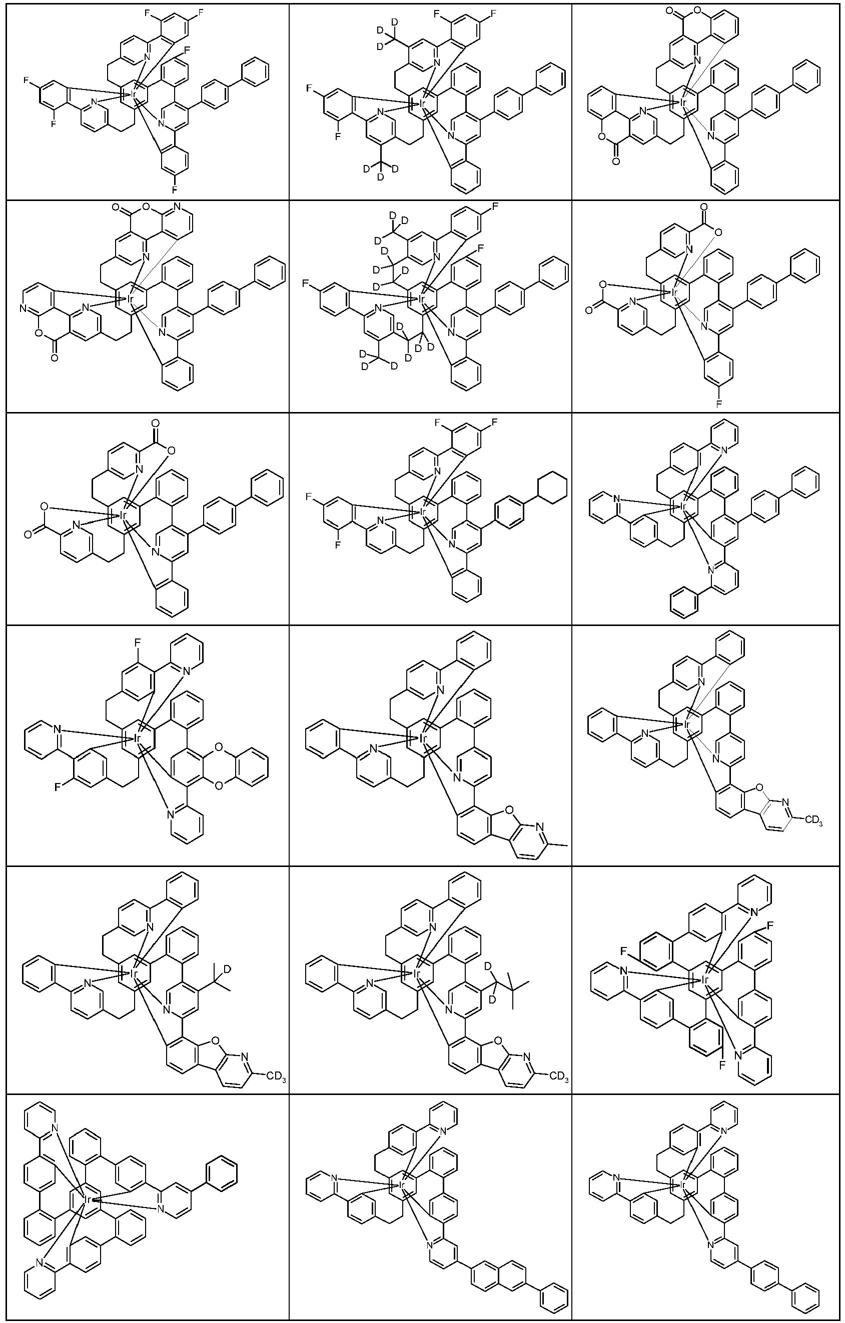

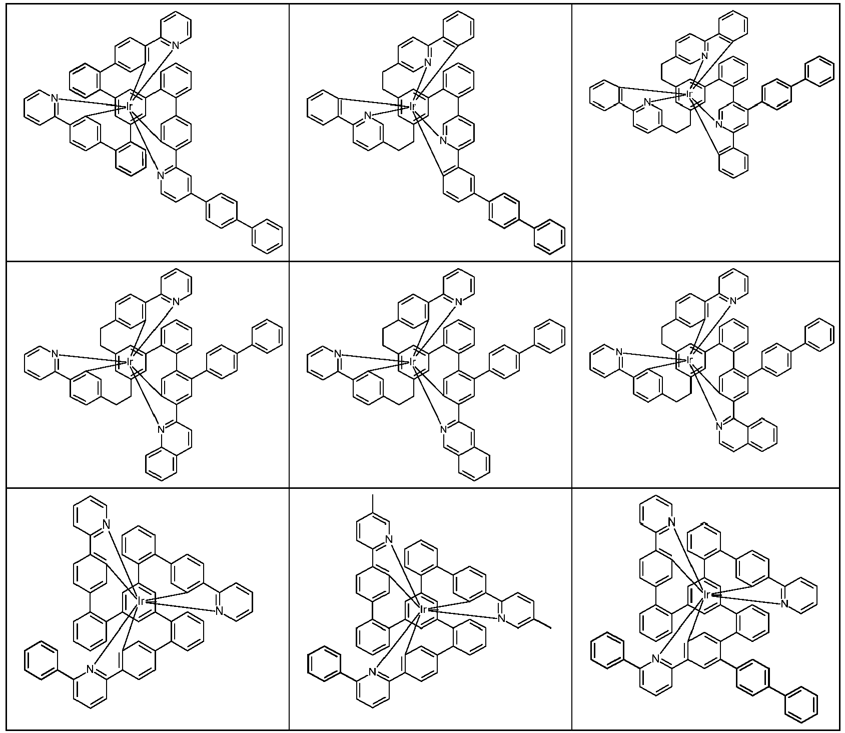

### Fluoreszierende Emitter:

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, Benzoindenofluorenamine bzw. -diamine, und Dibenzoindenofluorenamine bzw. -diamine, sowie Indenofluorenderivate mit kondensierten Arylgruppen. Ebenfalls bevorzugt sind Pyren-Arylamine. Ebenfalls bevorzugt sind Benzoindenofluoren-Amine, Benzofluoren-Amine, erweiterte Benzoindenofluorene, Phenoxazine, und Fluoren-Derivate, die mit Furan-Einheiten oder mit Thiophen-Einheiten verbunden sind.

### Matrixmaterialien für fluoreszierende Emitter:

Bevorzugte Matrixmaterialien für fluoreszierende Emitter sind ausgewählt aus den Klassen der Oligoarylene (z. B. 2,2',7,7'-Tetraphenylspirobifluoren), insbesondere der Oligoarylene enthaltend kondensierte aromatische Gruppen, der Oligoarylenvinylene, der polypodalen Metallkomplexe, der lochleitenden Verbindungen, der elektronenleitenden Verbindungen, insbesondere Ketone, Phosphinoxide, und Sulfoxide; der Atropisomere, der Boronsäurederivate oder der Benzanthracene. Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

### Matrixmaterialien für phosphoreszierende Emitter:

Bevorzugte Matrixmaterialien für phosphoreszierende Emitter sind neben den Verbindungen der Formel (I) aromatische Ketone, aromatische Phosphinoxide oder aromatische Sulfoxide oder Sulfone, Triarylamine, Carbazolderivate, z. B. CBP (N,N-Biscarbazolylbiphenyl) oder Carbazol-derivate, Indolocarbazolderivate, Indenocarbazolderivate, Azacarbazolderivate, bipolare Matrixmaterialien, Silane, Azaborole oder Boronester, Triazinderivate, Zinkkomplexe, Diazasilol- bzw. Tetraazasilol-Derivate, Diazaphosphol-Derivate, überbrückte Carbazol-Derivate, Triphenylenderivate, oder Lactame.

### Elektronentransportierende Materialien:

Geeignete elektronentransportierende Materialien sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, die gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate.

Bevorzugte Elektronentransport- und Elektroneninjektionsmaterialien sind in der folgenden Tabelle gezeigt:

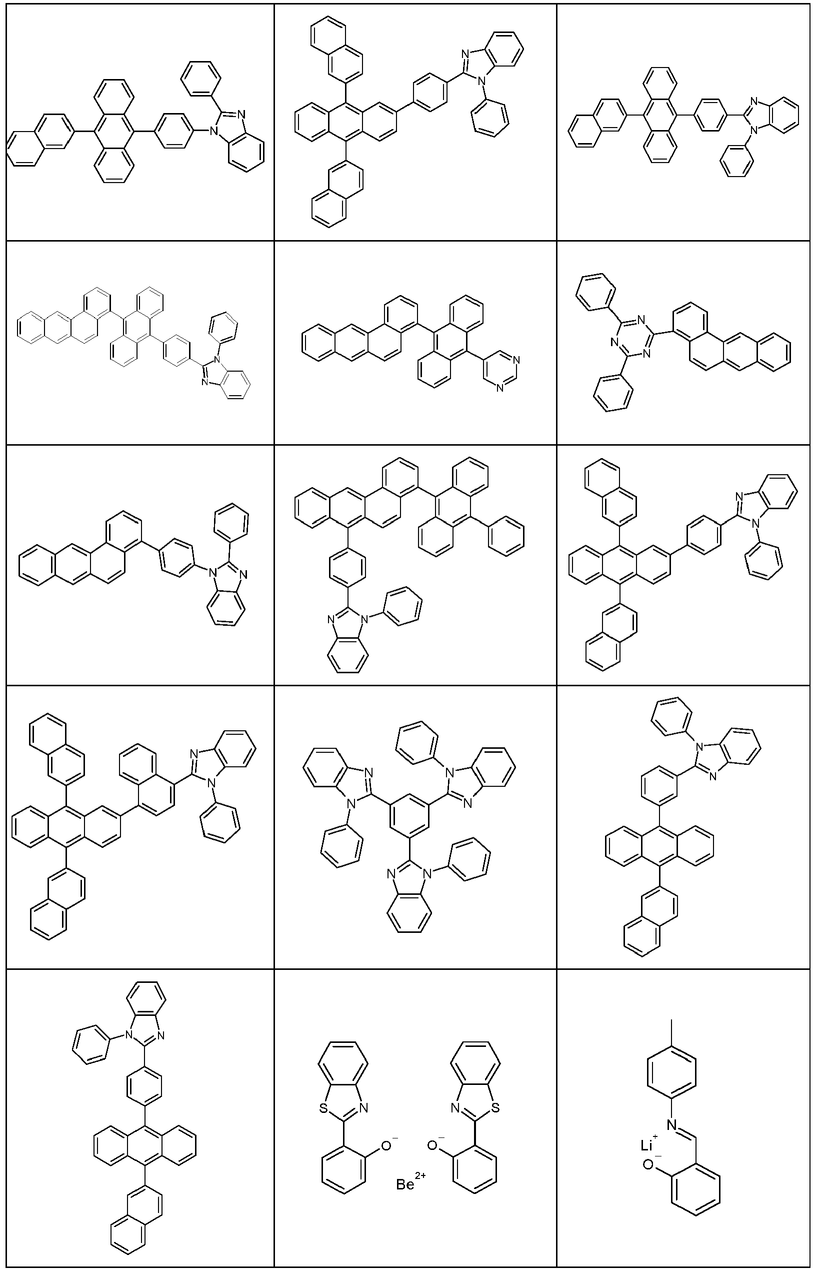

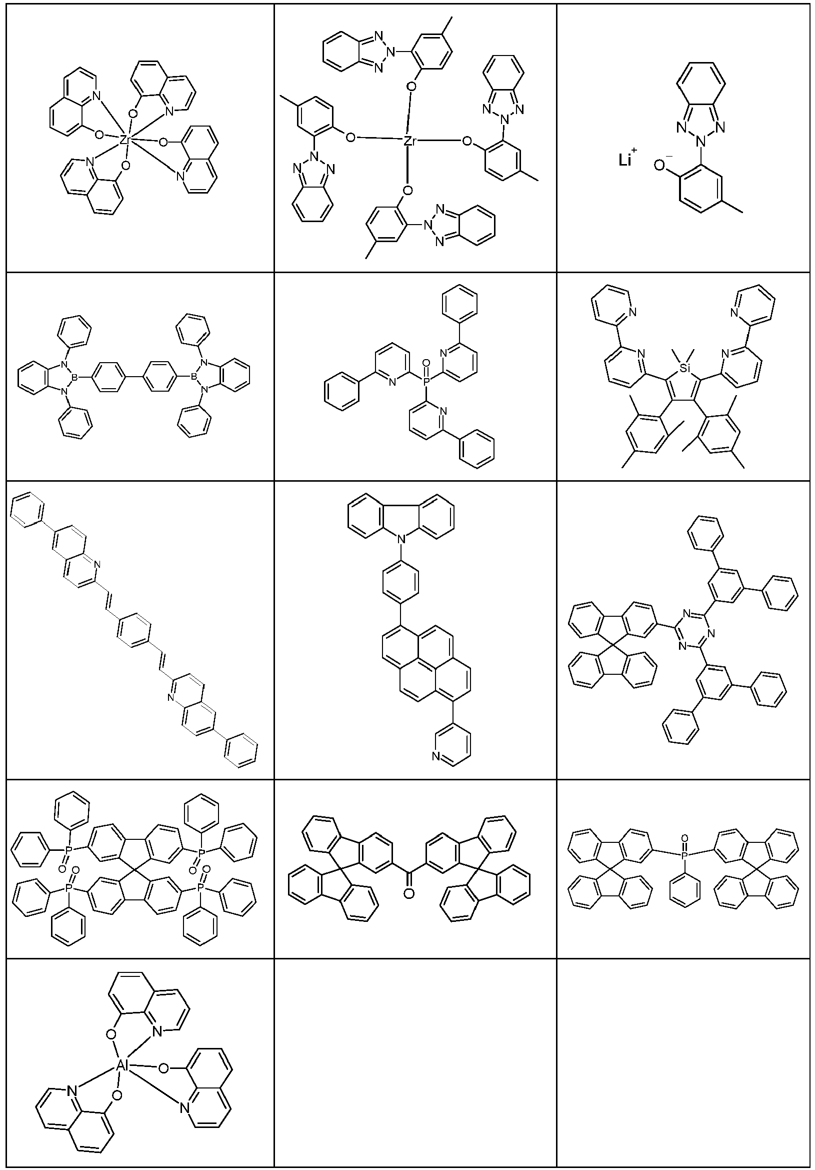

### Lochtransportierende Materialien:

Weitere Verbindungen, die neben den Verbindungen der Formel (I) bevorzugt in lochtransportierenden Schichten der erfindungsgemäßen OLEDs eingesetzt werden, sind Indenofluorenamin-Derivate, Aminderivate, Hexaazatriphenylenderivate, Aminderivate mit kondensierten Aromaten, Monobenzoindenofluorenamine, Dibenzoindenofluorenamine, Spirobifluoren-Amine, Fluoren-Amine, Spiro-Dibenzopyran-Amine, Dihydroacridin-Derivate, Spirodibenzofurane und Spirodibenzothiophene, Phenanthren-Diarylamine, Spiro-Tribenzotropolone, Spirobifluorene mit meta-Phenyldiamingruppen, Spiro-Bisacridine, Xanthen-Diarylamine, und 9,10-Dihydroanthracen-Spiroverbindungen mit Diarylaminogruppen. Insbesondere bevorzugt sind hierfür die in der Tabelle von S. 76-80 der WO 2020/109434 A1 offenbarten Verbindungen.

Die oben genannten Verbindungen gewählt aus Indenofluorenamin-Derivaten, Aminderivaten, Hexaazatriphenylenderivaten, Aminderivaten mit kondensierten Aromaten, Monobenzoindenofluorenaminen, Dibenzoindenofluorenaminen, Spirobifluoren-Aminen, Fluoren-Aminen, Spiro-Dibenzopyran-Aminen, Dihydroacridin-Derivaten, Spirodibenzofuranen und Spirodibenzothiophenen, Phenanthren-Diarylaminen, Spiro-Tribenzotropolonen, Spirobifluorenen mit meta-Phenyldiamingruppen, Spiro-Bisacridinen, Xanthen-Diarylaminen, und 9,10-Dihydroanthracen-Spiroverbindungen mit Diarylaminogruppen, und den in der Tabelle von S. 76-80 der WO 2020/109434 A1 offenbarten Verbindungen, sind allgemein zur Verwendung in Schichten mit lochtransportierender Funktion geeignet. Zu den Schichten mit lochtransportierender Funktion gehören Lochinjektionsschichten, Lochtransportschichten, Elektronenblockierschichten und auch emittierende Schichten. Bei Verwendung in emittierenden Schichten sind die Verbindungen als Matrixmaterialien, insbesondere als Matrixmaterialien mit lochtransportierenden Eigenschaften, geeignet. Dies gilt für OLEDs jeglichen Aufbaus, nicht nur für OLEDs gemäß den Definitionen der vorliegenden Anmeldung.

Insbesondere geeignet zur Verwendung in Schichten mit lochtransportierender Funktion jeglicher OLEDs, nicht nur der OLEDs gemäß den Definitionen der vorliegenden Anmeldung, sind weiterhin die folgenden Verbindungen:

| | |
|---|---|
| 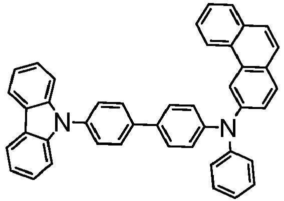 | 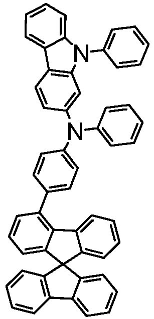 |
| HT-1 | HT-2 |
| WO 2013/182263 | WO 2013/120577 |
| 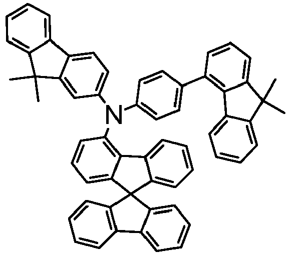 | 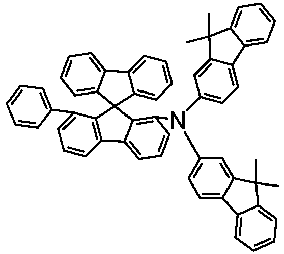 |
| HT-3 | HT-4 |
| WO 2013/120577 | WO 2013/120577 |
| 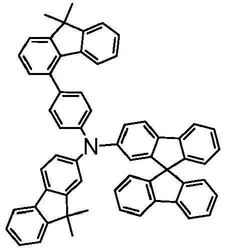 | 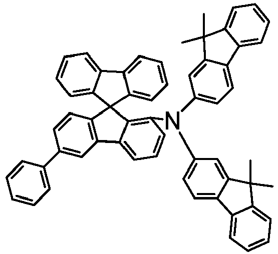 |
| HT-5 | HT-6 |
| WO 2012/034627 | WO 2013/120577 |
| 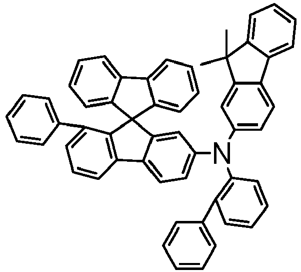 | 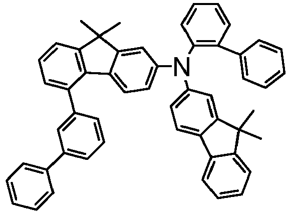 |
| HT-7 | HT-8 |
| WO 2012/034627 | WO 2019/115577 |
| 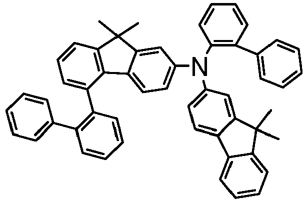 | 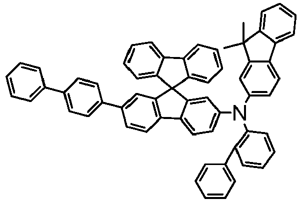 |
| HT-9 | HT-10 |
| WO 2019/115577 | WO 2012/034627 |
| 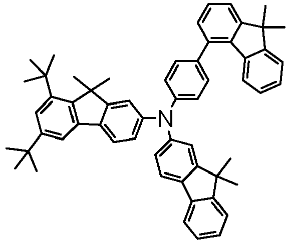 | 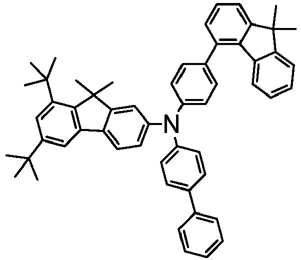 |
| HT-11 | HT-12 |
| WO 2019/115577 | WO 2019/115577 |
| 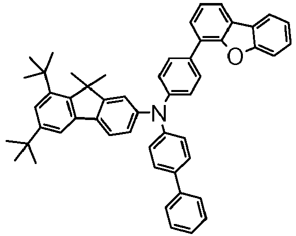 | 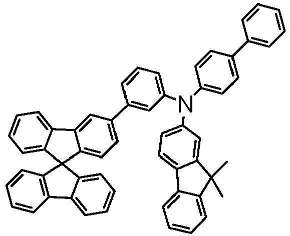 |
| HT-13 | HT-14 |
| WO 2019/115577 | WO 2013/120577 |
| 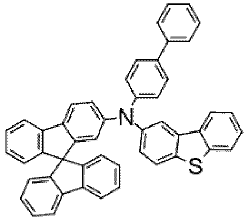 | 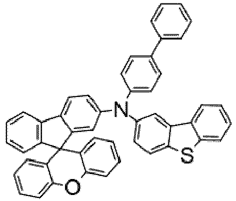 |
| HT-15 | HT-16 |
| WO 2012/034627 | WO 2014/072017 |

Die Verbindungen HT-1 bis HT-16 sind allgemein zur Verwendung in lochtransportierenden Schichten geeignet. Ihre Verwendung ist nicht beschränkt auf bestimmte OLEDs, wie beispielsweise die in der vorliegenden Anmeldung beschriebenen OLEDs.

Die Verbindungen HT-1 bis HT-16 können nach den Vorschriften hergestellt werden, die in den in der Tabelle oben genannten Offenlegungsschriften offenbart sind. Die weitere Lehre betreffend Verwendung und Herstellung der Verbindungen, die in den in der obenstehenden Tabelle aufgeführten Offenlegungsschriften offenbart ist, ist hiermit explizit einbezogen und ist bevorzugt mit der oben genannten Lehre betreffend die Verwendung der oben genannten Verbindungen als lochtransportierende Materialien zu kombinieren. Die Verbindungen HT-1 bis HT-16 zeigen hervorragende Eigenschaften bei der Verwendung in OLEDs, insbesondere hervorragende Lebensdauer und Effizienz.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Die Vorrichtung wird nach Aufbringen der Schichten, je nach Anwendung, strukturiert, kontaktiert und schließlich versiegelt, um schädigende Effekte von Wasser und Luft auszuschließen.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen eingesetzt werden.

### Beispiele

### A) Synthesebeispiele

### 1) Synthese N-[4-(naphthalen-1-yl)phenyl]-N-(4-{8-oxatricyclo[7.4.0.0^{2,7}]trideca-1(13),2,4,6,9,11-hexaen-6-yl}phenyl)-9,9'-spirobi[fluoren]-1-amin 1a

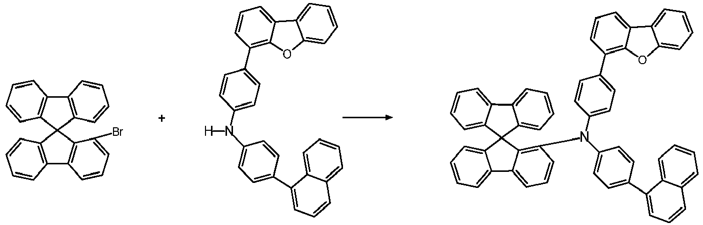

16.8 g 4-(naphthalen-1-yl)-N-(4-{8-oxatricyclo[7.4.0.0^{2,7}]trideca-1(13),2,4,6,9,11-hexaen-6yl}phenyl) anilin (36.4 mmol), und 1-bromo-9,9'-spirobi[fluorene] (13.8g, 34.7 mol) werden in 250 mL Toluol gelöst. Die Lösung wird entgast und mit N₂ gesättigt. Danach wird sie mit 1 g (5.1 mmol) S-Phos und 1.6 g (1.7 mmol) Pd₂(dba)₃ versetzt und anschließend werden 5 g Natrium-tert-butylat (52.05 mmol) zugegeben. Die Reaktionsmischung wird über Nacht unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Substanz wird abschließend im Hochvakuum sublimiert, Reinheit beträgt 99.9%, bestimmt über HPLC. Die Ausbeute beträgt 7.1 g (26% d. Th).

Analog dazu werden die folgenden Verbindungen hergestellt:

| **Bsp.** | **Br-spiro** | **Amin** | **Produkt** |
|---|---|---|---|
| 1b | 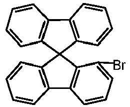 | 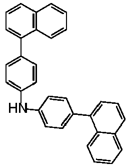 | 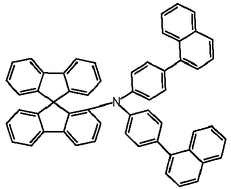 |
| 1c | 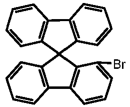 | 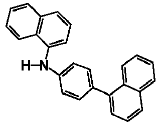 | 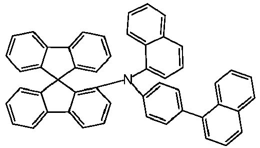 |
| 1d | 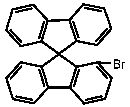 | 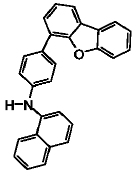 | 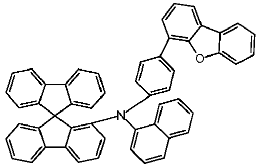 |
| 1e | 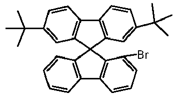 | 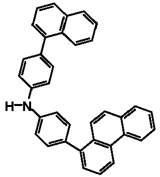 | 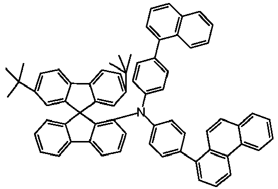 |
| 1f | 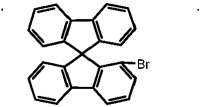 | 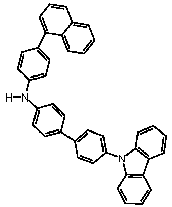 | 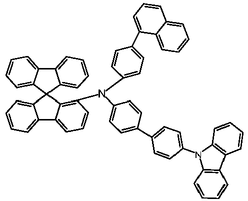 |
| 1g | 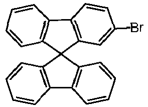 | 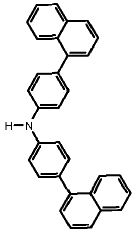 | 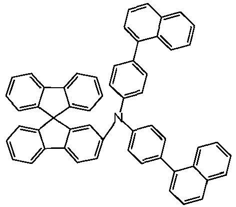 |
| 1h | 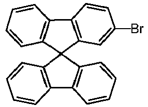 | 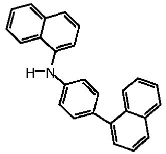 | 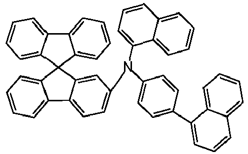 |
| 1i | 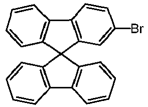 | 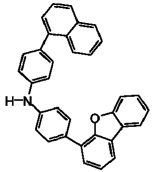 | 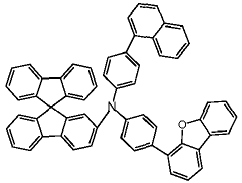 |
| 1j | 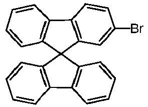 | 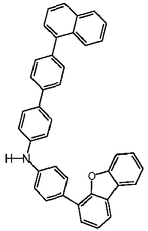 | 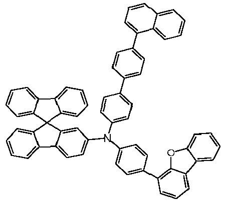 |
| 1k | 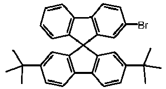 | 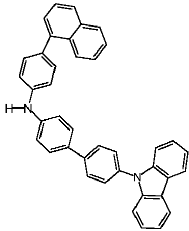 | 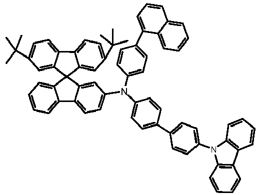 |
| 1l | 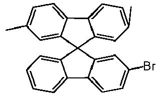 | 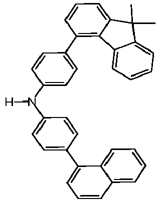 | 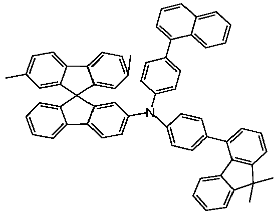 |
| 1m | 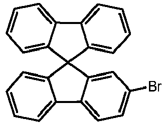 | 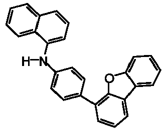 | 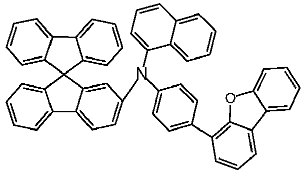 |
| 1n | 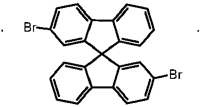 | 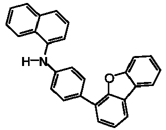 | 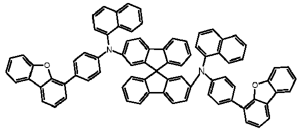 |
| 1o | 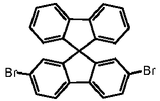 | 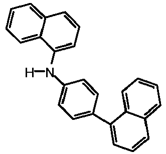 | 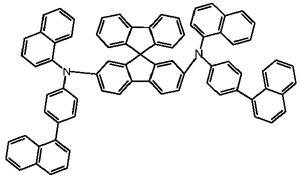 |
| 1p | 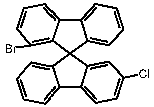 | 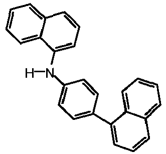 | 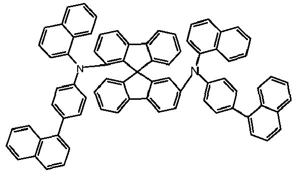 |
| 1p | 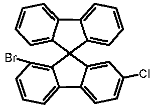 | 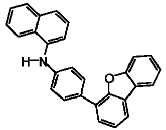 | 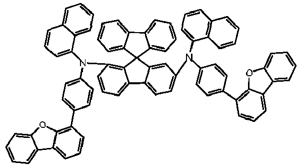 |

### 2) Synthese 1-{[4-(naphthalen-1-yl)phenyl](4-{8-oxatricyclo[7.4.0.0^{2,7}]trideca-1(13),2,4,6,9,11-hexaen-6-yl}phenyl)amino}-9H-fluoren-9-one 2a

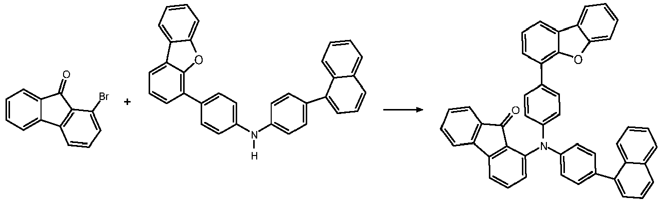

N-[4-(naphthalen-1-yl)phenyl]-4-{8-oxatricyclo[7.4.0.0^{2,7}]trideca-1(9),2(7),3,5,10,12-hexaen-6-yl}aniline (40.3 g; 87.4 mmol), 1-Bromo-fluoren-9-one (22.6 g, 87.4 mmol) und Natrium tert-pentoxide (20.2 g, 174.7 mmol) werden in 700 mL Toluol gelöst. Die Lösung wird entgast und mit N₂ gesättigt. Danach wird sie mit tri-tertbutylphosphin (3.5 ml; 3.5 mmol, 1M in Toluol) und 1.6 g (1,7 mmol) Pd₂(dba)₃ versetzt. Die Reaktionsmischung wird über Nacht unter Schutzatmosphäre zum Sieden erhitzt. Das Gemisch wird abgekühlt und zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 49.2 g (80% d. Th).

Analog dazu werden die folgenden Verbindungen hergestellt:

| **Bsp.** | **Bromo-fluorenon** | **Amin** | **Produkt: 1-Amin-fluorenon** |
|---|---|---|---|
| 2b | 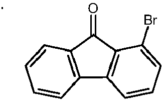 | 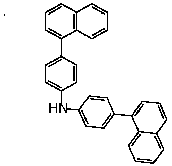 | 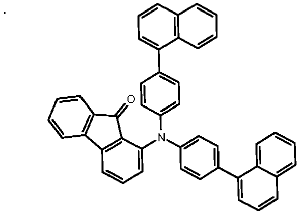 |
| 2c | 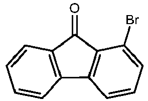 | 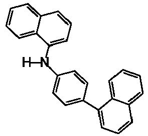 | 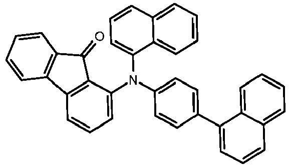 |
| 2d | 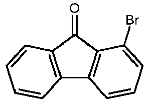 | 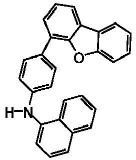 | 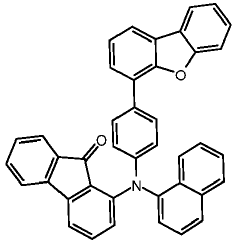 |
| 2e | 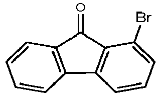 | 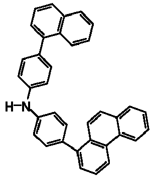 | 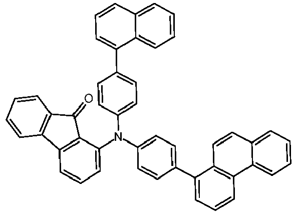 |
| 2f | 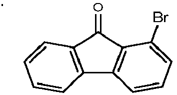 | 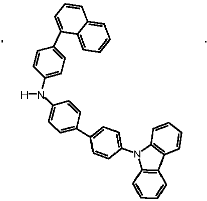 | 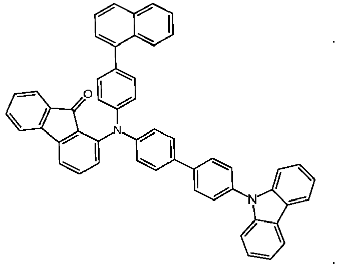 |
| 2g | 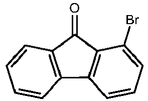 | 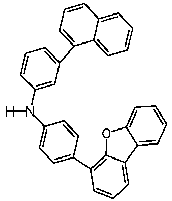 | 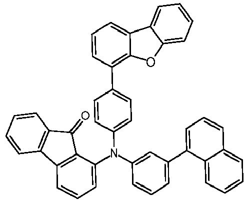 |
| 2h | 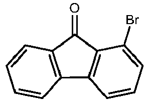 | 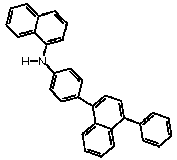 | 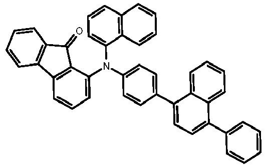 |
| 2i | 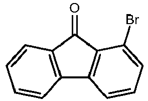 | 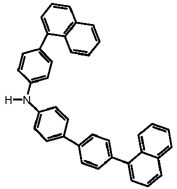 | 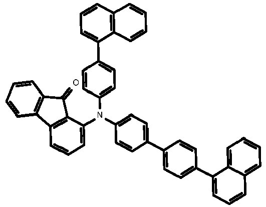 |
| 2j | 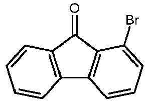 | 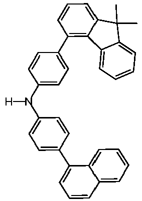 | 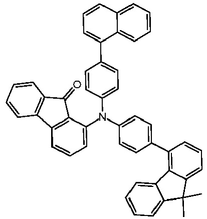 |

### 3) Synthese N-[4-(naphthalen-1-yl)phenyl]-N-(4-{8-oxatricyclo[7.4.0.0^{2,7}]trideca-1(13),2,4,6,9,11-hexaen-6-yl}phenyl)-9,9'-spirobi[fluoren]-1-amin 1a 3a

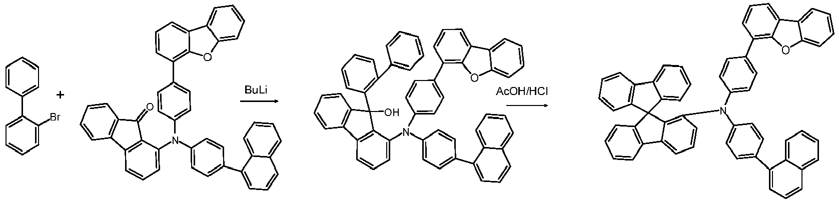

25 g (105.2 mmol) 2-Bromo-1,1'-biphenyl werden in einem ausgeheizten Kolben in 300 mL getrocknetem THF gelöst. Die Reaktionsmischung wird auf - 78°C gekühlt. Bei dieser Temperatur werden 39.3 mL einer 2,5 M-Lösung n-BuLi in Hexan (98.2 mmol) langsam zugetropft. Der Ansatz wird 1 Stunde bei - 70°C nachgerührt. Anschließend werden 49.2 g 1-{[4-(naphthalen-1-yl)phenyl](4-{8-oxatricyclo[7.4.0.0^{2,7}]trideca-1(13),2,4,6,9,11-hexaen-6-yl}phenyl)amino}-9H-fluoren-9-one (70.1 mmol) in 300 ml THF gelöst und bei - 70°C zugetropft. Nach beendeter Zugabe lässt man die Reaktionsmischung langsam auf Raumtemperatur erwärmen, stoppt die Reaktion mit NH₄Cl und engt anschließend am Rotationsverdampfer ein. Der Feststoff wird in 500 mL Toluol gelöscht und anschließend werden 720 mg (3.8 mmol) p-Toluol-sulfonsäure zugegeben. Der Ansatz wird 6 Stunden unter Reflux erhitzt, danach auf Raumtemperatur abkühlen gelassen und mit Wasser versetzt. Der ausgefallene Feststoff wird abgesaugt und mit Heptan nachgewaschen (40.10 g, 68% Ausbeute).

Der verbleibende Rückstand wird aus Heptan/Toluol umkristallisiert. Die Substanz wird abschließend im Hochvakuum sublimiert, Reinheit beträgt 99.9%, bestimmt über HPLC. Die Ausbeute beträgt 19.2 g (48% d. Th).

Analog dazu werden die folgenden Verbindungen hergestellt:

| **Bsp.** | **Edukt: 1-Amin-fluorenon** | **Br-Biphenyl** | **Produkt** |
|---|---|---|---|
| 3b | 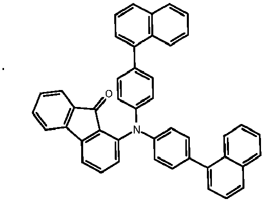 | 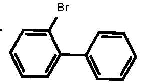 | 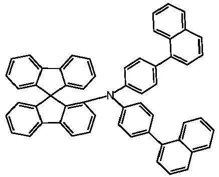 |
| 3c | 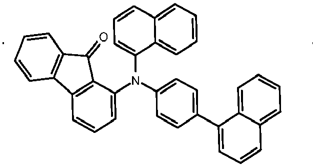 | 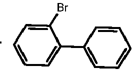 | 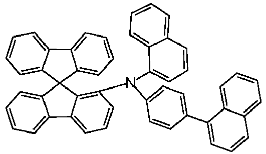 |
| 3d | 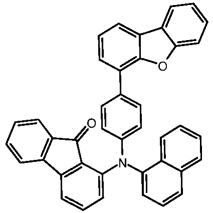 | 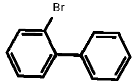 | 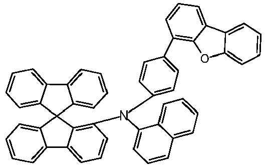 |
| 3e | 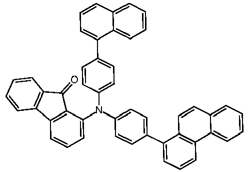 | 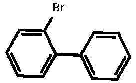 | 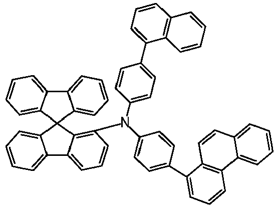 |
| 3f | 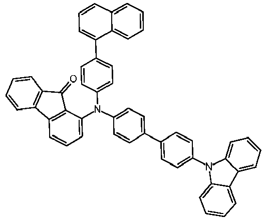 | 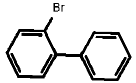 | 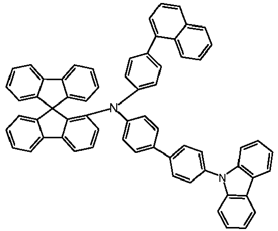 |
| 3g | 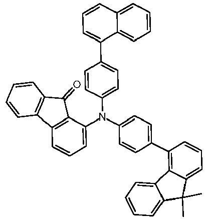 | 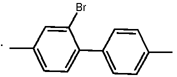 | 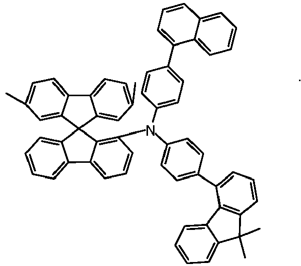 |

### 4) Synthesis of N-[4-(naphthalen-1-yl)phenyl]-N-(4-{8-oxatricyclo[7.4.0.0^{2,7}]trideca-1(13),2,4,6,9,11- hexaen-6-yl}phenyl)-4-{9,9'-spirobi[fluoren]-8-yl}aniline 4a

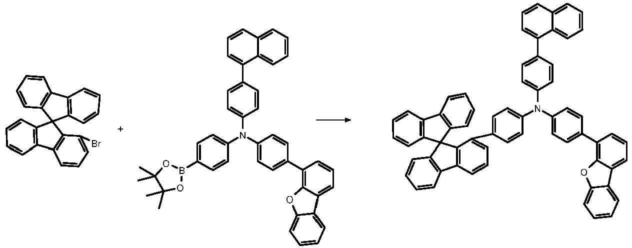

25.9 g (39 mmol) 4-(naphthalen-1-yl)-N-(4-{8-oxatricyclo[7.4.0.0^{2,7}]trideca-1(13),2,4,6,9,11-hexaen-6-yl} phenyl)-N-[4-(4,4,5,5-tetramethyl-1,3,2-dioxaborolan-2-yl)phenyl]anilin und 16.6 g (42 mmol) 8-bromo-9,9'-spirobi[fluorene] werden in 400 mL Dioxan und 13.7 g Caesiumfluorid (90 mmol) suspendiert. Zu dieser Suspension werden 4.0 g (5.4 mmol) Palladium dichlorid-bis(tricyclohexylphosphin) gegeben, und die Reaktionsmischung wird 18 h unter Rückfluss erhitzt. Nach Abkühlen wird die organische Phase abgetrennt, über Kieselgel filtriert, dreimal mit 80 mL Wasser gewaschen und anschließend zur Trockene eingeengt. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%, bestimmt über HPLC. Die Ausbeute beträgt 11 g (33% d. Th).

Analog dazu werden die folgenden Verbindungen hergestellt:

| **Bsp.** | **Spiro** | **Amin** | **Produkt** |
|---|---|---|---|
| 4b | 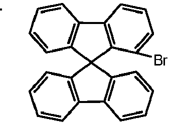 | 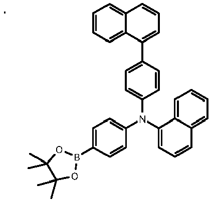 | 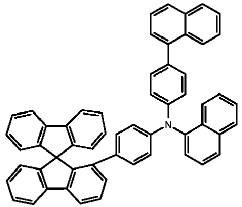 |
| 4c | 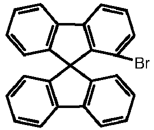 | 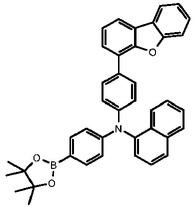 | 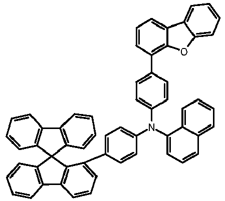 |
| 4d | 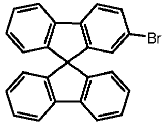 | 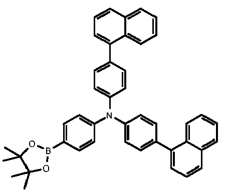 | 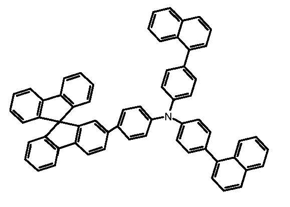 |
| 4e | 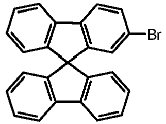 | 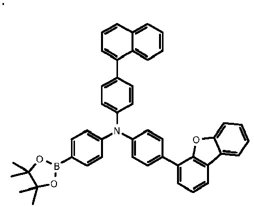 | 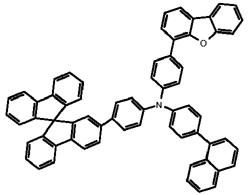 |
| 4f | 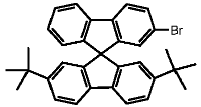 | 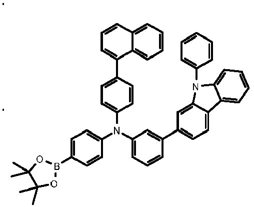 | 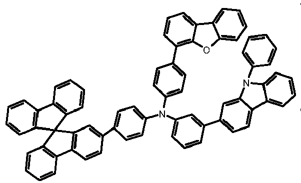 |

### 5) Synthesis of 1-(4-Chloro-phenyl)-fluoren-9-one 5a

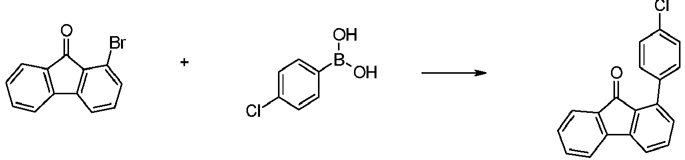

76 g (486 mmol) 4-Chlorphenylboronsäure,120 g (463 mmol) 1-Brom-Fluoren und 16 g (14 mmol) Pd(Ph₃P)₄ werden in 1.9L THF gelöst. Die Lösung wird entgast und mit N₂ gesättigt und 463 ml 2 M Kaliumcarbonatlösung werden dieser Suspension langsam zugegeben. Die Reaktionsmischung wird über Nacht unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der Rückstand wird durch Kristallisation mit MeOH gereinigt. Ausbeute: 125 g (93 % der Theorie), Reinheit nach HPLC >98%.

Analog dazu werden die folgenden Verbindungen hergestellt:

| **Bsp.** | **1-Br-Fluorenon** | **Boronsäure** | **Produkt** |
|---|---|---|---|
| 5b | 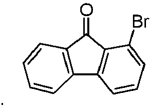 | 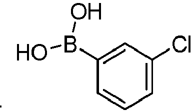 | 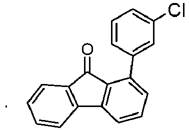 |
| 5c | 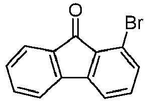 | 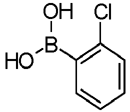 | 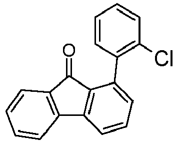 |
| 5d | 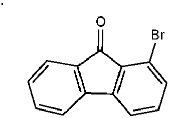 | 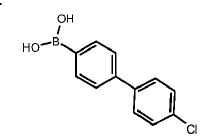 | 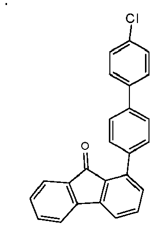 |
| 5e | 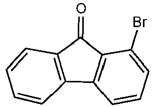 | 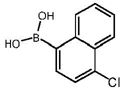 | 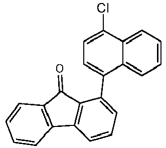 |

### 6)Synthese 1-(4-{[4-(naphthalen-1-yl)phenyl](4-{8-oxatricyclo[7.4.0.0^{2,7}]trideca-1(13),2,4,6,9,11-hexaen-6-yl}phenyl)amino}phenyl)-9H-fluoren-9-one 6a

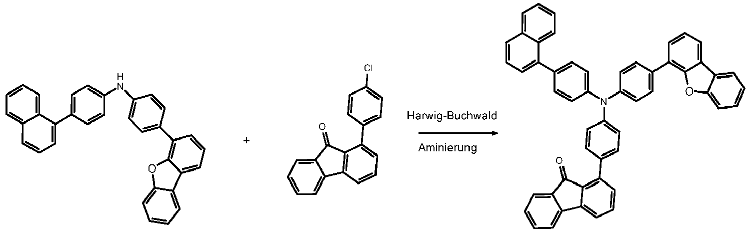

N-[4-(naphthalen-1-yl)phenyl]-4-{8-oxatricyclo[7.4.0.0^{2,7}]trideca-1(9),2(7),3,5,10,12-hexaen-6-yl}aniline (32 g, 69 mmol),1-1-(4-Chlor-Phenyl)-fluoren-9-one, (20 g, 69 mmol) und Natrium tert-butylat (9.5 g, 138 mmol) werden in 300 mL Toluol gelöst. Die Lösung wird entgast und mit N₂ gesättigt. Danach wird sie mit tri-tertbutylphosphine(2 ml; 2 mmol, 1M in Toluol) und 0.98 g (1 mmol) Pd₂(dba)₃ versetzt. Die Reaktionsmischung wird über Nacht unter Schutzatmosphäre zum Sieden erhitzt. Das Gemisch wird abgekühlt und zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 43 g (87% d. Th).

Analog dazu werden die folgenden Verbindungen hergestellt:

| **Bsp.** | **Fluorenon** | **Amin** | **Produkt** |
|---|---|---|---|
| 6b | 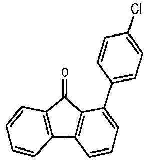 | 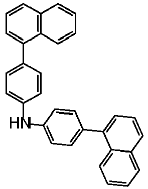 | 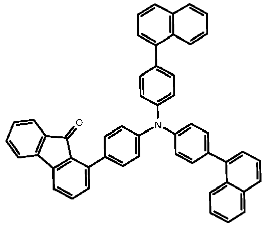 |
| 6c | 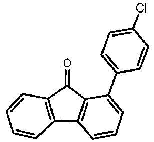 | 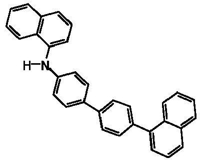 | 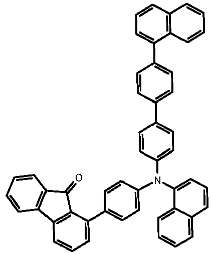 |
| 6d | 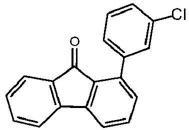 | 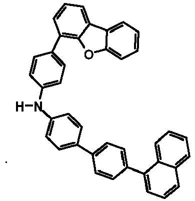 | 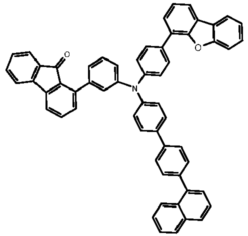 |
| 6f | 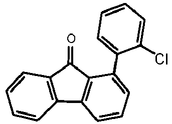 | 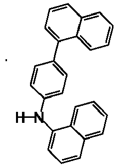 | 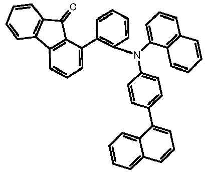 |
| 6g | 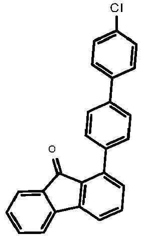 | 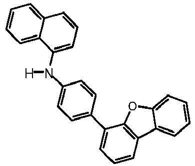 | 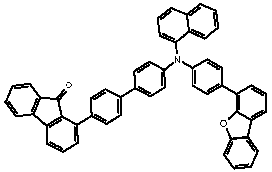 |

### 7) Synthese N-[4-(naphthalen-1-yl)phenyl]-N-(4-{8-oxatricyclo[7.4.0.0^{2,7}]trideca-1(13),2,4,6,9,11- hexaen-6-yl}phenyl)-4-{9,9'-spirobi[fluoren]-8-yl}aniline 7a

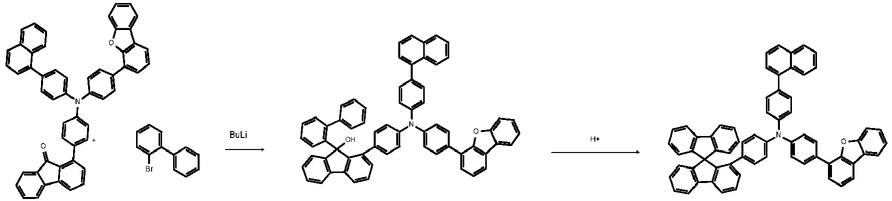

17 g (73 mmol) 2-Bromo-1,1'-biphenyl werden in einem ausgeheizten Kolben in 90 mL getrocknetem THF gelöst. Die Reaktionsmischung wird auf -78°C gekühlt. Bei dieser Temperatur werden 165 mL einer 2,5 M-Lösung n-BuLi in Hexan (66 mmol) langsam zugetropft. Der Ansatz wird 1 Stunde bei -70°C nachgerührt. Anschließend werden 50g 1-(4-{[4-(naphthalen-1-yl)phenyl](4-{8-oxatricyclo [7.4.0.0^{2,7}]trideca-1(13),2,4,6,9,11-hexaen-6-yl}phenyl)amino}phenyl)-9H-fluoren-9-one (70.1 mmol) in 300 ml THF gelöst und bei -70°C zugetropft. Nach beendeter Zugabe lässt man die Reaktionsmischung langsam auf Raumtemperatur erwärmen, stoppt die Reaktion mit NH₄Cl und engt anschließend am Rotationsverdampfer ein. Der Feststoff wird in 500 mL Toluol gelöscht und anschließend werden 720 mg (3.8 mmol) *p*-Toluol-sulfonsäure zugegeben. Der Ansatz wird 6 Stunden unter Reflux erhitzt, danach auf Raumtemperatur abkühlen gelassen und mit Wasser versetzt. Der ausgefallene Feststoff wird abgesaugt und mit Heptan nachgewaschen (40.10 g, 66 % Ausbeute).

Der verbleibende Rückstand wird aus Heptan/Toluol umkristallisiert. Die Substanz wird abschließend im Hochvakuum sublimiert, Reinheit beträgt 99.9%, bestimmt über HPLC. Die Ausbeute beträgt 28 g (48% d. Th).

Analog dazu werden die folgenden Verbindungen hergestellt:

| **Bsp.** | 1- **Amine-fluorenon** | **Br-Biphenyl** | **Produkt** |
|---|---|---|---|
| 7b | 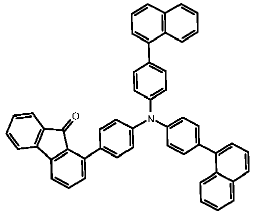 | 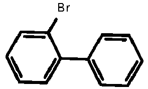 | 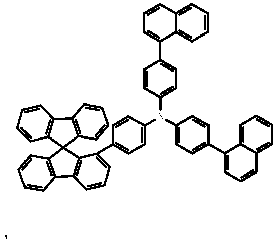 |
| 7c | 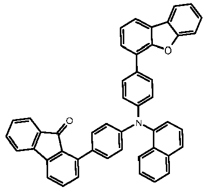 | 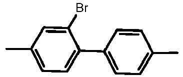 | 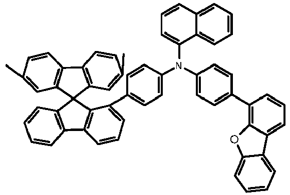 |
| 7d | 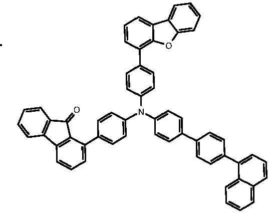 | 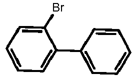 | 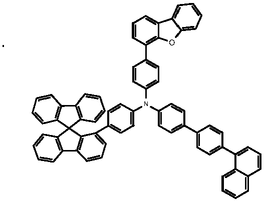 |
| 7e | 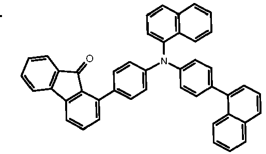 | 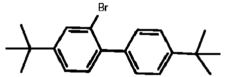 | 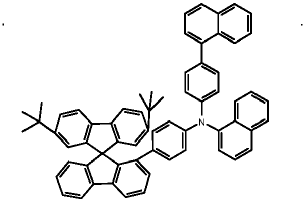 |

### 8) Synthese 8'-(4-chlorophenyl)-9,9'-spirobifluoren 8a

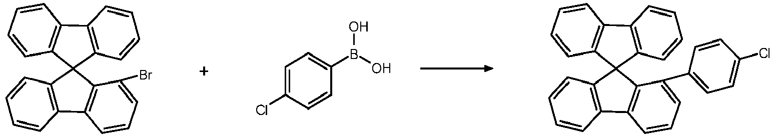

10.7 g (69 mmol) 4-Chlorphenylboronsäure, 27.2 g (69 mmol) 1-Brom-Spiro und 5.4 g (5 mmol) Pd(Ph₃P)₄ werden in 600 mL THF gelöst. Die Lösung wird entgast und mit N₂ gesättigt und 155 ml 2 M Kaliumcarbonat Lösung werden dieser Suspension langsam zugegeben. Die Reaktionsmischung wird über Nacht unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der Rückstand wird durch Kristallisation mit MeOH gereinigt. Ausbeute: 25 g (85 % der Theorie). Reinheit nach HPLC >98%.

Analog dazu werden die folgenden Verbindungen hergestellt:

| **Bsp.** | **Edukt 1** | **Edukt 2** | **Produkt** |
|---|---|---|---|
| 8b | 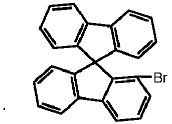 | 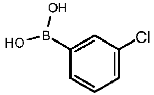 | 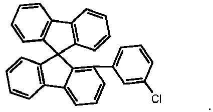 |
| 8c | 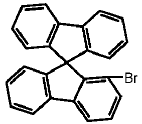 | 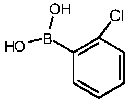 | 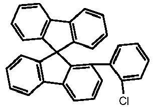 |
| 8d | 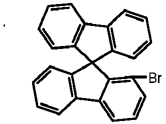 | 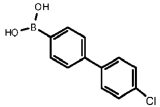 | 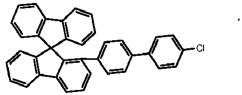 |
| 8e | 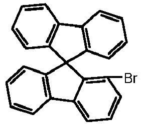 | 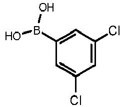 | 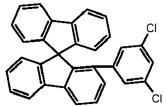 |

### 9) Synthese von N-((1,1'-biphenyl)-4-yl)N-(4-(2',7'-di-tert-butyl-9,9'-spirobi(fluorene)-1-yl)phenyl)-9,9-dimethylfluoren-2-amin 9a

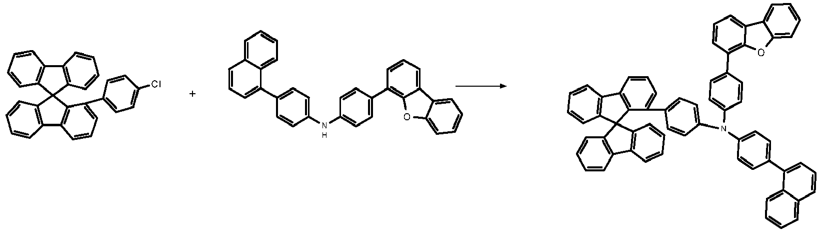

10,1 g (28 mmol) N-[4-(naphthalen-1-yl)phenyl]-4--8-oxatricyclo[7.4.0.02,7]trideca-1(9),2(7),3,5,10,12-hexaen-6-yl-aniline und 14,5 g (27 mol) des 8'-(4-chlorphenyl)-9,9'--spirobifluoren werden in 225 ml Toluol gelöst. Die Lösung wird entgast und mit N₂ gesättigt. Danach wird sie mit 2,1 ml (2,1 mmol) Tri-tertbutylphosphin-Lösung (1 M in Toluol) und 0.98 g (1 mmol) Pd₂(dba)₃ versetzt und anschließend werden 5.1 g Natrium-tert-butylat (53 mmol) zugegeben. Die Reaktionsmischung wird über Nacht unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Substanz wird abschließend im Hochvakuum sublimiert, Reinheit beträgt 99.9%, bestimmt über HPLC. Die Ausbeute beträgt 6 g (26% d. Th).

Analog dazu werden die folgenden Verbindungen hergestellt:

| **Bsp.** | **Spirofluoren** | **Amin** | **Produkt** |
|---|---|---|---|
| 9b | 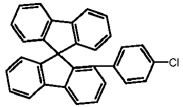 | 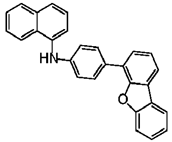 | 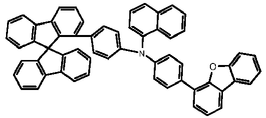 |
| 9c | 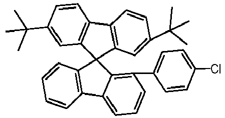 | 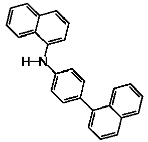 | 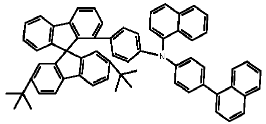 |
| 9d | 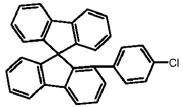 | 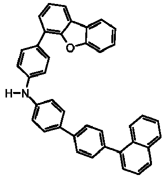 | 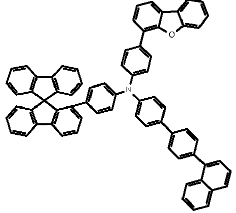 |
| 9e | 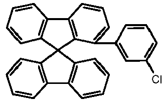 | 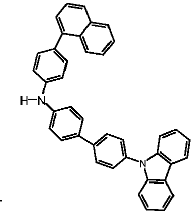 | 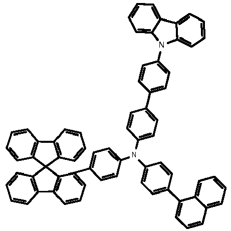 |
| 9f | 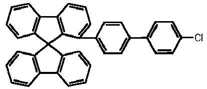 | 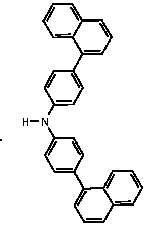 | 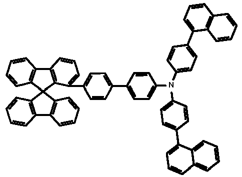 |
| 9g | 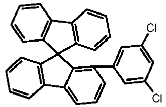 | 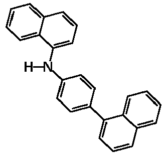 | 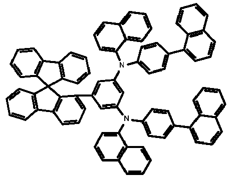 |

### 10) Synthese von N-[4-(naphthalen-1-yl)phenyl]-N-(4-{8-oxatricyclo[7.4.0.0^{2,7}]trideca-1(13),2,4,6,9,11-hexaen-6-yl}phenyl)-4'-{9,9'-spirobi[fluoren]-8-yl}-[1,1'-biphenyl]-4-amin 10a

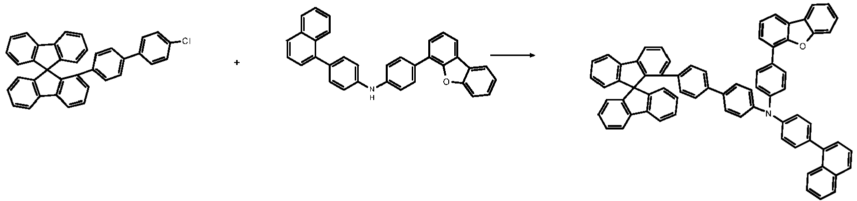

10,1 g (28 mmol) N-[4-(naphthalen-1-yl)phenyl]-4--8-oxatricyclo[7.4.0.02,7]trideca-1(9),2(7),3,5,10,12-hexaen-6-yl-anilin und 14,5 g (29 mmol) des 1-{4'-chloro-[1,1'-biphenyl]-4-yl}-9,9'-spirobi[fluoren] werden in 225 ml Toluol gelöst. Die Lösung wird entgast und mit N₂ gesättigt. Danach wird sie mit 2,1 ml (2,1 mmol) Tri-tertbutylphosphin-Lösung (1 M in Toluol) und 0.98 g (1 mmol) Pd₂(dba)₃ versetzt und anschließend werden 5.1 g Natrium-tert-butylat (53 mmol) zugegeben. Die Reaktionsmischung wird über Nacht unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Substanz wird abschließend im Hochvakuum sublimiert, Reinheit beträgt 99.9%. Die Ausbeute beträgt 8 g (30% d. Th).

Analog dazu werden die folgenden Verbindungen hergestellt:

| **Bsp.** | **Spirofluoren** | **Amin** | **Produkt** |
|---|---|---|---|
| 10b | 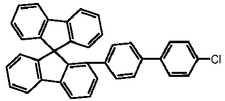 | 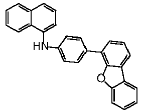 | 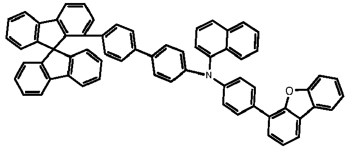 |
| 10c | 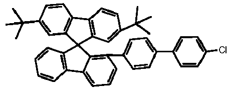 | 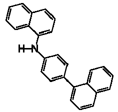 | 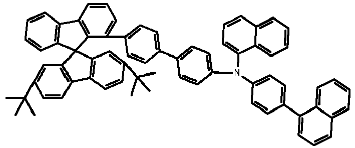 |
| 10d | 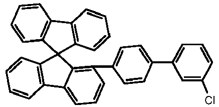 | 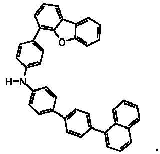 | 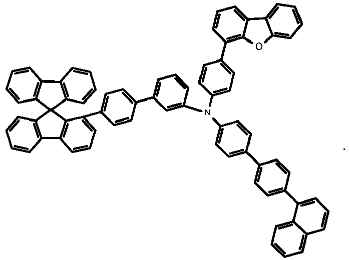 |
| 10e | 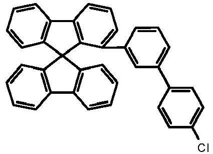 | 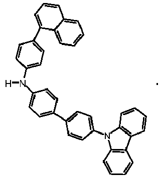 | 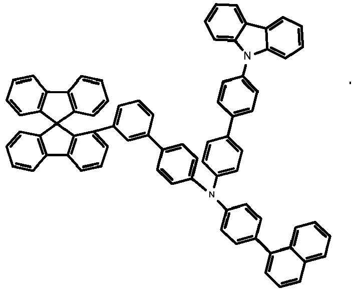 |
| 10f | 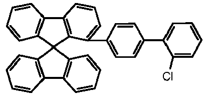 | 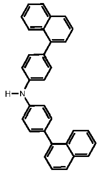 | 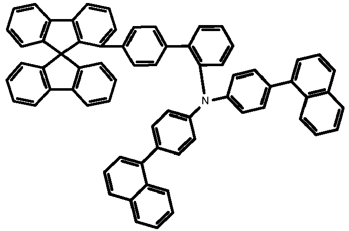 |
| 10g | 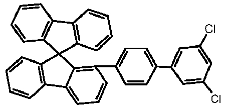 | 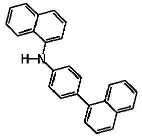 | 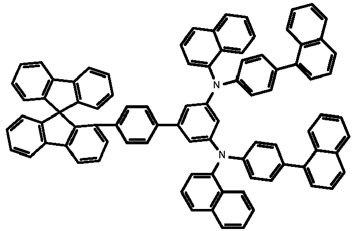 |

### B) Device-Beispiele

### 1) Allgemeines Herstellungsverfahren für die OLEDs und Charakterisierung der OLEDs

Glasplättchen, die mit strukturiertem ITO (Indium Zinn Oxid) der Dicke 50 nm beschichtet sind, bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / Elektroneninjektionsschicht (EIL) und abschließend eine Kathode. Die Kathode wird durch eine 100 nm dicke Aluminiumschicht gebildet. Der genaue Aufbau der OLEDs ist den folgenden Tabellen zu entnehmen. Die zur Herstellung der OLEDs verwendeten Materialien sind in einer folgenden Tabelle gezeigt. Als Material H wird dabei ein Anthracen-Derivat verwendet, und als Material SEB ein Spirobifluoren-Diamin. Als p-Dotand wird NDP-9 der Firma Novaled-AG, Dresden, verwendet.

Alle Materialien werden in einer Vakuumkammer thermisch aufgedampft. Dabei besteht die Emissionsschicht aus mindestens einem Matrixmaterial (Hostmaterial) und einem emittierenden Dotierstoff, der dem Matrixmaterial bzw. den Matrixmaterialien durch Co-Verdampfung in einem bestimmten Volumenanteil beigemischt wird. Eine Angabe wie H:SEB (95%:5%) bedeutet hierbei, dass das Material H in einem Volumenanteil von 95% und SEB in einem Anteil von 5% in der Schicht vorliegt. Analog bestehen auch die Elektronentransportschicht und die Lochinjektionsschicht aus einer Mischung von zwei Materialien. Die Strukturen der Materialien, die in den OLEDs verwendet werden, sind in Tabelle 3 gezeigt.

Die OLEDs werden standardmäßig charakterisiert. Hierfür werden die Elektrolumineszenzspektren, die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Angabe EQE @ 10mA/cm² bezeichnet die externe Quanteneffizienz, die bei 10mA/cm² erreicht wird. Die Angabe U @ 10 mA/cm² bezeichnet die Betriebsspannung bei 10 mA/cm². Als Lebensdauer LT wird die Zeit definiert, nach der die Leuchtdichte bei Betrieb mit konstanter Stromdichte von der Startleuchtdichte auf einen gewissen Anteil absinkt. Eine Angabe LT90 bedeutet dabei, dass die angegebene Lebensdauer der Zeit entspricht, nach der die Leuchtdichte auf 90% ihres Anfangswerts abgesunken ist. Die Angabe @80 mA/cm² bedeutet dabei, dass die betreffende Lebensdauer bei 80 mA/cm² gemessen wird.

### 2) Erfindungsgemäße OLEDs enthaltend eine Verbindung der Formel (I) in der EBL von blau fluoreszierenden OLEDs: Vergleich der erfindungsgemäßen Substitutionsposition 1 und 2 mit den Substitutionspositionen 3 und 4

Vorrichtungen wie in der folgenden Tabelle gezeigt werden hergestellt:

| ***Bsp.*** | ***HIL*** | ***HTL*** | ***EBL*** | ***EML*** | ***HBL*** | ***ETL*** | ***EIL*** |
|---|---|---|---|---|---|---|---|
| | Dicke / nm | Dicke / nm | Dicke / nm | Dicke / nm | Dicke / nm | Dicke / nm | Dicke / nm |
| E1 | HTM: p-dopant (5%) 10 nm | HTM 180 nm | HTM-1 10 nm | H:SEB(3%) 20 nm | HBM 10nm | ETM:LiQ(50%) 30 nm | LiQ 1 nm |
| E2 | HTM: p-dopant (5%) 10 nm | HTM 180 nm | HTM-2 10 nm | H:SEB(3%) 20 nm | HBM 10nm | ETM:LiQ(50%) 30 nm | LiQ 1 nm |
| E-C1 | HTM: p-dopant (5%) 10 nm | HTM 180 nm | HTM-C1 10 nm | H:SEB(3%) 20 nm | HBM 10nm | ETM:LiQ(50%) 30 nm | LiQ 1 nm |
| E-C2 | HTM: p-dopant (5%) 10 nm | HTM 180 nm | HTM-C2 10 nm | H:SEB(3%) 20 nm | HBM 10nm | ETM:LiQ(50%) 30 nm | LiQ 1 nm |

Dabei werden Verbindungen gemäß der vorliegenden Anmeldung in den OLEDs E1 und E2 eingesetzt. OLEDs E-C1 und E-C2 dienen als Referenz und enthalten Verbindungen, die in den Positionen 3 bzw. 4 am Spiro mit der Aminogruppe substituiert sind.

Die erfindungsgemäßen OLEDs (Substitutionsposition am Spirobifluoren 1 bzw. 2) zeigen laut Messung eine verbesserte Lebensdauer gegenüber den OLEDs E-C1 und E-C2 mit Substitutionsposition 3 bzw. 4 der Aminogruppe am Spirobifluoren:

| | LT90 @ 60 mA/cm² (h) |
|---|---|
| E1 | 210 |
| E2 | 240 |
| E-C1 | 180 |
| E-C2 | 180 |

### 3) Verwendung der erfindungsgemäßen Verbindungen in der EBL von grün phosphoreszierenden OLEDs

Die folgenden OLEDs werden hergestellt:

| ***Bsp.*** | ***HIL*** | ***HTL*** | ***EBL*** | ***EML*** | ***HBL*** | ***ETL*** | ***EIL*** |
|---|---|---|---|---|---|---|---|
| | Dicke / nm | Dicke / nm | Dicke / nm | Dicke / nm | Dicke / nm | Dicke / nm | Dicke / nm |
| E-3 | HTM: p-Dotand (5%) 20 nm | HTM 90 nm | HTM-1 20 nm | TMM-1 (32%) TMM-2(60%) TEG (8%) 30 nm | HBM 10nm | ETM:LiQ(50%) 30 nm | LiQ 1 nm |
| E-4 | HTM: p-Dotand (5%) 20 nm | HTM 90 nm | HTM-2 20 nm | TMM-1 (32%) TMM-2(60%) TEG (8%) 30 nm | HBM 10nm | ETM:LiQ(50%) 30 nm | LiQ 1 nm |
| E-5 | HTM: p-Dotand (5%) 20 nm | HTM 90 nm | HTM-3 20 nm | TMM-1 (32%) TMM-2(60%) TEG (8%) 30 nm | HBM 10nm | ETM:LiQ(50%) 30 nm | LiQ 1 nm |

Dabei werden mit den erfindungsgemäßen Verbindungen HTM-1 bis HTM-3 gute Leistungsdaten erhalten bzgl. Lebensdauer (90 - 120 h LT90@80mA), Betriebsspannung (3.5V-3.9V U@10mA) und Effizienz (22.2-24.4% EQE@10mA).

### 4) Verwendung der erfindungsgemäßen Verbindungen in der HIL und HTL von blau fluoreszierenden OLEDs

Die folgenden OLEDs werden hergestellt:

| ***Bsp.*** | ***HIL*** | ***HTL*** | ***EBL*** | ***EML*** | ***HBL*** | ***ETL*** | ***EIL*** |
|---|---|---|---|---|---|---|---|
| | Dicke / nm | Dicke / nm | Dicke / nm | Dicke / nm | Dicke / nm | Dicke / nm | Dicke / nm |
| E-6 | HTM-1: p-Dotand (5%) 10 nm | HTM-1 180 nm | EBM 10 nm | H:SEB(3%) 20 nm | HBM 10nm | ETM:LiQ(50%) 30 nm | LiQ 1 nm |
| E-7 | HTM-2: p-Dotand (5%) 10 nm | HTM-2 180 nm | EBM 10 nm | H:SEB(3%) 20 nm | HBM 10nm | ETM:LiQ(50%) 30 nm | LiQ 1 nm |

Dabei werden mit den erfindungsgemäßen Verbindungen gute Leistungsdaten erhalten insbesondere bzgl. Effizienz (8.0 - 9.0 % EQE@10mA), bei einer Betriebsspannung von 4.0 - 8.0 V U@10mA.

### 5) Verwendung der erfindungsgemäßen Verbindungen in der EBL von blau fluoreszierenden OLEDs

Die folgende OLED wird hergestellt:

| ***Bsp.*** | ***HIL*** | ***HTL1*** | ***HTL2*** | ***EBL*** | ***EML*** | ***ETL*** | ***EIL*** |
|---|---|---|---|---|---|---|---|
| E-8 | HTM-A: p-Dotand (5%) 20 nm | HTM-A 160 nm | HTM-4: p-Dotand (5%) 20nm | HTM-4 10nm | H-1:SEB-1(5%) 20 nm | ETM-1 : LiQ(50%) 30 nm | LiQ 1 nm |

Dabei werden mit der erfindungsgemäßen Verbindung HTM-4 gute Ergebnisse erhalten. Die Lebensdauer LT90@60mA liegt bei 210 h, die Effizienz EQE@10mA bei ca. 8%, und die Spannung U@10mA bei ca. 4 V.

| **Tabelle 3: Strukturen der Verbindungen** | |
|---|---|
| 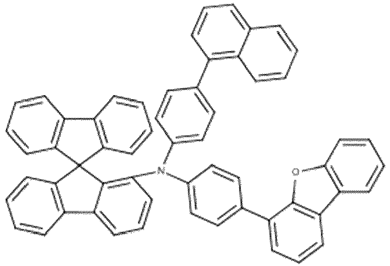 | 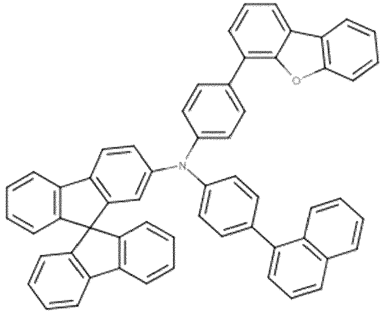 |
| HTM-1 | HTM-2 |
| 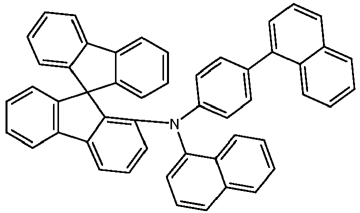 | 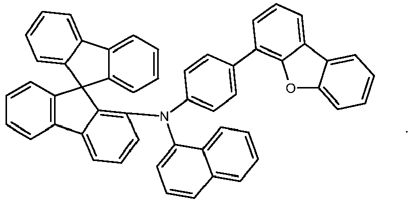 |
| HTM-3 | HTM-4 |
| 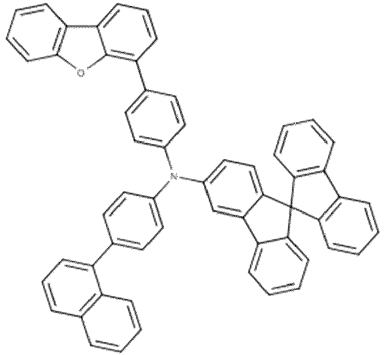 | 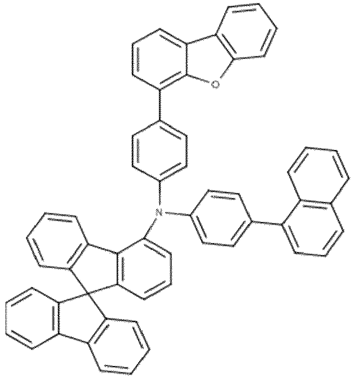 |
| HTM-C1 | HTM-C2 |
| 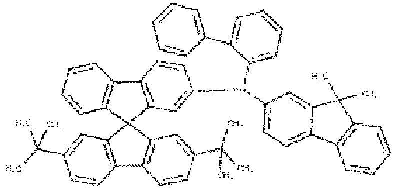 | 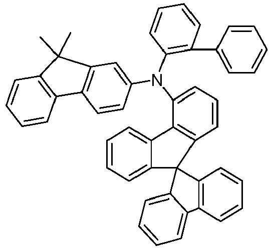 |
| HTM | EBM |
| 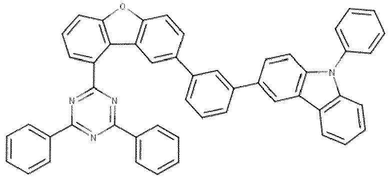 | 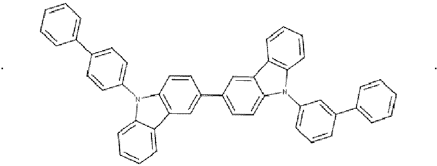 |
| TMM-1 | TMM-2 |
| 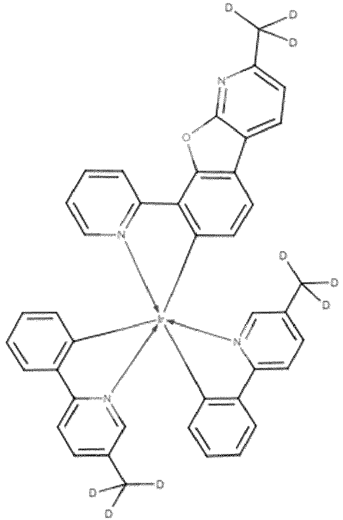 | 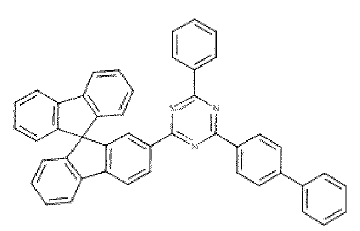 |
| TEG | ETM |
| 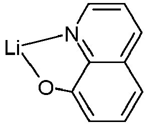 | 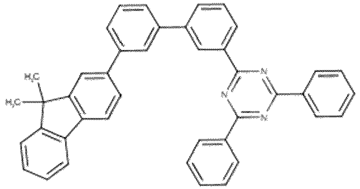 |
| LiQ | HBM |
| 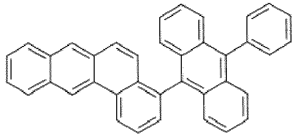 | 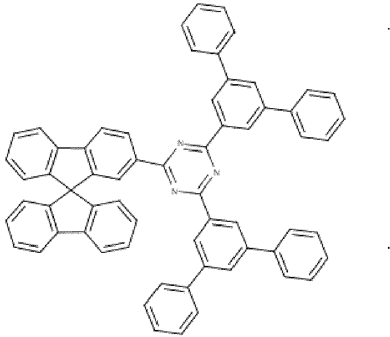 |
| H-1 | ETM-1 |
| 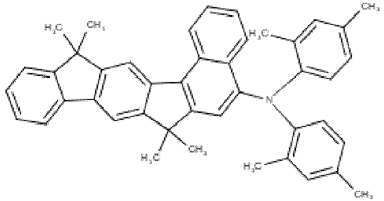 | 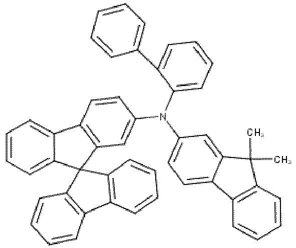 |
| SEB-1 | HTM-A |

## Patentansprüche

1. Verbindung einer Formel (I) 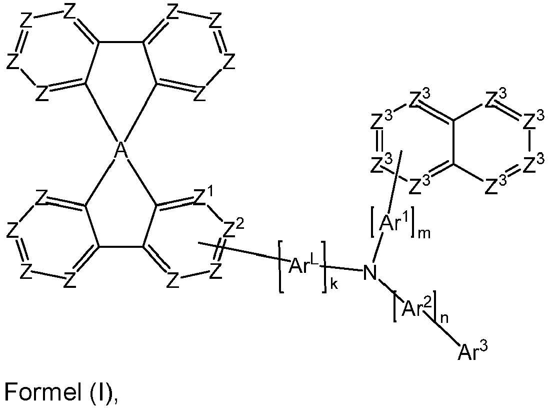
wobei die mit variabler Bindungsposition am Spirobifluoren gezeichnete Aminogruppe in Formel (I) entweder an Z¹ oder an Z² gebunden ist; und
wobei für die auftretenden Gruppen und Indices gilt:
A ist gleich C oder Si;
Ar^{L} ist bei jedem Auftreten gleich oder verschieden gewählt aus aromatischen Ringsystemen mit 6 bis 40 aromatischen Ringatomen, die jeweils mit Resten R² substituiert sind, und heteroaromatischen Ringsystemen mit 5 bis 40 aromatischen Ringatomen, die jeweils mit Resten R² substituiert sind;
Ar¹ ist bei jedem Auftreten gleich oder verschieden gewählt aus aromatischen Ringsystemen mit 6 bis 24 aromatischen Ringatomen, die mit Resten R³ substituiert sind, und heteroaromatischen Ringsystemen mit 5 bis 24 aromatischen Ringatomen, die mit Resten R³ substituiert sind;
Ar² ist bei jedem Auftreten gleich oder verschieden gewählt aus Phenyl, das mit Resten R⁵ substituiert ist, Naphthyl, das mit Resten R⁵ substituiert ist, und heteroaromatischen Ringsystemen mit 6 bis 10 aromatischen Ringatomen, die jeweils mit Resten R⁵ substituiert sind;
Ar³ ist gewählt aus Gruppen der Formeln (Ar³-1) bis (Ar³-7), die über die mit * markierte Bindung an den Rest der Formel (I) binden 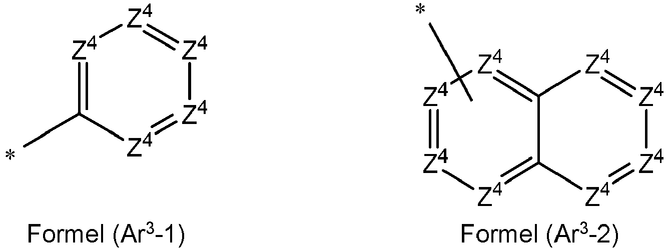 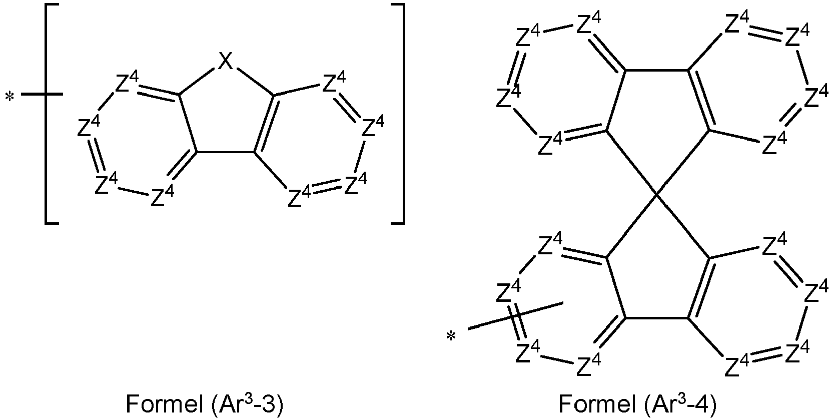 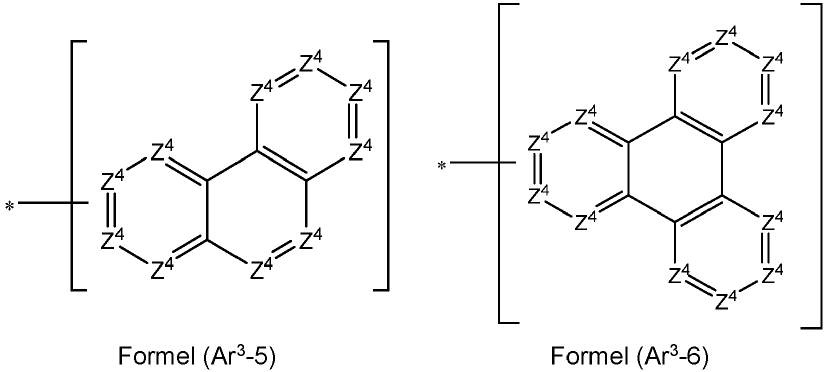 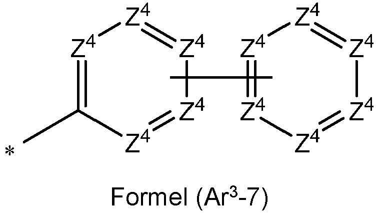
wobei Z⁴ bei jedem Auftreten gleich oder verschieden gewählt ist aus CR⁶ und N, und wobei in Formeln (Ar³-2) bis (Ar³-7) diejenige Gruppe Z⁴ gleich C ist, die an eine frei gezeichnete Bindung bindet; wobei in Formel (Ar³-3) auch X gleich C(R⁶)₂ an den Rest der Formel (I) binden kann, wobei dann eine Gruppe R⁶ in dieser Gruppe X durch die Bindung an den Rest der Formel (I) ersetzt ist;
und wobei X gewählt ist aus O, S, NR⁶ und C(R⁶)₂; und wobei in Formel (Ar³-3) eine oder mehrere Einheiten Z⁴=Z⁴ durch eine Einheit gewählt aus den Einheiten 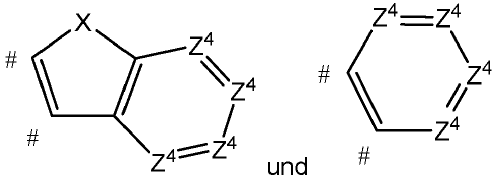
ersetzt sein können, wobei die mit # markierten Atome jeweils die Atome Z⁴ ersetzen;
und wobei in Formel (Ar³-3) eine oder mehrere Einheiten Z⁴-Z⁴ durch eine Einheit gewählt aus den Einheiten 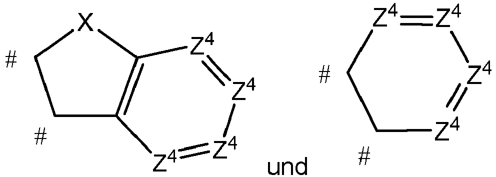
ersetzt sein können, wobei die mit # markierten Atome jeweils die Atome Z⁴ ersetzen;
Z ist bei jedem Auftreten gleich oder verschieden gewählt aus CR¹ und N;
Z¹ und Z² sind jeweils C, wenn die mit variabler Bindungsposition gezeichnete Aminogruppe daran gebunden ist, und ansonsten gewählt aus CR¹ und N;
Z³ ist bei jedem Auftreten gleich oder verschieden gewählt aus CR⁴ und N;
R¹ ist bei jedem Auftreten gleich oder verschieden gewählt aus H, D, F, Cl, Br, I, C(=O)R⁷, CN, Si(R⁷)₃, N(R⁷)₂, -(Ar^{L})ₖ-N(Ar⁴)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen;
wobei zwei oder mehr Reste R¹, R², R³, R⁴, R⁵ bzw. R⁶ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁷ substituiert sind; und wobei eine oder mehrere CH₂-Gruppen in den genannten Alkyl-, Alkoxy-, Alkenyl- und Alkinylgruppen durch - R⁷C=CR⁷-, -C≡C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
R², R³, R⁴, R⁵ und R⁶ sind jeweils bei jedem Auftreten gleich oder verschieden gewählt aus H, D, F, Cl, Br, I, C(=O)R⁷, CN, Si(R⁷)₃, N(R⁷)₂, P(=O)(R⁷)₂, OR⁷, S(=O)R⁷, S(=O)₂R⁷, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R², R³, R⁴, R⁵ bzw. R⁶ miteinander verknüpft sein können und einen Ring bilden können;
wobei die genannten Alkyl-, Alkoxy-, Alkenyl- und Alkinylgruppen und die genannten aromatischen Ringsysteme und heteroaromatischen Ringsysteme jeweils mit Resten R⁷ substituiert sind; und wobei eine oder mehrere CH₂-Gruppen in den genannten Alkyl-, Alkoxy-, Alkenyl- und Alkinylgruppen durch -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO oder SO₂ ersetzt sein können;
Ar⁴ ist bei jedem Auftreten gleich oder verschieden gewählt aus aromatischen Ringsystemen mit 6 bis 40 aromatischen Ringatomen, die mit einem oder mehreren Resten R⁷ substituiert sind, und heteroaromatischen Ringsystemen mit 5 bis 40 aromatischen Ringatomen, die mit einem oder mehreren Resten R⁷ substituiert sind;
R⁷ ist bei jedem Auftreten gleich oder verschieden gewählt aus H, D, F, Cl, Br, I, C(=O)R⁸, CN, Si(R⁸)₃, N(R⁸)₂, P(=O)(R⁸)₂, OR⁸, S(=O)R⁸, S(=O)₂R⁸, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹, R², R³, R⁴, R⁵ bzw. R⁶ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R⁸ substituiert sind; und wobei eine oder mehrere CH₂-Gruppen in den genannten Alkyl-, Alkoxy-, Alkenyl- und Alkinylgruppen durch -R⁸C=CR⁸-, -C=C-, Si(R⁸)₂, C=O, C=NR⁸, -C(=O)O-, -C(=O)NR⁸-, NR⁸, P(=O)(R⁸), -O-, -S-, SO oder SO₂ ersetzt sein können
R⁸ ist bei jedem Auftreten gleich oder verschieden gewählt aus H, D, F, Cl, Br, I, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁸ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit einem oder mehreren Resten gewählt aus F und CN substituiert sein können;
k ist gleich 0, 1, 2, 3 oder 4;
m ist gleich 0, 1, 2, 3 oder 4;
n ist gleich 1, 2, 3 oder 4.

2. Verbindung nach Anspruch 1, **dadurch gekennzeichnet, dass** Z¹ und Z² jeweils C sind, wenn die mit variabler Bindungsposition gezeichnete Aminogruppe daran gebunden ist, und ansonsten gleich CR¹ sind, Z gleich CR¹ ist, Z³ gleich CR⁴ ist, und Z⁴ gleich CR⁶ ist.

3. Verbindung nach Anspruch 1 oder 2, **dadurch gekennzeichnet, dass** R¹ bei jedem Auftreten gleich oder verschieden gewählt ist aus H, -[Ar^{L}]ₖ-N(Ar⁴)₂, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit Resten R⁷ substituiert sind.

4. Verbindung nach einem oder mehreren der Ansprüche 1 bis 3, **dadurch gekennzeichnet, dass** R², R³, R⁴, R⁵ und R⁶ bei jedem Auftreten gleich oder verschieden gewählt sind aus H, D, F, CN, Si(R⁷)₃, N(R⁷)₂, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit Resten R⁷ substituiert sind; und wobei in den genannten Alkyl- oder Alkoxygruppen eine oder mehrere CH₂-Gruppen durch -C=C-, -R⁷C=CR⁷-, Si(R⁷)₂, C=O, C=NR⁷, -NR⁷-, -O-, -S-, -C(=O)O- oder -C(=O)NR⁷- ersetzt sein können.

5. Verbindung nach einem oder mehreren der Ansprüche 1 bis 4, **dadurch gekennzeichnet, dass** sie mindestens eine Gruppe R⁴ enthält, die gewählt ist aus aromatischen Ringsystemen mit 6 bis 40 aromatischen Ringatomen, und heteroaromatischen Ringsystemen mit 5 bis 40 aromatischen Ringatome, die jeweils mit Resten R⁷ substituiert sind.

6. Verbindung nach einem oder mehreren der Ansprüche 1 bis 5, **dadurch gekennzeichnet, dass** Index k gleich 0 ist.

7. Verbindung nach einem oder mehreren der Ansprüche 1 bis 6, **dadurch gekennzeichnet, dass** Ar¹ bei jedem Auftreten gleich oder verschieden gewählt ist aus Phenyl, Naphthyl, Fluorenyl, und Biphenyl, die jeweils mit Resten R³ substituiert sind.

8. Verbindung nach einem oder mehreren der Ansprüche 1 bis 7, **dadurch gekennzeichnet, dass** sie einer Formel gewählt aus den folgenden Formeln entspricht:
| |
|---|
| 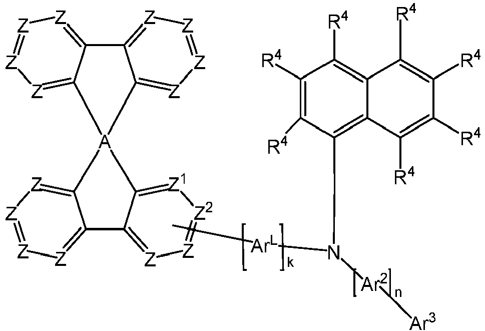 |
| Formel (I-sub-1) |
| 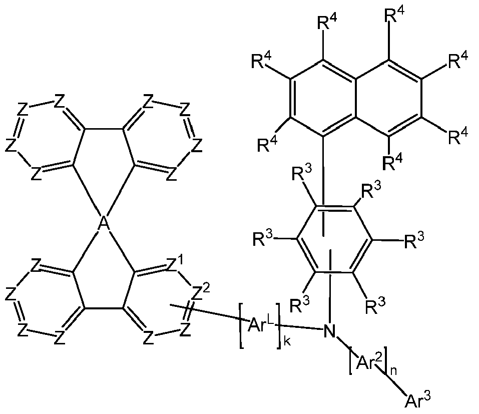 |
| Formel (I-sub-2) |
| 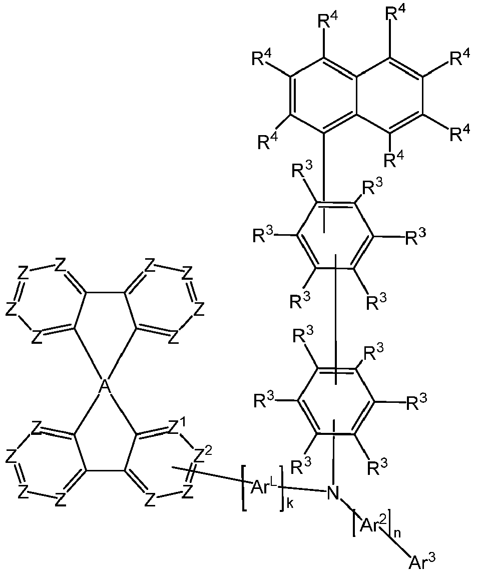 |
| Formel (I-sub-3) |
wobei die auftretenden Gruppen und Indices definiert sind wie in einem oder mehreren der Ansprüche 1 bis 7.

9. Verbindung nach einem oder mehreren der Ansprüche 1 bis 8, **dadurch gekennzeichnet, dass** sie einer der folgenden Formeln entspricht:
| |
|---|
| 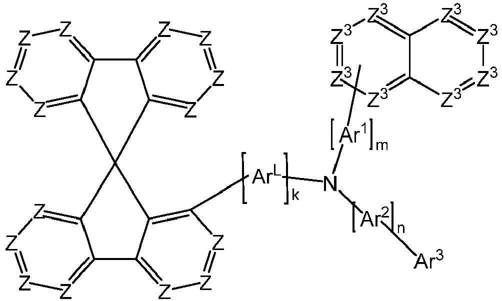 |
| (I-1) |
| 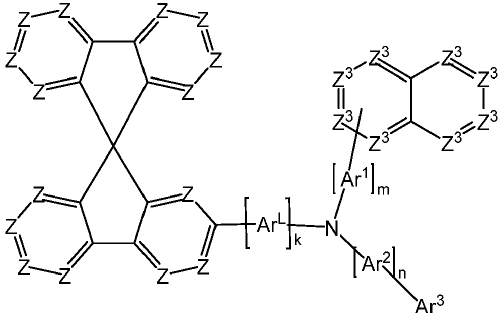 |
| (I-2) |
| 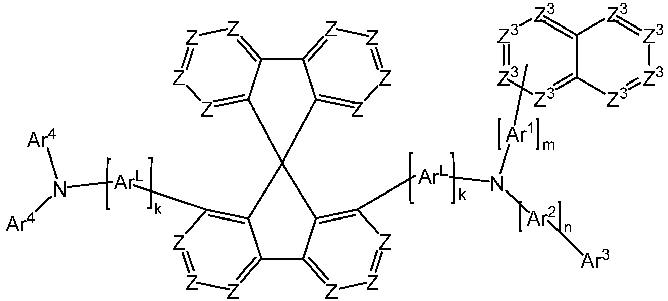 |
| (I-3) |
| 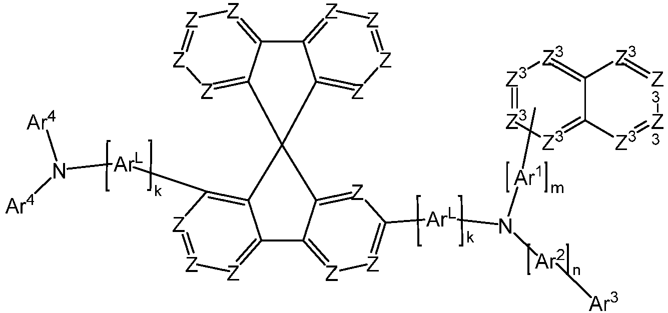 |
| (I-4) |
| 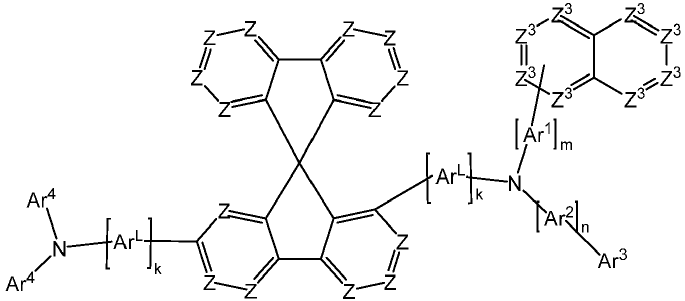 |
| (I-5) |
| 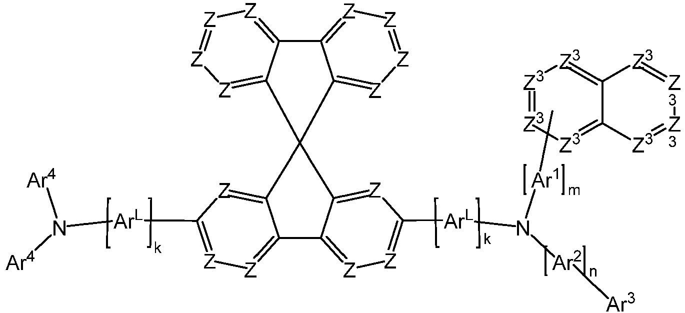 |
| (I-6) |
| 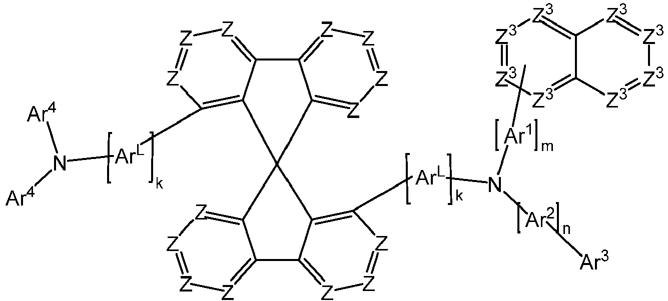 |
| (I-7) |
| 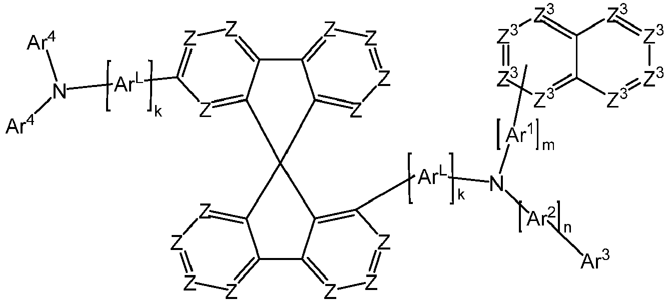 |
| (I-8) |
| 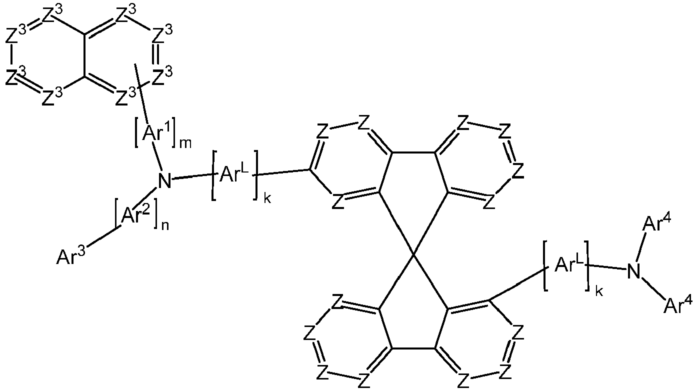 |
| (I-9) |
| 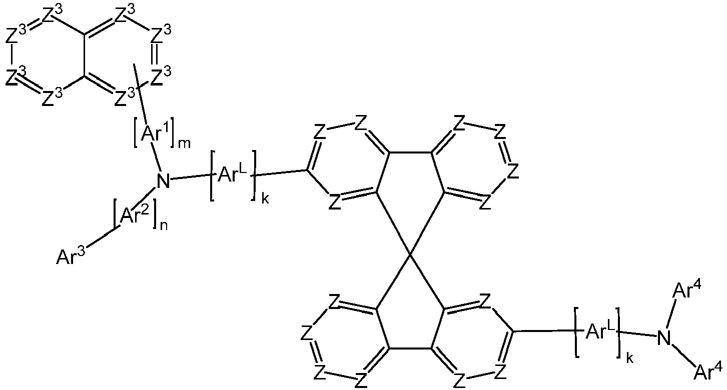 |
| (I-10) |
wobei die auftretenden Indices definiert sind wie in einem oder mehreren der Ansprüche 1 bis 8.

10. Verbindung nach einem oder mehreren der Ansprüche 1 bis 9, **dadurch gekennzeichnet, dass** Ar² bei jedem Auftreten gleich oder verschieden gewählt ist aus Phenyl und Naphthyl, die jeweils mit Resten R⁵ substituiert sind.

11. Verbindung nach einem oder mehreren der Ansprüche 1 bis 10, **dadurch gekennzeichnet, dass** Index n gleich 1 oder 2 ist.

12. Verbindung nach einem oder mehreren der Ansprüche 1 bis 11, **dadurch gekennzeichnet, dass** Ar³ aus Gruppen der Formeln (Ar³-2) und (Ar³-3) gewählt ist:

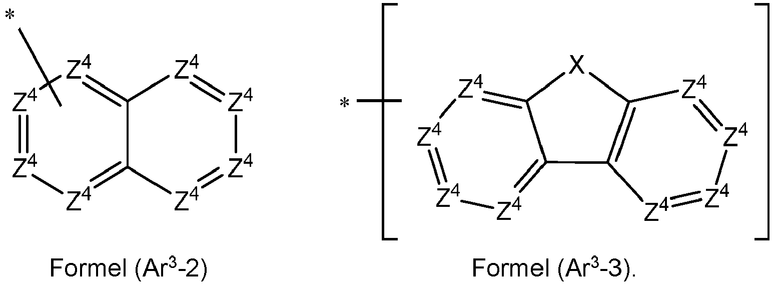

13. Verbindung nach einem oder mehreren der Ansprüche 1 bis 12, **dadurch gekennzeichnet, dass** A gleich C ist.

14. Verfahren zur Herstellung einer Verbindung nach einem oder mehreren der Ansprüche 1 bis 13, **dadurch gekennzeichnet, dass** mittels einer metallorganischen Kupplungsreaktion eine Aminogruppe eingeführt wird.

15. Oligomer, Polymer oder Dendrimer, enthaltend eine oder mehrere Verbindungen nach einem oder mehreren der Ansprüche 1 bis 13, wobei die Bindung(en) zum Polymer, Oligomer oder Dendrimer an beliebigen, in Formel (I) mit R¹, R², R³, R⁴, R⁵ oder R⁶ substituierten Positionen lokalisiert sein können.

16. Formulierung, enthaltend mindestens eine Verbindung nach einem oder mehreren der Ansprüche 1 bis 13 oder mindestens ein Polymer, Oligomer oder Dendrimer nach Anspruch 15, sowie mindestens ein Lösungsmittel.

17. Elektronische Vorrichtung, enthaltend mindestens eine Verbindung nach einem oder mehreren der Ansprüche 1 bis 13, oder mindestens ein Polymer, Oligomer oder Dendrimer nach Anspruch 15.

18. Elektronische Vorrichtung nach Anspruch 17, **dadurch gekennzeichnet, dass** sie eine organische Elektrolumineszenzvorrichtung ist und Anode, Kathode und mindestens eine emittierende Schicht enthält, und dass die Verbindung in einer lochtransportierenden Schicht oder in einer emittierenden Schicht der Vorrichtung enthalten ist.

19. Verwendung einer Verbindung nach einem oder mehreren der Ansprüche 1 bis 13 in einer elektronischen Vorrichtung.

## Claims

1. Compound of a formula (I) 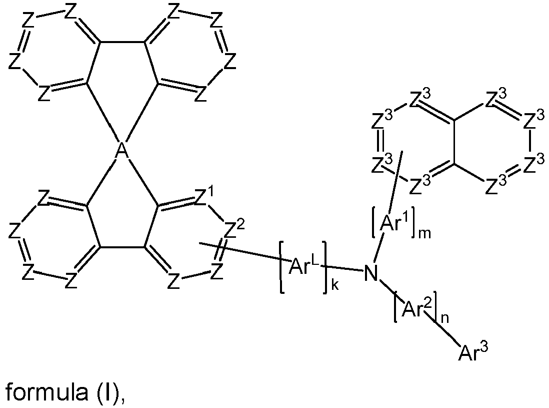
where the amino group drawn with variable bonding position to the spiro-bifluorene in formula (I) is bonded either to Z¹ or to Z²; and
where the following applies to the groups and indices occurring:
A is equal to C or Si;
Ar^{L} is selected on each occurrence, identically or differently, from aromatic ring systems having 6 to 40 aromatic ring atoms, which are in each case substituted by radicals R², and heteroaromatic ring systems having 5 to 40 aromatic ring atoms, which are in each case substituted by radicals R²;
Ar¹ is selected on each occurrence, identically or differently, from aromatic ring systems having 6 to 24 aromatic ring atoms, which are substituted by radicals R³, and heteroaromatic ring systems having 5 to 24 aromatic ring atoms, which are substituted by radicals R³;
Ar² is selected on each occurrence, identically or differently, from phenyl, which is substituted by radicals R⁵, naphthyl, which is substituted by radicals R⁵, and heteroaromatic ring systems having 6 to 10 aromatic ring atoms, which are in each case substituted by radicals R⁵;
Ar³ is selected from groups of the formulae (Ar³-1) to (Ar³-7), which are bonded to the remainder of the formula (I) via the bond marked with * 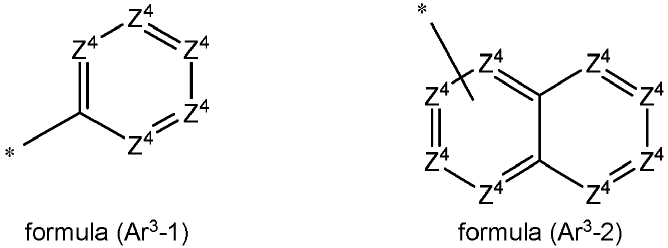 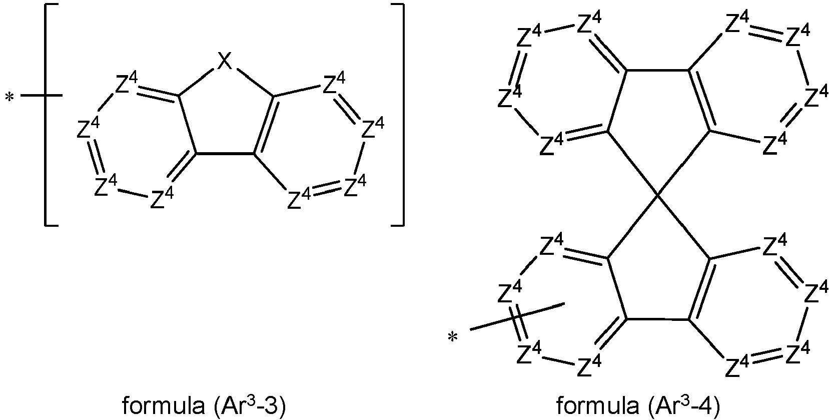 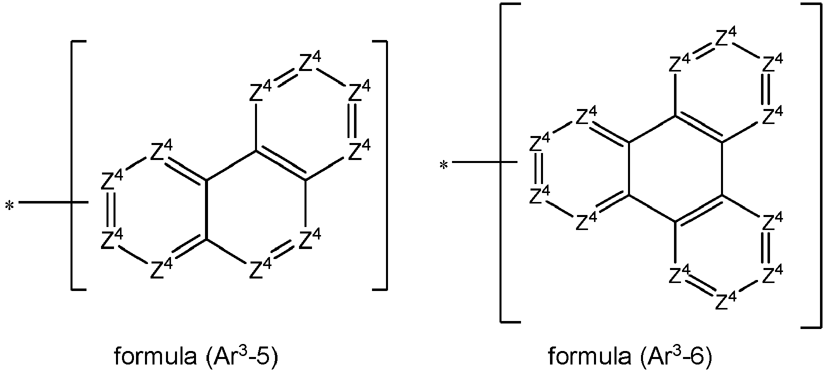 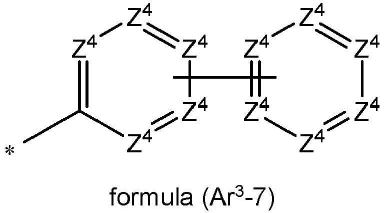
where Z⁴ is selected on each occurrence, identically or differently, from CR⁶ and N, and where in formulae (Ar³-2) to (Ar³-7) the group Z⁴ that is bonded via a freely drawn bond is C; where in formula (Ar³-3) X equals C(R⁶)₂ may also be bonded to the remainder of the formula (I), in which case a group R⁶ in this group X is replaced by the bond to the remainder of the formula (I);
and where X is selected from O, S, NR⁶ and C(R⁶)₂; and where in formula (Ar³-3) one or more units Z⁴=Z⁴ may be replaced by a unit selected from the units 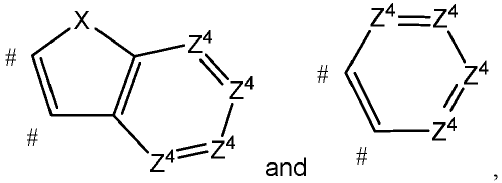
where the atoms marked with # in each case replace the atoms Z⁴; and where in formula (Ar³-3) one or more units Z⁴-Z⁴ may be replaced by a unit selected from the units 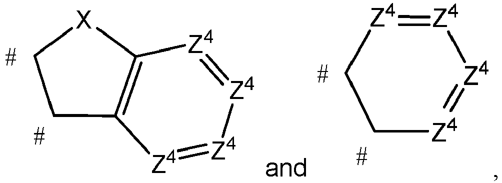
where the atoms marked with # in each case replace the atoms Z⁴;
Z is selected on each occurrence, identically or differently, from CR¹ and N;
Z¹ and Z² are in each case C if the amino group drawn with variable bonding position is bonded thereto and are otherwise selected from CR¹ and N;
Z³ is selected on each occurrence, identically or differently, from CR⁴ and N;
R¹ is selected on each occurrence, identically or differently, from H, D, F, Cl, Br, I, C(=O)R⁷, CN, Si(R⁷)₃, N(R⁷)₂, -(Ar^{L})ₖ-N(Ar⁴)₂, P(=O)(R⁷)₂, OR⁷, S(=O)R⁷, S(=O)₂R⁷, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹, R², R³, R⁴, R⁵ or R⁶ may be linked to one another and may form a ring; where the said alkyl, alkoxy, alkenyl and alkynyl groups and the said aromatic ring systems and heteroaromatic ring systems are in each case substituted by radicals R⁷; and where one or more CH₂ groups in the said alkyl, alkoxy, alkenyl and alkynyl groups may be replaced by -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO or SO₂;
R², R³, R⁴, R⁵ and R⁶ are in each case selected, on each occurrence identically or differently, from H, D, F, Cl, Br, I, C(=O)R⁷, CN, Si(R⁷)₃, N(R⁷)₂, P(=O)(R⁷)₂, OR⁷, S(=O)R⁷, S(=O)₂R⁷, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R², R³, R⁴, R⁵ or R⁶ may be linked to one another and may form a ring; where the said alkyl, alkoxy, alkenyl and alkynyl groups and the said aromatic ring systems and heteroaromatic ring systems are in each case substituted by radicals R⁷; and where one or more CH₂ groups in the said alkyl, alkoxy, alkenyl and alkynyl groups may be replaced by -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO or SO₂; Ar⁴ is selected on each occurrence, identically or differently, from aromatic ring systems having 6 to 40 aromatic ring atoms, which are substituted by one or more radicals R⁷, and heteroaromatic ring systems having 5 to 40 aromatic ring atoms, which are substituted by one or more radicals R⁷;
R⁷ is selected on each occurrence, identically or differently, from H, D, F, Cl, Br, I, C(=O)R⁸, CN, Si(R⁸)₃, N(R⁸)₂, P(=O)(R⁸)₂, OR⁸, S(=O)R⁸, S(=O)₂R⁸, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹, R², R³, R⁴, R⁵ or R⁶ may be linked to one another and may form a ring; where the said alkyl, alkoxy, alkenyl and alkynyl groups and the said aromatic ring systems and heteroaromatic ring systems are in each case substituted by radicals R⁸; and where one or more CH₂ groups in the said alkyl, alkoxy, alkenyl and alkynyl groups may be replaced by -R⁸C=CR⁸-, -C=C-, Si(R⁸)₂, C=O, C=NR⁸, -C(=O)O-, -C(=O)NR⁸-, NR⁸, P(=O)(R⁸), -O-, -S-, SO or SO₂;
R⁸ is selected on each occurrence, identically or differently, from H, D, F, Cl, Br, I, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁸ may be linked to one another and may form a ring; and where the said alkyl, alkoxy, alkenyl and alkynyl groups, aromatic ring systems and heteroaromatic ring systems may be substituted by one or more radicals selected from F and CN;
k is equal to 0, 1, 2, 3 or 4;
m is equal to 0, 1, 2, 3 or 4;
n is equal to 1, 2, 3 or 4.

2. Compound according to Claim 1, **characterised in that** Z¹ and Z² are in each case C if the amino group drawn with variable bonding position is bonded thereto, and otherwise are equal to CR¹, Z is equal to CR¹, Z³ is equal to CR⁴ and Z⁴ is equal to CR⁶.

3. Compound according to Claim 1 or 2, **characterised in that** R¹ is selected on each occurrence, identically or differently, from H, -[Ar^{L}]ₖ-N(Ar⁴)₂, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where the said alkyl groups, the said aromatic ring systems and the said heteroaromatic ring systems are in each case substituted by radicals R⁷.

4. Compound according to one or more of Claims 1 to 3, **characterised in that** R², R³, R⁴, R⁵ and R⁶ are selected on each occurrence, identically or differently, from H, D, F, CN, Si(R⁷)₃, N(R⁷)₂, straight-chain alkyl or alkoxy groups having 1 to 20 C atoms, branched or cyclic alkyl or alkoxy groups having 3 to 20 C atoms, aromatic ring systems having 6 to 40 aromatic ring atoms and heteroaromatic ring systems having 5 to 40 aromatic ring atoms; where the said alkyl and alkoxy groups, the said aromatic ring systems and the said heteroaromatic ring systems are in each case substituted by radicals R⁷; and where one or more CH₂ groups in the said alkyl or alkoxy groups may be replaced by -C=C-, -R⁷C=CR⁷-, Si(R⁷)₂, C=O, C=NR⁷, -NR⁷-, -O-, -S-, -C(=O)O- or -C(=O)NR⁷-.

5. Compound according to one or more or Claims 1 to 4, **characterised in that** it contains at least one group R⁴ that is selected from aromatic ring systems having 6 to 40 aromatic ring atoms and heteroaromatic ring systems having 5 to 40 aromatic ring atoms, which are in each case substituted by radicals R⁷.

6. Compound according to one or more of Claims 1 to 5, **characterised in that** index k is equal to 0.

7. Compound according to one or more of Claims 1 to 6, **characterised in that** Ar¹ is selected on each occurrence, identically or differently, from phenyl, naphthyl, fluorenyl and biphenyl, which are in each case substituted by radicals R³.

8. Compound according to one or more of Claims 1 to 7, **characterised in that** it conforms to a formula selected from the following formulae:
| |
|---|
| 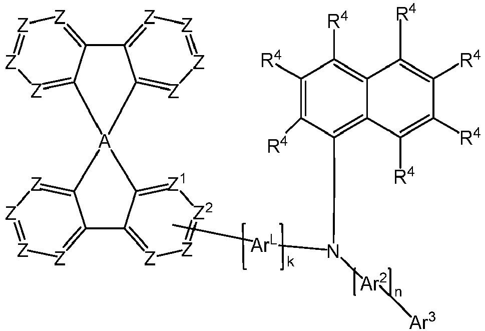 |
| formula (I-sub-1) |
| 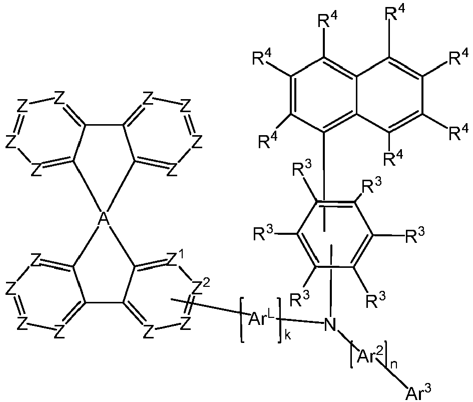 |
| formula (I-sub-2) |
| 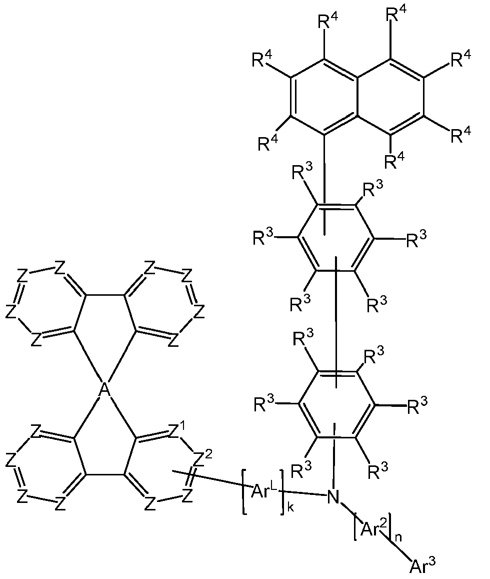 |
| formula (I-sub-3) |
where the groups and indices occurring are defined as in one or more of Claims 1 to 7.

9. Compound according to one or more of Claims 1 to 8, **characterised in that** it conforms to one of the following formulae:
| |
|---|
| 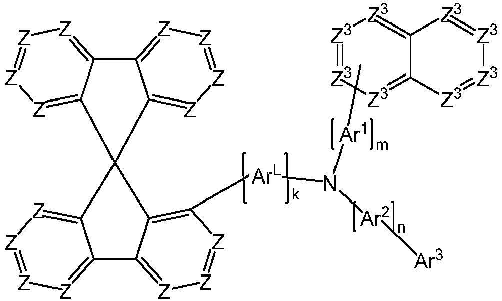 |
| (I-1) |
| 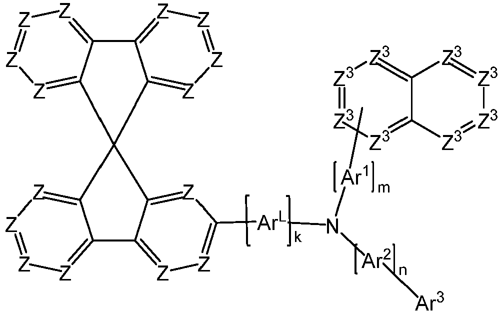 |
| (I-2) |
| 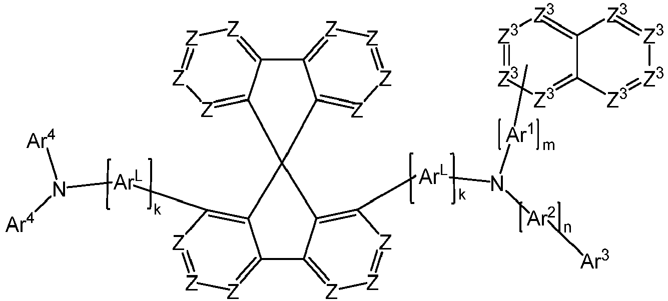 |
| (I-3) |
| 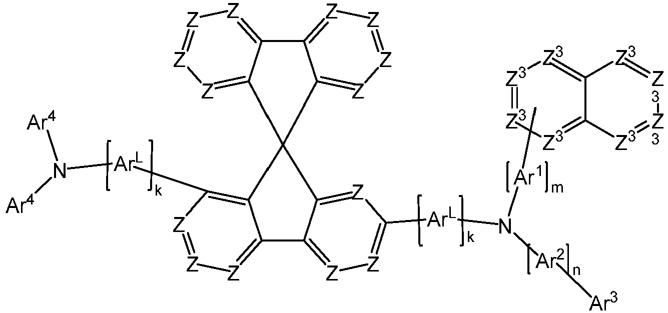 |
| (I-4) |
| 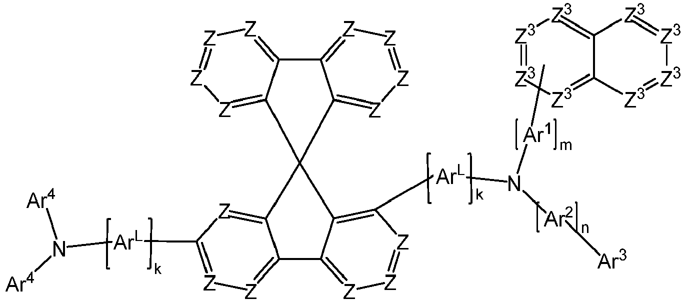 |
| (I-5) |
| 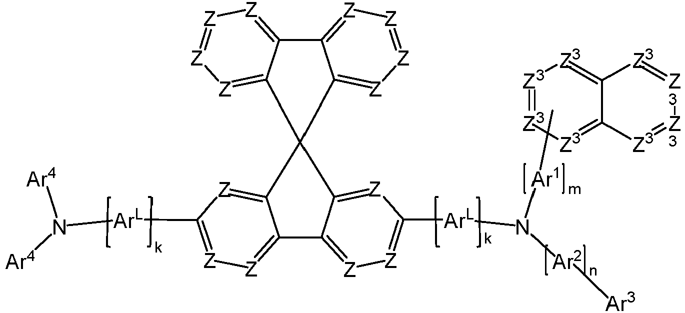 |
| (I-6) |
| 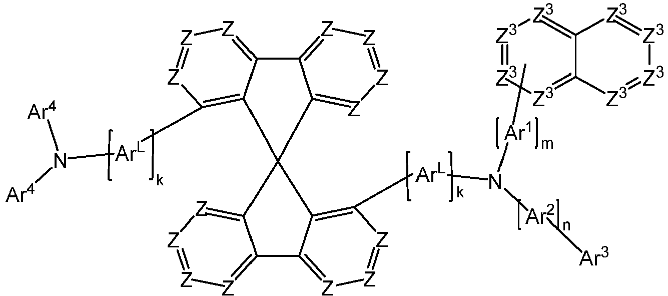 |
| (I-7) |
| 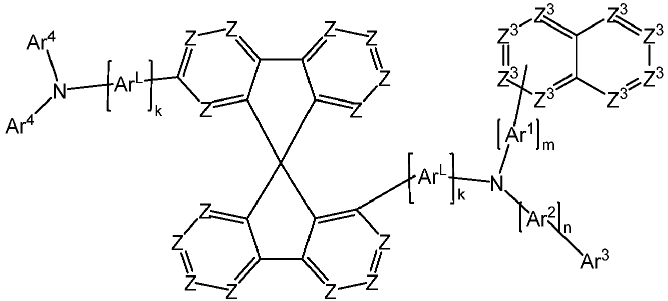 |
| (I-8) |
| 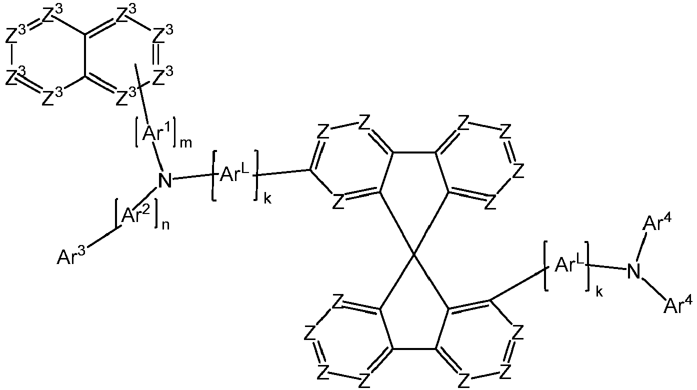 |
| (I-9) |
| 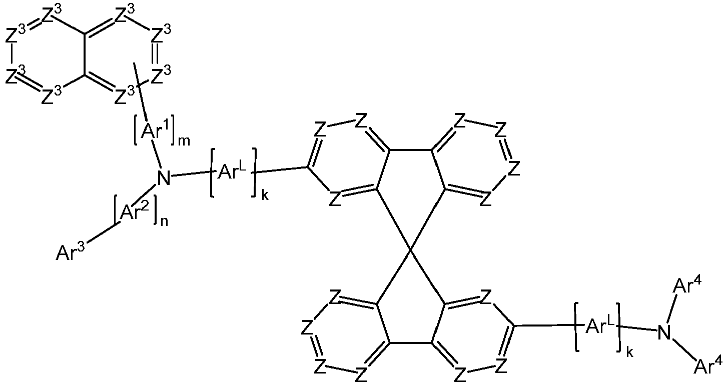 |
| (I-10) |
where the indices occurring are defined as in one or more of Claims 1 to 8.

10. Compound according to one or more of Claims 1 to 9, **characterised in that** Ar² is selected on each occurrence, identically or differently, from phenyl and naphthyl, which are in each case substituted by radicals R⁵.

11. Compound according to one or more of Claims 1 to 10, **characterised in that** index n is equal to 1 or 2.

12. Compound according to one or more of Claims 1 to 11, **characterised in that** Ar³ is selected from groups of the formulae (Ar³-2) and (Ar³-3):

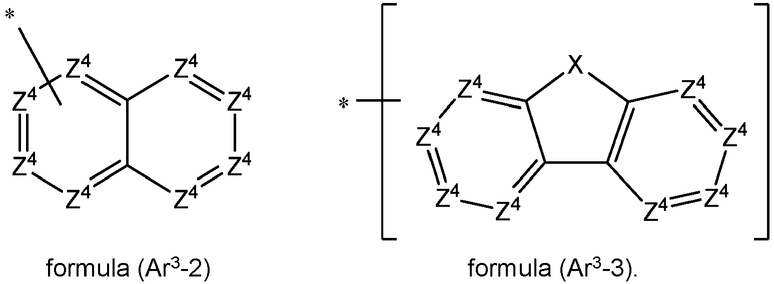

13. Compound according to one or more of Claims 1 to 12, **characterised in that** A is equal to C.

14. Process for the preparation of a compound according to one or more of Claims 1 to 13, **characterised in that** an amino group is introduced by means of an organometallic coupling reaction.

15. Oligomer, polymer or dendrimer containing one or more compounds according to one or more of Claims 1 to 13, where the bond(s) to the polymer, oligomer or dendrimer may be localised at any desired positions that are substituted by R¹, R², R³, R⁴, R⁵ or R⁶ in formula (I).

16. Formulation comprising at least one compound according to one or more of Claims 1 to 13 or at least one polymer, oligomer or dendrimer according to Claim 15 and at least one solvent.

17. Electronic device containing at least one compound according to one or more of Claims 1 to 13 or at least one polymer, oligomer or dendrimer according to Claim 15.

18. Electronic device according to Claim 17, **characterised in that** it is an organic electroluminescent device and comprises anode, cathode and at least one emitting layer, and **in that** the compound is present in a hole-transporting layer or in an emitting layer of the device.

19. Use of a compound according to one or more of Claims 1 to 13 in an electronic device.

## Revendications

1. Composé de formule (I) 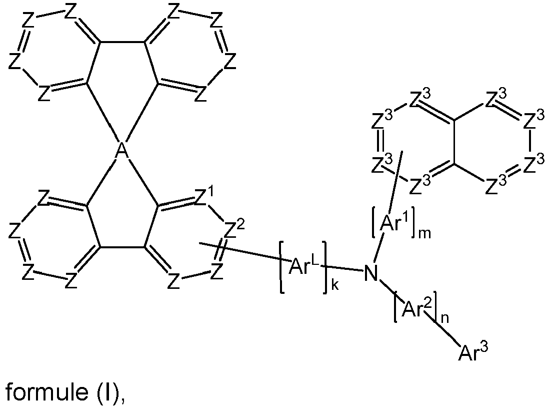
dans laquelle le groupement amino dessiné avec une position de liaison variable au spirobifluorène dans la formule (I) est lié à Z¹ ou bien à Z² ; et
où ce qui suit s'applique aux groupements et indices présents :
A est égal à C ou Si ;
Ar^{L} est choisi à chaque occurrence, de manière identique ou différente, parmi des noyaux aromatiques ayant de 6 à 40 atomes de cycle aromatique, qui sont dans chaque cas substitués par des radicaux R², et des noyaux hétéroaromatiques ayant de 5 à 40 atomes de cycle aromatique, qui sont dans chaque cas substitués par des radicaux R² ;
Ar¹ est choisi à chaque occurrence, de manière identique ou différente, parmi des noyaux aromatiques ayant de 6 à 24 atomes de cycle aromatique, qui sont substitués par des radicaux R³, et des noyaux hétéroaromatiques ayant de 5 à 24 atomes de cycle aromatique, qui sont substitués par des radicaux R³ ;
Ar² est choisi à chaque occurrence, de manière identique ou différente, parmi phényle, qui est substitué par des radicaux R⁵, naphtyle, qui est substitué par des radicaux R⁵, et des noyaux hétéroaromatiques ayant de 6 à 10 atomes de cycle aromatique, qui sont dans chaque cas substitués par des radicaux R⁵ ;
Ar³ est choisi parmi les groupements de formules (Ar³-1) à (Ar³-7), qui sont liés au reste de la formule (I) via la liaison marquée par * 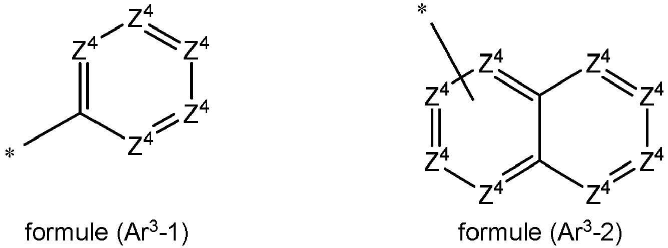 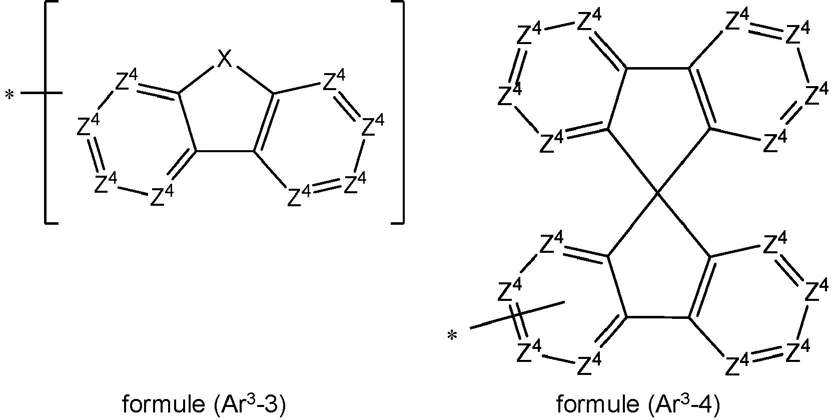 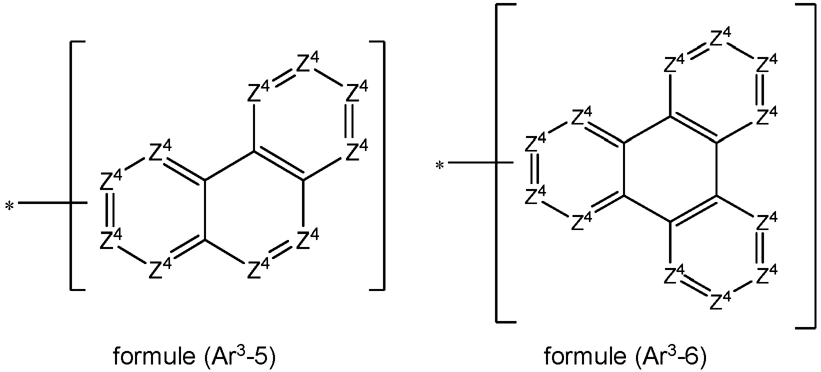 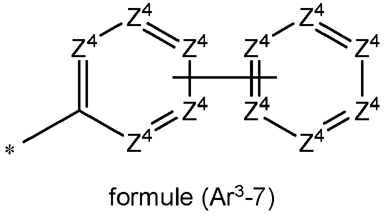
où Z⁴ est choisi à chaque occurrence, de manière identique ou différente, parmi CR⁶ et N, et où dans les formules (Ar³-2) à (Ar³-7) le groupement Z⁴ qui est lié via une liaison librement dessinée est C ; où dans la formule (Ar³-3) X équivalent à C(R⁶)₂ peut également être lié au reste de la formule (I), auquel cas un groupement R⁶ dans ce groupement X est remplacé par la liaison au reste de la formule (I) ;
et où X est choisi parmi O, S, NR⁶ et C(R⁶)₂ ; et où dans la formule (Ar³-3) un ou plusieurs motifs Z⁴=Z⁴ peuvent être remplacés par un motif choisi parmi les motifs 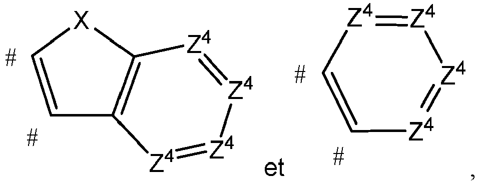
où les atomes marqués par # dans chaque cas remplacent les atomes Z⁴ ; et où dans la formule (Ar³-3) un ou plusieurs motifs Z⁴-Z⁴ peuvent être remplacés par un motif choisi parmi les motifs 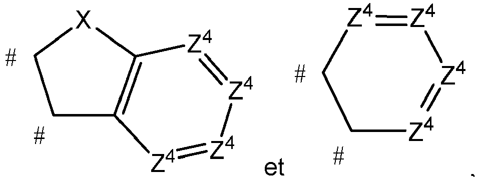
où les atomes marqués par # dans chaque cas remplacent les atomes Z⁴ ;
Z est choisi à chaque occurrence, de manière identique ou différente, parmi CR¹ et N ;
Z¹ et Z² sont dans chaque cas C si le groupement amino dessiné avec la position de liaison variable y est lié et sont sinon choisis parmi CR¹ et N ;
Z³ est choisi à chaque occurrence, de manière identique ou différente, parmi CR⁴ et N ;
R¹ est choisi à chaque occurrence, de manière identique ou différente, parmi H, D, F, Cl, Br, I, C(=O)R⁷, CN, Si(R⁷)₃, N(R⁷)₂, -(Ar^{L})ₖ-N(Ar⁴)₂, P(=O)(R⁷)₂, OR⁷, S(=O)R⁷, S(=O)₂R⁷, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹, R², R³, R⁴, R⁵ ou R⁶ peuvent être liés les uns aux autres et peuvent former un cycle ; où lesdits groupements alkyle, alcoxy, alcényle et alcynyle et lesdits noyaux aromatiques et noyaux hétéroaromatiques sont dans chaque cas substitués par des radicaux R⁷ ; et où un ou plusieurs groupements CH₂ dans lesdits groupements alkyle, alcoxy, alcényle et alcynyle peuvent être remplacés par -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO ou SO₂ ;
R², R³, R⁴, R⁵ et R⁶ sont dans chaque cas choisis, à chaque occurrence de manière identique ou différente, parmi H, D, F, Cl, Br, I, C(=O)R⁷, CN, Si(R⁷)₃, N(R⁷)₂, P(=O)(R⁷)₂, OR⁷, S(=O)R⁷, S(=O)₂R⁷, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R², R³, R⁴, R⁵ ou R⁶ peuvent être liés les uns aux autres et peuvent former un cycle ; où lesdits groupements alkyle, alcoxy, alcényle et alcynyle et lesdits noyaux aromatiques et noyaux hétéroaromatiques sont dans chaque cas substitués par des radicaux R⁷ ; et où un ou plusieurs groupements CH₂ dans lesdits groupements alkyle, alcoxy, alcényle et alcynyle peuvent être remplacés par -R⁷C=CR⁷-, -C=C-, Si(R⁷)₂, C=O, C=NR⁷, -C(=O)O-, -C(=O)NR⁷-, NR⁷, P(=O)(R⁷), -O-, -S-, SO ou SO₂ ;
Ar⁴ est choisi à chaque occurrence, de manière identique ou différente, parmi des noyaux aromatiques ayant de 6 à 40 atomes de cycle aromatique, qui sont substitués par un ou plusieurs radicaux R⁷, et des noyaux hétéroaromatiques ayant de 5 à 40 atomes de cycle aromatique, qui sont substitués par un ou plusieurs radicaux R⁷ ;
R⁷ est choisi à chaque occurrence, de manière identique ou différente, parmi H, D, F, Cl, Br, I, C(=O)R⁸, CN, Si(R⁸)₃, N(R⁸)₂, P(=O)(R⁸)₂, OR⁸, S(=O)R⁸, S(=O)₂R⁸, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹, R², R³, R⁴, R⁵ ou R⁶ peuvent être liés les uns aux autres et peuvent former un cycle ; où lesdits groupements alkyle, alcoxy, alcényle et alcynyle et lesdits noyaux aromatiques et noyaux hétéroaromatiques sont dans chaque cas substitués par des radicaux R⁸ ; et où un ou plusieurs groupements CH₂ dans lesdits groupements alkyle, alcoxy, alcényle et alcynyle peuvent être remplacés par -R⁸C=CR⁸-, -C=C-, Si(R⁸)₂, C=O, C=NR⁸, -C(=O)O-, -C(=O)NR⁸-, NR⁸, P(=O)(R⁸), -O-, -S-, SO ou SO₂ ;
R⁸ est choisi à chaque occurrence, de manière identique ou différente, parmi H, D, F, Cl, Br, I, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R⁸ peuvent être liés les uns aux autres et peuvent former un cycle ; et où lesdits groupements alkyle, alcoxy, alcényle et alcynyle, noyaux aromatiques et noyaux hétéroaromatiques peuvent être substitués par un ou plusieurs radicaux choisis parmi F et CN;
k est égal à 0, 1, 2, 3 ou 4 ;
m est égal à 0, 1, 2, 3 ou 4 ;
n est égal à 1, 2, 3 ou 4.

2. Composé selon la revendication 1, **caractérisé en ce que** Z¹ et Z² sont dans chaque cas C si le groupement amino dessiné avec la position de liaison variable y est lié, et sont sinon égaux à CR¹, Z est égal à CR¹, Z³ est égal à CR⁴ et Z⁴ est égal à CR⁶.

3. Composé selon la revendication 1 ou 2, **caractérisé en ce que** R¹ est choisi à chaque occurrence, de manière identique ou différente, parmi H, -[Ar^{L}]ₖ-N(Ar⁴)₂, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, lesdits noyaux aromatiques et lesdits noyaux hétéroaromatiques sont dans chaque cas substitués par des radicaux R⁷.

4. Composé selon l'une ou plusieurs parmi les revendications 1 à 3, **caractérisé en ce que** R², R³, R⁴, R⁵ et R⁶ sont choisis à chaque occurrence, de manière identique ou différente, parmi H, D, F, CN, Si(R⁷)₃, N(R⁷)₂, des groupements alkyle ou alcoxy à chaîne linéaire ayant de 1 à 20 atomes de C, des groupements alkyle ou alcoxy ramifiés ou cycliques ayant de 3 à 20 atomes de C, des noyaux aromatiques ayant de 6 à 40 atomes de cycle aromatique et des noyaux hétéroaromatiques ayant de 5 à 40 atomes de cycle aromatique ; où lesdits groupements alkyle et alcoxy, lesdits noyaux aromatiques et lesdits noyaux hétéroaromatiques sont dans chaque cas substitués par des radicaux R⁷ ; et où un ou plusieurs groupements CH₂ dans lesdits groupements alkyle ou alcoxy peuvent être remplacés par -C=C-, -R⁷C=CR⁷-, Si(R⁷)₂, C=O, C=NR⁷, -NR⁷-, -O-, -S-, -C(=O)O- ou -C(=O)NR⁷-.

5. Composé selon l'une ou plusieurs parmi les revendications 1 à 4, **caractérisé en ce qu'**il contient au moins un groupement R⁴ qui est choisi parmi des noyaux aromatiques ayant de 6 à 40 atomes de cycle aromatique et des noyaux hétéroaromatiques ayant de 5 à 40 atomes de cycle aromatique, qui sont dans chaque cas substitués par des radicaux R⁷.

6. Composé selon l'une ou plusieurs parmi les revendications 1 à 5, **caractérisé en ce que** l'indice k est égal à 0.

7. Composé selon l'une ou plusieurs parmi les revendications 1 à 6, **caractérisé en ce que** Ar¹ est choisi à chaque occurrence, de manière identique ou différente, parmi phényle, naphtyle, fluorényle et biphényle, qui sont dans chaque cas substitués par des radicaux R³.

8. Composé selon l'une ou plusieurs parmi les revendications 1 à 7, **caractérisé en ce qu'**il répond à une formule choisie parmi les formules suivantes :
| |
|---|
| 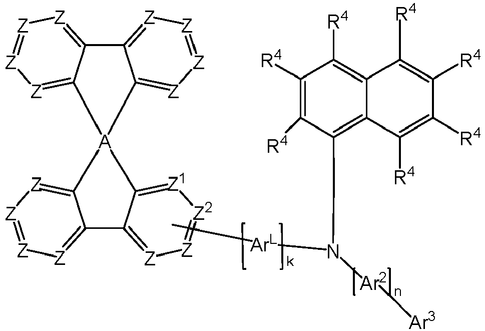 |
| formule (I-sub-1) |
| 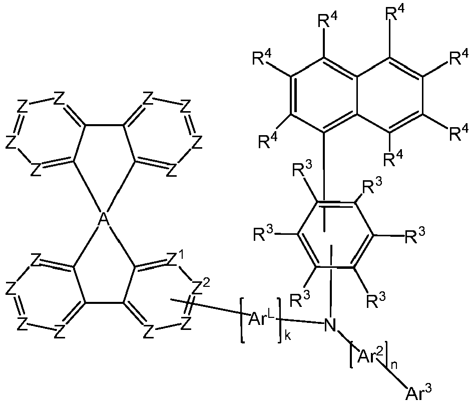 |
| formule (I-sub-2) |
| 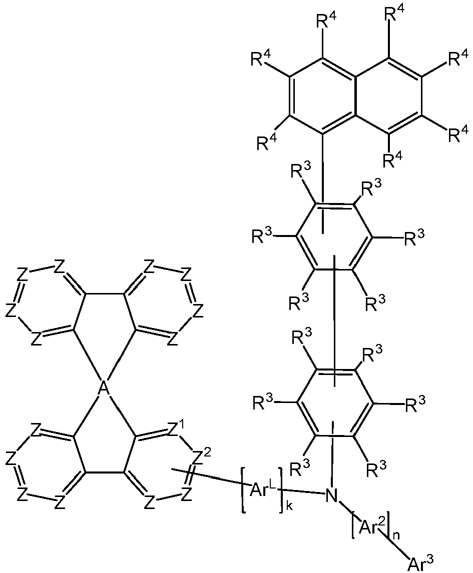 |
| formule (I-sub-3) |
dans lesquelles les groupements et les indices présents sont tels que définis selon l'une ou plusieurs parmi les revendications 1 à 7.

9. Composé selon l'une ou plusieurs parmi les revendications 1 à 8, **caractérisé en ce qu'**il répond à l'une des formules suivantes :
| |
|---|
| 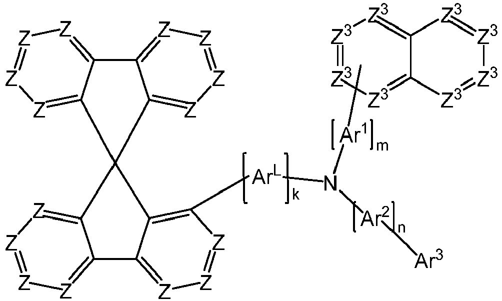 |
| (I-1) |
| 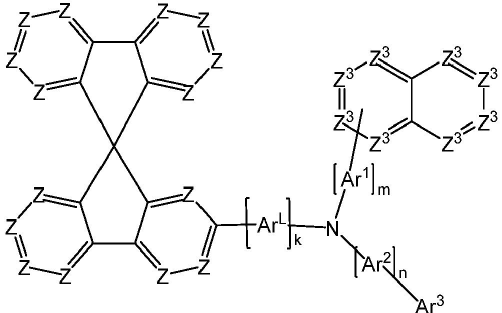 |
| (I-2) |
| 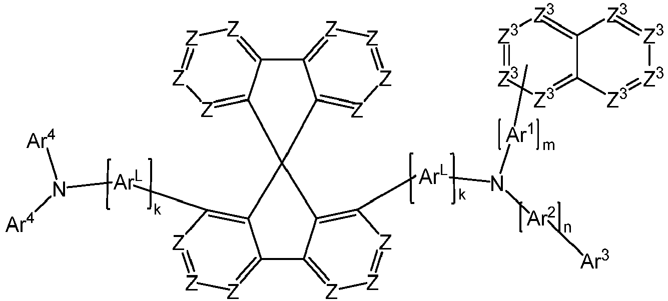 |
| (I-3) |
| 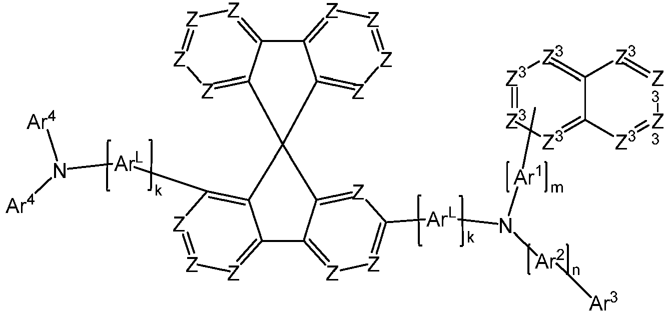 |
| (I-4) |
| 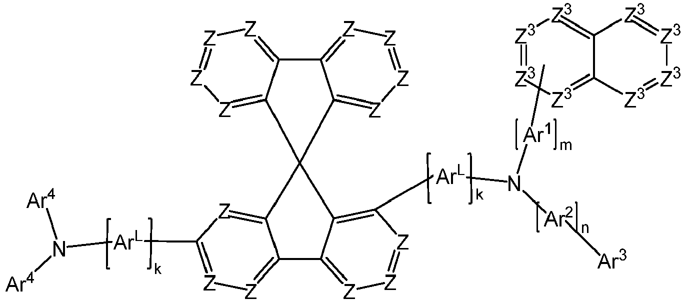 |
| (I-5) |
| 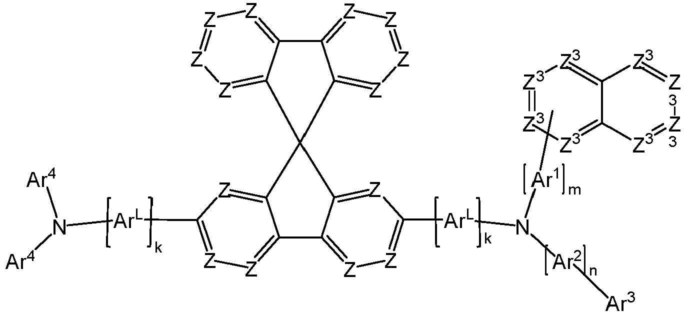 |
| (I-6) |
| 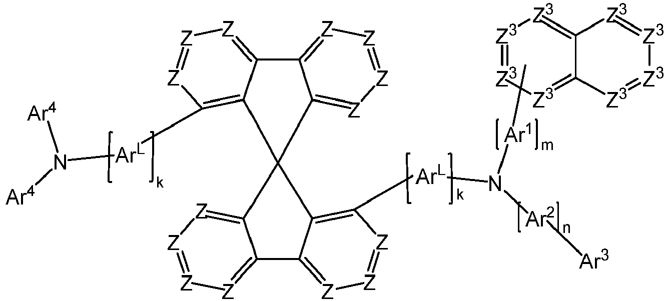 |
| (I-7) |
| 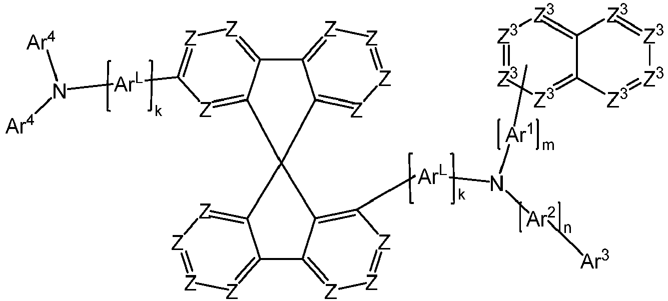 |
| (I-8) |
| 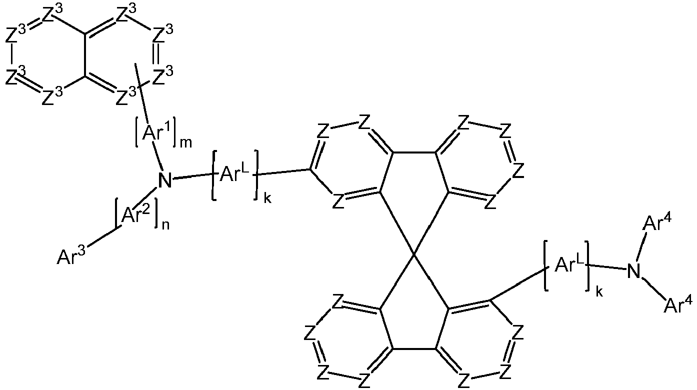 |
| (I-9) |
| 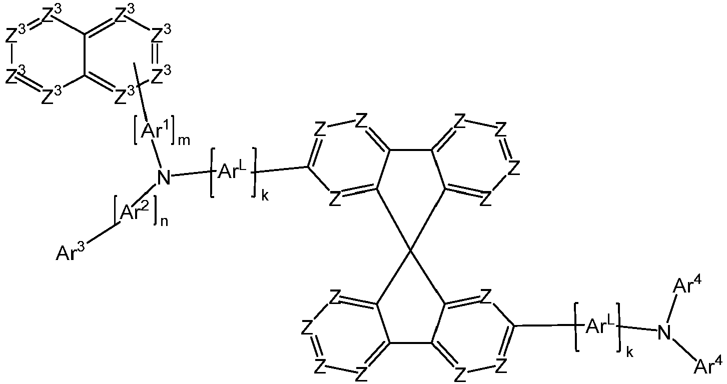 |
| (I-10) |
dans lesquelles les indices présents sont tels que définis selon l'une ou plusieurs parmi les revendications 1 à 8.

10. Composé selon l'une ou plusieurs parmi les revendications 1 à 9, **caractérisé en ce que** Ar² est choisi à chaque occurrence, de manière identique ou différente, parmi phényle et naphtyle, qui sont dans chaque cas substitués par des radicaux R⁵.

11. Composé selon l'une ou plusieurs parmi les revendications 1 à 10, **caractérisé en ce que** l'indice n est égal à 1 ou 2.

12. Composé selon l'une ou plusieurs parmi les revendications 1 à 11, **caractérisé en ce que** Ar³ est choisi parmi les groupements de formules (Ar³-2) et (Ar³-3) :

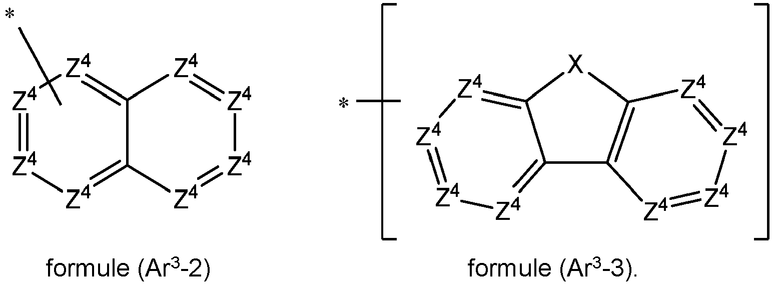

13. Composé selon l'une ou plusieurs parmi les revendications 1 à 12, **caractérisé en ce que** A est égal à C.

14. Procédé de préparation d'un composé selon l'une ou plusieurs parmi les revendications 1 à 13, **caractérisé en ce qu'**un groupement amino est introduit au moyen d'un réaction de couplage organométallique.

15. Oligomère, polymère ou dendrimère contenant un ou plusieurs composés selon l'une ou plusieurs parmi les revendications 1 à 13, la ou les liaisons au polymère, à l'oligomère ou au dendrimère pouvant être situées au niveau de positions désirées quelconques qui sont substituées par R¹, R², R³, R⁴, R⁵ ou R⁶ dans la formule (I).

16. Formulation comprenant au moins un composé selon l'une ou plusieurs parmi les revendications 1 à 13 ou au moins un polymère, oligomère ou dendrimère selon la revendication 15 et au moins un solvant.

17. Dispositif électronique contenant au moins un composé selon l'une ou plusieurs parmi les revendications 1 à 13 ou au moins un polymère, oligomère ou dendrimère selon la revendication 15.

18. Dispositif électronique selon la revendication 17, **caractérisé en ce qu'**il s'agit d'un dispositif électroluminescent organique et qu'il comprend une anode, une cathode et au moins une couche émettrice, et **en ce que** le composé est présent dans une couche de transport de trous ou dans une couche émettrice du dispositif.

19. Utilisation d'un composé selon l'une ou plusieurs parmi les revendications 1 à 13 dans un dispositif électronique.
